# EUROPEAN PATENT SPECIFICATION

(11) **EP 3 494 143 B1**
(45) Date of publication and mention of the grant of the patent: **05.10.2022**
(21) Application number: 17751568.1
(22) Date of filing: 02.08.2017
(51) Int. Cl.: C07K 16/44

(54) **PLAZOMICIN ANTIBODIES AND METHODS OF USE**
PLAZOMICIN-ANTIKÖRPER UND VERFAHREN ZUR VERWENDUNG
ANTICORPS CONTRE LA PLAZOMICINE ET PROCÉDÉS D'UTILISATION

(30) Priority: 03.08.2016 US 201662370580 P
(43) Date of publication of application: 12.06.2019
(73) Proprietor: Cipla USA, Inc., Warren NJ 07059 (US)
(72) Inventor: BENDER, Kristina, Oresic, South San Francisco, CA 94080 (US); HENRY, Christopher, E., South San Francisco, CA 94080 (US); THEOLIS, Richard, South San Francisco, CA 94080 (US); WU, Shuang, South San Francisco, CA 94080 (US)
(74) Representative: Marks & Clerk LLP
(86) International application number: PCT/US2017/045183
(87) International publication number: WO 2018/026969

(56) References cited:
- WO-A1-94/25053
- WO-A1-2015/127407
- WO-A2-2010/036460
- US-A1- 2003 060 430
- J. B. AGGEN ET AL: "Synthesis and Spectrum of the Neoglycoside ACHN-490", ANTIMICROBIAL AGENTS AND CHEMOTHERAPY, vol. 54, no. 11, 1 November 2010 (2010-11-01), pages 4636-4642, XP055417336, ISSN: 0066-4804, DOI: 10.1128/AAC.00572-10
- ELIANA S ARMSTRONG ET AL: "Combating evolution with intelligent design: the neoglycoside ACHN-490", CURRENT OPINION IN MICROBIOLOGY, vol. 13, no. 5, 1 October 2010 (2010-10-01), pages 565-573, XP055417322, GB ISSN: 1369-5274, DOI: 10.1016/j.mib.2010.09.004
- M L NOLLI ET AL: "Antibodies against the antibiotics: an overview", ANN. IST. SUPER. SANITÀ, vol. 27, no. 1, 1 January 1991 (1991-01-01), pages 149-154, XP055417241,
- MORAN SHALEV ET AL: "Development of generic immunoassay for the detection of a series of aminoglycosides with 6'-OH group for the treatment of genetic diseases in biological samples", JOURNAL OF PHARMACEUTICAL AND BIOMEDICAL ANALYSIS, vol. 75, 1 March 2013 (2013-03-01), pages 33-40, XP055417130, US ISSN: 0731-7085, DOI: 10.1016/j.jpba.2012.11.014
- WANG S ET AL: "Development of enzyme-linked immunosorbent assay (ELISA) for the detection of neomycin residues in pig muscle, chicken muscle, egg, fish, milk and kidney", MEAT SCIENCE, ELSEVIER SCIENCE, GB, vol. 82, no. 1, 1 May 2009 (2009-05-01), pages 53-58, XP025953423, ISSN: 0309-1740, DOI: 10.1016/J.MEATSCI.2008.12.003 [retrieved on 2008-12-14]
- STEAD D A: "Current methodologies for the analysis of aminoglycosides", JOURNAL OF CHROMATOGRAPHY B: BIOMEDICAL SCIENCES AND APPLICAT, ELSEVIER SCIENCE PUBLISHERS, NL, vol. 747, no. 1-2, 29 September 2000 (2000-09-29), pages 69-93, XP004217519, ISSN: 0378-4347, DOI: 10.1016/S0378-4347(00)00133-X

## Description

### TECHNICAL FIELD

The present invention relates generally to anti-plazomicin antibodies and methods of use thereof. In certain aspects, the invention is more specifically related to anti-plazomicin antibodies for use in determining levels of plazomicin in samples from human patients undergoing treatment with plazomicin. Such antibodies are useful, for example, in monitoring plazomicin levels during treatment for an infectious disease.

Any references in the description to methods of diagnosis (or treatment) refer to the compounds, pharmaceutical compositions and medicaments of the present invention for use in a method for diagnosis (or treatment) of the human (or animal) body by therapy. **BACKGROUND**

The antibiotic plazomicin is a member of the aminoglycoside class of antibacterial agents. Aminoglycosides have been successfully used for the treatment of serious bacterial infections for more than 50 years. However, the spread of resistance to currently marketed aminoglycosides has decreased their clinical utility. Plazomicin was developed by chemically modifying an existing aminoglycoside, sisomicin, in order to overcome common aminoglycoside resistance mechanisms.

Clinically, aminoglycosides demonstrate significant variability in the relationship between the dose of drug administered and the levels detected in the blood. Further, aminoglycoside antibiotic treatment can be associated with nephrotoxicity (Mingeot-Leclercq, Antimicrob. Agents Chemother., 1999, 43(5) 1003-1012). Therefore, aminoglycosides have the potential to cause renal damage, notably in patients with renal impairment (Riddle et al, ECCMID 2013, poster # P-918). In order to achieve optimal drug dosing for each patient and to mitigate potential nephrotoxic effects, therapeutic drug monitoring (TDM) of aminoglycosides, including plazomicin treatment, may be performed.

There is a need in the art for methods of determining plazomicin levels in the blood of patients treated with plazomicin, as well as reagents that specifically bind plazomicin with minimal cross-reactivity with other clinically relevant aminoglycosides. This invention provides these and other advantages described further herein.

### BRIEF DESCRIPTION OF THE DRAWINGS

Figure 1A: ELISA results of MC329, MC330, MC331, MC332, MC334, MC335, MC335, MC337, MC339, and MC340 binding to BSA-plazomicin. Graph shows A450 (y-axis) versus aminoglycoside concentration (nM, x-axis). IC50 (nM) values for each antibody are indicated in the table.
Figure 1B: Competitive ELISA results assessing the ability of MC329, MC330, MC331, MC332, MC334, MC335, MC335, MC337, MC339, and MC340 to bind four different aminoglycosides; plazomicin (circles), amikacin (squares), sisomicin (upward pointing triangles), and gentamicin (downward pointing triangles). Graphs show A450 (y-axis) versus antibiotic concentrations (µg/mL, x-axis). IC50 values for each antibiotic in µg/ml are also shown in Table 5.
Figure 2A shows a sequence alignment of variable light chain regions for antibodies described herein, aligned according to Kabat numbering. CDR delineations according to various schemes are illustrated above the residues.
Figure 2b shows a sequence alignment of variable heavy chain regions for antibodies described herein, aligned according to Kabat numbering. CDR delineations according to various schemes are illustrated above the residues.

### BRIEF SUMMARY

The present invention provides plazomicin-specific antibodies and methods of determining levels of plazomicin in a biological sample.

Provided herein is an isolated antibody, as defined by claim 1,or an antigen-binding fragment thereof, wherein the antibody or antigen-binding fragment thereof specifically binds plazomicin or a pharmaceutically acceptable salt thereof. In some embodiments, the antibody or antigen-binding fragment thereof specifically binds plazomicin. In some embodiments, the isolated antibody or antigen-binding fragment thereof comprises: a light chain variable region comprising an amino acid sequence at least 85%, 86%, 87%, 88%, 89%, 90%, 91%, 92%, 93%, 94%, 95%, 96%, 97%, 98%, 99% identical to the amino acid sequence set forth in any one of SEQ ID NOs: 26, 30, 38, 46, 54, 62, 70, 78, 86, 94, or 102; and a heavy chain variable region comprising an amino acid sequence at least 85%, 86%, 87%, 88%, 89%, 90%, 91%, 92%, 93%, 94%, 95%, 96%, 97%, 98%, 99% identical to the amino acid sequence set forth in any one of SEQ ID NOS: 22, 34, 42, 50, 58, 66, 74, 82, 90, or 98.

Also provided herein is an isolated antibody or antigen-binding fragment thereof, comprising: a light chain variable region comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 26, 30, 38, 46, 54, 62, 70, 78, 86, 94, or 102; and/or a heavy chain variable region comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOS: 22, 34, 42, 50, 58, 66, 74, 82, 90 or 98.

In some teachings, the isolated antibody or antigen-binding fragment thereof comprises a light chain variable region comprising a VL-CDR2 comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 199, 206, or 229.

In some teachings, the isolated antibody or antigen-binding fragment thereof comprises a light chain variable region comprising a VL-CDR3 comprising the sequence according to SEQ ID NO: 29, SEQ ID NO: 57, SEQ ID NO: 73, SEQ ID NO: 81; SEQ ID NO: 97, or SEQ ID NO: 223.

In some teachings, the isolated antibody or antigen-binding fragment thereof comprises a heavy chain variable region comprising (i) a VH-CDR1 comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 106, 115, 124, 133, 151, 169, 178, or 187; (ii) a VH-CDR2 comprising an amino acid sequence having at least 85% identity to an amino acid sequence selected from any one of SEQ ID NOs: 109, 118, 127, 136, 154, 163, 172, 181, or 190; and/or (iii) a VH-CDR3 comprising an amino acid sequence having at least 85% identity to an amino acid sequence selected from any one of SEQ ID NOs: 112, 121, 130,139, 157, 175, 184, 193.

In some teachings, the isolated antibody or antigen-binding fragment thereof comprises a heavy chain variable region comprising (i) a VH-CDR1 comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 106, 115, 124, 133, 151, 169, 178, or 187, (ii) a VH-CDR2 comprising an amino acid sequence having at least 85% identity to an amino acid sequence selected from any one of SEQ ID NOs: 109, 118, 127, 136, 154, 163, 172, 181, or 190, and (iii) a VH-CDR3 comprising an amino acid sequence having at least 85% identity to an amino acid sequence selected from any one of SEQ ID NOs: 112, 121, 130 ,139, 157, 175, 184, 193; and/or a light chain variable region comprising (i) a VL-CDR1 comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 196, 203, 218, 226, or 254, (ii) a VL-CDR2 comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 199, 206, or 229, and (iii) a VL-CDR3 comprising an amino acid sequence having at least 85% identity to an amino acid sequence selected from any one of SEQ ID NOs: 29, 57, 73, 81, 97, 208, or 223.

Further provided herein is an isolated antibody or antigen-binding fragment thereof comprising a heavy chain variable region and a light chain variable region, wherein: the heavy chain variable region comprising (i) a VH-CDR1 comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 106, 115, 124, 133, 151, 169, 178, or 187, (ii) a VH-CDR2 comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 109, 118, 127, 136, 154, 163, 172, 181, or 190, and (iii) a VH-CDR3 comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 112, 121, 130,139, 157, 175, 184, 193; and the light chain variable region comprising (i) a VL-CDR1 comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 196, 203, 218, 226, or 254, (ii) a VL-CDR2 comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 199, 206, or 229, and (iii) a VL-CDR3 comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 29, 57, 73, 81, 97, 208, or 223.

In some teachings, the isolated antibody or antigen-binding fragment thereof comprises a heavy chain variable region comprises: (i) a VH-CDR1 comprising the amino acid sequence selected from SEQ ID NOs: 106, 115, 124, 133, 151, 169, 178, or 187; (ii) a VH-CDR2 comprising the amino acid sequence selected from SEQ ID NOs: 109, 118, 127, 136, 154, 163, 172, 181, or 190,; and (iii) a VH-CDR3 comprising the amino acid sequence selected from SEQ ID NOs: 112, 121, 130, 139, 157, 175, 184, 193; and/or a light chain variable region comprises: (i) a VL-CDR1 comprising the amino acid sequence selected from SEQ ID NOs: 196, 203, 218, 226, or 254; (ii) a VL-CDR2 comprising the amino acid sequence selected from SEQ ID NOs: 199, 206, or 229; and (iii) a VL-CDR3 comprising the amino acid sequence selected from SEQ ID NOs: 29, 57, 73, 81, 97, 208, or 223.

Also provided herein is an isolated antibody or the antigen-binding fragment thereof, comprises a light chain variable region comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOs: 26, 30, 38, 46, 54, 62, 70, 78, 86, 94, or 102, wherein the light chain variable region comprises a VL-CDR1 set forth in SEQ ID NO: SEQ ID NO: 196, 203, 218, 226, or 254; a VL-CDR2 set forth in SEQ ID NO: 199, 206 or 229; and a VL-CDR3 set forth in 29, 57, 73, 81, 97, 208, or 223; and a heavy chain variable region comprising an amino acid sequence having at least 85%, 86%, 87%, 88%, 89%, 90%, 91%, 92%, 93%, 94%, 95%, 96%, 97%, 98%, 99% identity to an amino acid sequence selected from any one of SEQ ID NOS: 22, 34, 42, 50, 58, 66, 74, 82, 90 or 98, wherein the heavy chain variable region comprises a VH-CDR1 set forth in 106, 115, 124, 133, 151, 169, 178, or 187; a VH-CDR2 set forth in SEQ ID NO: 109, 118, 127, 136, 154, 163, 172, 181, or 190; and a VL-CDR3 set forth in SEQ ID NOs: 29, 57, 73, 81, 97, 208, or 223. In accordance with the invention, the antibody or antigen-binding fragment thereof specifically binds plazomicin or a pharmaceutically acceptable salt thereof.

Further provided herein is an isolated antibody or antigen-binding fragment thereof, comprising: a light chain variable region comprising a VL-CDR1, a VL-CDR2, and a VL-CDR3, respectively, comprising the amino acid sequence of a VL-CDR1, a VL-CDR2, and a VL-CDR3 contained within the light chain variable region set forth in SEQ ID NO: 26, SEQ ID NO: 30, SEQ ID NO: 38, SEQ ID NO: 46, SEQ ID NO: 54, SEQ ID NO: 62, SEQ ID NO: 70, SEQ ID NO: 78, SEQ ID NO: 86, SEQ ID NO: 94, or SEQ ID NO: 102; and a heavy chain variable region comprising a VH-CDR1, a VH-CDR2, and a VH-CDR3, respectively, comprising the amino acid sequence of a VH-CDR1, a VH-CDR2, and a VH-CDR3 contained within the heavy chain variable region set forth in SEQ ID NO: 22, SEQ ID NO: 34, SEQ ID NO: 42, SEQ ID NO: 50, SEQ ID NO: 58, SEQ ID NO: 66, SEQ ID NO: 74: SEQ ID NO: 82; SEQ ID NO: 90, and SEQ ID NO: 98.

In some teachings of the provided antibody or antigen binding fragment thereof, the isolated antibody or antigen-binding fragment thereof comprises (a) a VL-CDR1 comprising the amino acid sequence of SEQ ID NO: 196; a VL-CDR2 comprising the amino acid sequence of SEQ ID NO: 199; and a VL-CDR3 comprising the amino acid sequence of SEQ ID NO: 29; (b) a VL-CDR1 comprising the amino acid sequence of SEQ ID NO: 203; a VL-CDR2 comprising the amino acid sequence of SEQ ID NO: 206; and a VL-CDR3 comprising the amino acid sequence of SEQ ID NO: 208; (c) a VL-CDR1 comprising the amino acid sequence of SEQ ID NO: 218; a VL-CDR2 comprising the amino acid sequence of SEQ ID NO: 221; and a VL-CDR3 comprising the amino acid sequence of SEQ ID NO: 223; (d) a VL-CDR1 comprising the amino acid sequence of SEQ ID NO: 226; a VL-CDR2 comprising the amino acid sequence of SEQ ID NO: 229; and a VL-CDR3 comprising the amino acid sequence of SEQ ID NO: 57; (e) a VL-CDR1 comprising the amino acid sequence of SEQ ID NO: 240; a VL-CDR2 comprising the amino acid sequence of SEQ ID NO: 243; and a VL-CDR3 comprising the amino acid sequence of SEQ ID NO: 73; (f) a VL-CDR1 comprising the amino acid sequence of SEQ ID NO: 247; a VL-CDR2 comprising the amino acid sequence of SEQ ID NO: 250; and a VL-CDR3 comprising the amino acid sequence of SEQ ID NO: 81; (g) a VL-CDR1 comprising the amino acid sequence of SEQ ID NO: 254; a VL-CDR2 comprising the amino acid sequence of SEQ ID NO: 257; and a VL-CDR3 comprising the amino acid sequence of SEQ ID NO: 89; or (h) a VL-CDR1 comprising the amino acid sequence of SEQ ID NO: 261; a VL-CDR2 comprising the amino acid sequence of SEQ ID NO: 264; and a VL-CDR3 comprising the amino acid sequence of SEQ ID NO: 97.

In some teachings, the light chain variable region comprising a murine framework.

In some embodiments, the isolated antibody or antigen-binding fragment thereof comprises a light chain variable region comprising the sequence set forth in SEQ ID NO: 26, SEQ ID NO: 30, SEQ ID NO: 38, SEQ ID NO: 46, SEQ ID NO: 54, SEQ ID NO: 62, SEQ ID NO: 70, SEQ ID NO: 78, SEQ ID NO: 86, SEQ ID NO: 94, or SEQ ID NO: 102.

In some teachings, the isolated antibody or antigen-binding fragment thereof comprises a heavy chain variable region comprising: (a) a VH-CDR1 comprising the amino acid sequence of SEQ ID NO: 106; a VH-CDR2 comprising the amino acid sequence of SEQ ID NO: 109; and a VH-CDR3 comprising the amino acid sequence of SEQ ID NO: 112; (b) a VH-CDR1 comprising the amino acid sequence of SEQ ID NO: 115; a VH-CDR2 comprising the amino acid sequence of SEQ ID NO: 118; and a VH-CDR3 comprising the amino acid sequence of SEQ ID NO: 121; (c) a VH-CDR1 comprising the amino acid sequence of SEQ ID NO: 124; a VH-CDR2 comprising the amino acid sequence of SEQ ID NO: 127; and a VH-CDR3 comprising the amino acid sequence of SEQ ID NO: 130; (d) a VH-CDR1 comprising the amino acid sequence of SEQ ID NO: 133; a VH-CDR2 comprising the amino acid sequence of SEQ ID NO: 136; and a VH-CDR3 comprising the amino acid sequence of SEQ ID NO: 139; (e) a VH-CDR1 comprising the amino acid sequence of SEQ ID NO: 151; a VH-CDR2 comprising the amino acid sequence of SEQ ID NO: 154; and a VH-CDR3 comprising the amino acid sequence of SEQ ID NO: 157; (f) a VH-CDR1 comprising the amino acid sequence of SEQ ID NO: 160; a VH-CDR2 comprising the amino acid sequence of SEQ ID NO: 163; and a VH-CDR3 comprising the amino acid sequence of SEQ ID NO: 166; (g) a VH-CDR1 comprising the amino acid sequence of SEQ ID NO: 169; a VH-CDR2 comprising the amino acid sequence of SEQ ID NO: 172; and a VH-CDR3 comprising the amino acid sequence of SEQ ID NO: 175; (h) a VH-CDR1 comprising the amino acid sequence of SEQ ID NO: 178; a VH-CDR2 comprising the amino acid sequence of SEQ ID NO: 181; and a VH-CDR3 comprising the amino acid sequence of SEQ ID NO: 184; or (i) a VH-CDR1 comprising the amino acid sequence of SEQ ID NO: 187; a VH-CDR2 comprising the amino acid sequence of SEQ ID NO: 190; and a VH-CDR3 comprising the amino acid sequence of SEQ ID NO: 193.

In some teachings, the antibody or antigen binding fragment thereof comprises a heavy chain variable region comprising a murine framework.

In some embodiments, the antibody or antigen binding fragment thereof comprises a heavy chain variable region comprising the sequence set forth in SEQ ID NO: 22, SEQ ID NO: 34, SEQ ID NO: 42, SEQ ID NO: 50, SEQ ID NO: 58, SEQ ID NO: 66, SEQ ID NO: 74: SEQ ID NO: 82; SEQ ID NO: 90, or SEQ ID NO: 98.

In some teachings, the antibody or antigen binding fragment thereof comprises (a) a light chain variable region comprising VL-CDR1, a VL-CDR2 and VL-CDR3 set forth in SEQ ID NO: 196, SEQ ID NO: 199, and SEQ ID NO: 29, respectively; and; a heavy chain variable region comprising VH-CDR1, VH-CDR2 and VH-CDR3 set forth in SEQ ID NO: 106, SEQ ID NO: 109, and SEQ ID NO: 112, respectively; (b) a light chain variable region comprising VL-CDR1, a VL-CDR2 and VL-CDR3 set forth in SEQ ID NO: 203, SEQ ID NO: 206, and SEQ ID NO: 208, respectively; and; a heavy chain variable region comprising VH-CDR1, VH-CDR2 and VH-CDR3 set forth in SEQ ID NO: 106, SEQ ID NO: 109, and SEQ ID NO: 112, respectively; (c) a light chain variable region comprising VL-CDR1, a VL-CDR2 and VL-CDR3 set forth in SEQ ID NO: 211, SEQ ID NO: 214, and SEQ ID NO: 41, respectively; and; a heavy chain variable region comprising VH-CDR1, VH-CDR2 and VH-CDR3 set forth in SEQ ID NO: 115, SEQ ID NO: 118, and SEQ ID NO: 121, respectively; (d) a light chain variable region comprising VL-CDR1, a VL-CDR2 and VL-CDR3 set forth in SEQ ID NO: 218, SEQ ID NO: 221, and SEQ ID NO: 223, respectively; and; a heavy chain variable region comprising VH-CDR1, VH-CDR2 and VH-CDR3 set forth in SEQ ID NO: 124, SEQ ID NO: 127, and SEQ ID NO: 130, respectively; (e) a light chain variable region comprising VL-CDR1, a VL-CDR2 and VL-CDR3 set forth in SEQ ID NO: 226, SEQ ID NO: 229, and SEQ ID NO: 57, respectively; and; a heavy chain variable region comprising VH-CDR1, VH-CDR2 and VH-CDR3 set forth in SEQ ID NO: 133, SEQ ID NO: 136, and SEQ ID NO: 139, respectively; (f) a light chain variable region comprising VL-CDR1, a VL-CDR2 and VL-CDR3 set forth in SEQ ID NO: 240, SEQ ID NO: 243, and SEQ ID NO: 73, respectively; and; a heavy chain variable region comprising VH-CDR1, VH-CDR2 and VH-CDR3 set forth in SEQ ID NO: 151, SEQ ID NO: 154, and SEQ ID NO: 157, respectively; (g) a light chain variable region comprising VL-CDR1, a VL-CDR2 and VL-CDR3 set forth in SEQ ID NO: 247, SEQ ID NO: 250, and SEQ ID NO: 81, respectively; and; a heavy chain variable region comprising VH-CDR1, VH-CDR2 and VH-CDR3 set forth in SEQ ID NO: 160, SEQ ID NO: 163, and SEQ ID NO: 166, respectively; (h) a light chain variable region comprising VL-CDR1, a VL-CDR2 and VL-CDR3 set forth in SEQ ID NO: 254, SEQ ID NO: 257, and SEQ ID NO: 89, respectively; and; a heavy chain variable region comprising VH-CDR1, VH-CDR2 and VH-CDR3 set forth in SEQ ID NO: 169, SEQ ID NO: 172, and SEQ ID NO: 175, respectively; (i) a light chain variable region comprising VL-CDR1, a VL-CDR2 and VL-CDR3 set forth in SEQ ID NO: 261, SEQ ID NO: 264, and SEQ ID NO: 97, respectively; and; a heavy chain variable region comprising VH-CDR1, VH-CDR2 and VH-CDR3 set forth in SEQ ID NO: 178, SEQ ID NO: 181, and SEQ ID NO: 184, respectively; or (j) a light chain variable region comprising VL-CDR1, a VL-CDR2 and VL-CDR3 set forth in SEQ ID NO: 268, SEQ ID NO: 271, and SEQ ID NO: 105, respectively; and; a heavy chain variable region comprising VH-CDR1, VH-CDR2 and VH-CDR3 set forth in SEQ ID NO: 187, SEQ ID NO: 190, and SEQ ID NO: 193, respectively.

In some teachings, the antibody or antigen binding fragment thereof comprises (a) a variable light chain region that is at least 85% identical to SEQ ID NO: 26, and a variable heavy chain region that is at least 85% identical to SEQ ID NO: 22; (b) a variable light chain region that is at least 85% identical to SEQ ID NO: 30, and a variable heavy chain region that is at least 85% identical to SEQ ID NO: 22; (c) a variable light chain region that is at least 85% identical to SEQ ID NO: 38, and a variable heavy chain region that is at least 85% identical to SEQ ID NO: 34; (d) a variable light chain region that is at least 85% identical to SEQ ID NO: 46, and a variable heavy chain region that is at least 85% identical to SEQ ID NO: 42; (e) a variable light chain region that is at least 85% identical to SEQ ID NO: 54, and a variable heavy chain region that is at least 85% identical to SEQ ID NO: 50; (f) a variable light chain region that is at least 85% identical to SEQ ID NO: 62, and a variable heavy chain region that is at least 85% identical to SEQ ID NO: 58; (g) a variable light chain region that is at least 85% identical to SEQ ID NO: 70, and a variable heavy chain region that is at least 85% identical to SEQ ID NO: 66; (h) a variable light chain region that is at least 85% identical to SEQ ID NO: 78, and a variable heavy chain region that is at least 85% identical to SEQ ID NO: 74; (i) a variable light chain region that is at least 85% identical to SEQ ID NO: 86, and a variable heavy chain region that is at least 85% identical to SEQ ID NO: 82; (j) a variable light chain region that is at least 85% identical to SEQ ID NO: 94, and a variable heavy chain region that is at least 85% identical to SEQ ID NO: 90; or (k) a variable light chain region that is at least 85% identical to SEQ ID NO: 102, and a variable heavy chain region that is at least 85% identical to SEQ ID NO: 98. In any of the above embodiments/teachings, the variable light chain region is at least 86%, 87%, 88%, 89%, 90%, 91%, 92%, 93%, 94%, 95%, 96%, 97%, 98%, 99% identical to the reference sequence. In any of the above embodiments/teachings, the variable heavy chain region is at least 86%, 87%, 88%, 89%, 90%, 91%, 92%, 93%, 94%, 95%, 96%, 97%, 98%, 99% identical to the reference sequence.

In some teachings, the antibody or antigen binding fragment thereof comprises (a) a variable light chain region that is at least 95% identical to SEQ ID NO: 26, and a variable heavy chain region that is at least 95% identical to SEQ ID NO: 22; (b) a variable light chain region that is at least 95% identical to SEQ ID NO: 30, and a variable heavy chain region that is at least 95% identical to SEQ ID NO: 22; (c) a variable light chain region that is at least 95% identical to SEQ ID NO: 38, and a variable heavy chain region that is at least 95% identical to SEQ ID NO: 34; (d) a variable light chain region that is at least 95% identical to SEQ ID NO: 46, and a variable heavy chain region that is at least 95% identical to SEQ ID NO: 42; (e) a variable light chain region that is at least 95% identical to SEQ ID NO: 54, and a variable heavy chain region that is at least 95% identical to SEQ ID NO: 50; (f) a variable light chain region that is at least 95% identical to SEQ ID NO: 62, and a variable heavy chain region that is at least 95% identical to SEQ ID NO: 58; (g) a variable light chain region that is at least 95% identical to SEQ ID NO: 70, and a variable heavy chain region that is at least 95% identical to SEQ ID NO: 66; (h) a variable light chain region that is at least 95% identical to SEQ ID NO: 78, and a variable heavy chain region that is at least 95% identical to SEQ ID NO: 74; (i) a variable light chain region that is at least 95% identical to SEQ ID NO: 86, and a variable heavy chain region that is at least 95% identical to SEQ ID NO: 82; (j) a variable light chain region that is at least 95% identical to SEQ ID NO: 94, and a variable heavy chain region that is at least 95% identical to SEQ ID NO: 90; or (k) a variable light chain region that is at least 95% identical to SEQ ID NO: 102, and a variable heavy chain region that is at least 95% identical to SEQ ID NO: 98.

In some teachings, the antibody or antigen binding fragment thereof comprises (a) a variable light chain region according to SEQ ID NO: 26, and a variable heavy chain region according to SEQ ID NO: 22; (b) a variable light chain region according to SEQ ID NO: 30, and a variable heavy chain region according to SEQ ID NO: 22; (c) a variable light chain region according to SEQ ID NO: 38, and a variable heavy chain region according 1 to SEQ ID NO: 34; (d) a variable light chain region according to SEQ ID NO: 46, and a variable heavy chain region according to SEQ ID NO: 42; (e) a variable light chain region according to SEQ ID NO: 54, and a variable heavy chain region according to SEQ ID NO: 50; (f) a variable light chain region according to SEQ ID NO: 62, and a variable heavy chain region according to SEQ ID NO: 58; (g) a variable light chain region according to SEQ ID NO: 70, and a variable heavy chain region according to SEQ ID NO: 66; (h) a variable light chain region according to SEQ ID NO: 78, and a variable heavy chain region according to SEQ ID NO: 74; (i) a variable light chain region according to SEQ ID NO: 86, and a variable heavy chain region according to SEQ ID NO: 82; (j) a variable light chain region according to SEQ ID NO: 94, and a variable heavy chain region according to SEQ ID NO: 90; or (k) a variable light chain region according to SEQ ID NO: 102, and a variable heavy chain region according to SEQ ID NO: 98.

In some embodiments, the antibody or antigen binding fragment thereof as provided is a whole antibody. In some embodiments, the antibody or antigen binding fragment thereof as provided is an antigen-binding fragment selected from the group consisting of, a univalent antibody generated from an IgG4 antibody with the hinge region removed , a single chain variable fragment (scFv), a Fab fragment and a F(ab')₂ fragment.

In accordance with the inventions the provided isolated antibody or antigen binding fragment thereof, the isolated antibody or antigen binding fragment thereof specifically binds plazomicin or a pharmaceutically acceptable salt thereof. In accordance with the invention the provided isolated antibody or antigen binding fragment thereof, the isolated antibody or antigen binding fragment thereof specifically binds plazomicin.

In some embodiments of the provided isolated antibody or antigen binding fragment thereof, the antibody or antigen binding fragment thereof specifically binds to plazomicin or a pharmaceutically acceptable salt thereof with an IC50 of about 100 µg/mL or less. In some embodiments of the provided isolated antibody or antigen binding fragment, the antibody or antigen binding fragment thereof specifically binds to plazomicin or a pharmaceutically acceptable salt thereof with an IC50 of about 19 µg/mL or less. In some embodiments of the provided isolated antibody or antigen binding fragment thereof, the antibody or antigen binding fragment thereof specifically binds to plazomicin or a pharmaceutically acceptable salt thereof with an IC50 of about 0.02 µg/mL or more. In some embodiments, the IC50 is determined by a competitive ELISA assay.

In some embodiments of the provided isolated antibody or antigen binding fragment thereof, the antibody or antigen binding fragment thereof binds to plazomicin or the pharmaceutically acceptable salt thereof with a binding affinity greater than the binding affinity of the antibody or antigen binding fragment thereof to a non-plazomicin aminoglycoside. In some embodiments, the non-plazomicin aminoglycoside is amikacin, sisomicin, or gentamicin. In some embodiments, the antibody or antigen binding fragment thereof binds to plazomicin or the pharmaceutically acceptable salt thereof with an equilibrium dissociation constant of about 10⁻⁷ M or less.

In some embodiments, the isolated antibody or antigen binding fragment thereof as provided is a humanized antibody, a chimeric antibody or a human antibody. In some embodiments, the isolated antibody or antigen binding fragment thereof as provided is murine antibody.

In some embodiments, the antibody or antigen-binding fragment further comprises a detectable marker.

Also provided herein is a nucleic acid encoding a an antibody or antigen-binding fragment as provided. Further provided herein is a nucleic acid encoding a heavy chain comprising a variable heavy chain region of a provided antibody or antigen-binding fragment. Further provided herein is a nucleic acid encoding a light chain comprising a variable light chain region of a provided antibody or antigen-binding fragment. In some embodiments, the nucleic acid comprises cDNA. In some embodiments, the nucleic acid is codon-optimized for expression in a cell. Also provided herein is a vector comprising the nucleic acid. In some embodiments, the vector is an expression vector. Further provided is a host cell comprising the vector. Additionally, provided herein is a method of producing an antibody comprising culturing the host cell under a condition that produces the antibody. In some embodiments, the method comprises recovering the antibody produced by the host cell.

Further provided is a composition comprising any of the antibodies or antigen-binding fragments as provided. In some embodiments, the composition comprises a pharmaceutically acceptable carrier.

Also described herein is a solid support, comprising immobilized thereto an antibody or antigen-binding fragment as provided. In some embodiments, the solid support is a bead, a column, an array, an assay plate, a microwell, a stick, a filter, or a strip. Also described herein is a device comprising the solid support.

Also provided herein is a solid support, comprising immobilized thereto plazomicin or a pharmaceutically acceptable salt thereof. In some embodiments, the solid support is a bead, a column, an array, an assay plate, a microwell, a stick, a filter, or a strip. Also described herein is a device comprising the solid support.

Further taught herein is a kit comprising any one or more of the provided antibodies or antigen-binding fragments, or compositions, or the solid support or the device as provided, and instructions for use. In some teachings, the kit further comprises plazomicin. In some teachings, the kit comprises one or more of the provided antibody or antigen-binding fragment and plazomicin and a pharmaceutically acceptable salt thereof. In some teachings, the antibody or antigen-binding fragment is immobilized on a solid support. In some teachings, the plazomicin or a pharmaceutically acceptable salt thereof is immobilized on a solid support. In some teachings, the instructions provide instruction for performing an assay for detecting plazomicin or a pharmaceutically acceptable salt thereof in a biological sample and/or for determining an amount of plazomicin or a pharmaceutically acceptable salt thereof in a biological sample. In some teachings, the instructions specify the detecting is performed using an immunoassay. In some teachings, the immunoassay is selected from the group consisting of a cloned enzyme donor immunoassay (CEDIA), a turbidity assay, and a competitive ELISA.

Additionally, described herein is a method of determining a level of plazomicin or a pharmaceutically acceptable salt thereof in a biological sample, comprising: (i) contacting the sample with an isolated antibody, or an antigen-binding fragment thereof, as provided; and (ii) detecting the presence or absence of, or an amount of, the antibody bound to the plazomicin or the pharmaceutically acceptable salt thereof, thereby determining the level of plazomicin or the pharmaceutically acceptable salt thereof in the biological sample. In some embodiments, the biological sample is a liquid. In some embodiments, the biological sample is a serum, a plasma, blood, urine, saliva, or sputum. In some embodiments, the detecting is performed using an immunoassay. In some embodiments, the immunoassay is selected from the group consisting of a cloned enzyme donor immunoassay (CEDIA), a turbidity assay, and a competitive ELISA. In some embodiments, the biological sample was obtained from a subject to whom plazomicin or a pharmaceutically acceptable salt thereof had been administered. In some embodiments, the biological sample is obtained after a period of time after administering the plazomicin or a pharmaceutically acceptable salt thereof.

In some teachings, the method further comprises selecting a subject for administering a subsequent dose of plazomicin or a pharmaceutically acceptable salt thereof, wherein: if the plazomicin or a pharmaceutically acceptable salt thereof in the biological sample is below or equal to a predetermined cut-off value, the subject is selected to receive the subsequent dose; or if the plazomicin or a pharmaceutically acceptable salt thereof in the biological sample is above the predetermined cut-off value, the subject is selected to not receive the subsequent dose. In some teachings, the method further comprises selecting a subject for administering a subsequent dose of plazomicin or a pharmaceutically acceptable salt thereof, wherein: if the plazomicin or a pharmaceutically acceptable salt thereof in the biological sample is below or equal to a predetermined cut-off value, the subject is selected to receive a subsequent dose equal to or greater than the prior dose; or if the plazomicin or a pharmaceutically acceptable salt thereof in the biological sample is above the predetermined cut-off value, the subject is selected to receive a subsequent dose that is lower than the prior dose. In some teachings, the method comprises administering the subsequent dose to the subject.

Also provided herein is a method of treating bacterial infection in a subject, comprising: (i) administering a dose of plazomicin or a pharmaceutically acceptable salt thereof to the subject; and (ii) determining the level of plazomicin or a pharmaceutically acceptable salt thereof in a biological sample obtained from the subject, such as in accord with the provided method for determining the level of plazomicin or a pharmaceutically acceptable salt thereof in a biological sample . In some teachings, the method comprises waiting a period of time between administering the dose and determining the level of plazomicin or the pharmaceutically acceptable salt thereof in the biological sample obtained from the subject. In some teachings, the method comprises administering a subsequent dose of plazomicin or the pharmaceutically acceptable salt thereof to the subject if the determined level of plazomicin or the pharmaceutically acceptable salt thereof is below or equal to a predetermined cut-off value. In some teachings, the method comprises administering a subsequent dose of plazomicin or the pharmaceutically acceptable salt thereof to the subject, wherein the subsequent dose is: (a) greater than the prior dose if a determined level of plazomicin or the pharmaceutically acceptable salt thereof in a biological sample obtained from the subject is below or equal to a predetermined cut-off value; or (b) lower than the prior does if the determined level of plazomicin or the pharmaceutically acceptable salt thereof in the biological sample is above the predetermined cut-off value. In some teachings, the method comprises receiving the results of an assay for determining the level of plazomicin or a pharmaceutically acceptable salt thereof in the biological sample.

Also provided herein is a method of treating bacterial infection in a subject, comprising administering a subsequent dose of plazomicin or a pharmaceutically acceptable salt thereof to the subject, wherein the subject has previously received a prior dose of plazomicin or the pharmaceutically acceptable salt thereof, and wherein the subsequent dose is: (a) greater than the prior dose if a determined level of plazomicin or the pharmaceutically acceptable salt thereof in a biological sample obtained from the subject is below or equal to a predetermined cut-off value; or (b) lower than the prior does if the determined level of plazomicin or the pharmaceutically acceptable salt thereof in the biological sample is above the predetermined cut-off value; wherein the level of plazomicin or the pharmaceutically acceptable salt thereof is determined according to the method described above.

In some teachings of the provided methods, the bacterial infection is caused by Gram-negative bacteria. In some teachings, the bacterial infection is caused by multi-drug resistant (MDR) bacteria. In some teachings, the bacterial infection is a urinary tract infection.

Further provided herein is a method of selecting a subject with a bacterial infection for treatment, comprising (i) determining a level of plazomicin or a pharmaceutically acceptable salt thereof in a biological sample, and (ii) selecting the subject for treatment if the plazomicin or a pharmaceutically acceptable salt thereof in the biological sample is below or equal to a predetermined cut-off value. In some teachings, the treatment is a subsequent dose. In some teachings the subsequent dose is greater than a dose administered to the subject before the level of plazomicin or the pharmaceutically acceptable salt thereof is determined.

In some teachings, there is provided a use of any one of the provided antibodies or antigen-binding fragments thereof for detecting the presence, level, or amount of plazomicin or a pharmaceutically acceptable salt thereof in a sample. In some teachings, the sample is a biological sample.

In some teachings, there is provided a use of any one of the provided antibodies or antigen-binding fragments thereof in the preparation of a composition for detecting the presence, level, or amount of plazomicin or a pharmaceutically acceptable salt thereof in a sample. In some teachings, the sample is a biological sample.

In some teachings, there is provided a composition comprising of any one of the provided antibodies or antigen-binding fragments thereof for use in detecting the presence, level, or amount of plazomicin or a pharmaceutically acceptable salt thereof in a sample. In some teachings, the sample is a biological sample.

Some teachings of the current invention are directed to an isolated antibody, or antigen-binding fragment thereof, that binds to plazomicin, comprising a heavy chain variable region and a light chain variable region, wherein the heavy chain variable region comprises a variable heavy chain complementarity determining region 1 (VHCDR1) region comprising SEQ ID NO:23, a variable heavy chain complementarity determining region 2 (VHCDR2) region comprising SEQ ID NO:24, and a variable heavy chain complementarity determining region 3 (VHCDR3) region comprising SEQ ID NO:25; and/or the light chain variable region comprises a variable light chain complementarity determining region 1 (VLCDR1) region comprising SEQ ID NO:27, a variable light chain complementarity determining region 2 (VLCDR2) region comprising SEQ ID NO:28, and a variable light chain complementarity determining region 3 (VLCDR3) region comprising SEQ ID NO:29. In certain embodiments, the heavy chain variable region comprises SEQ ID NO: 22 and/or the light chain variable region comprises SEQ ID NO: 26.

Further teachings of the current invention are directed to an isolated antibody, or antigen-binding fragment thereof, that binds to plazomicin, comprising a heavy chain variable region and a light chain variable region, wherein the heavy chain variable region comprises a VHCDR1 region comprising SEQ ID NO:23, a VHCDR2 region comprising SEQ ID NO:24, and a VHCDR3 region comprising SEQ ID NO:25; and/or the light chain variable region comprises a variable VLCDR1 region comprising SEQ ID NO:31, a VLCDR2 region comprising SEQ ID NO:32, and a VLCDR3 region comprising SEQ ID NO:33. In certain embodiments, the heavy chain variable region comprises SEQ ID NO: 22 and/or the light chain variable region comprises SEQ ID NO: 30.

Further teachings include an isolated antibody, or antigen-binding fragment thereof, that binds to plazomicin, comprising a heavy chain variable region and a light chain variable region, wherein the heavy chain variable region comprises a VHCDR1 region comprising SEQ ID NO:35, a VHCDR2 region comprising SEQ ID NO:36, and a VHCDR3 region comprising SEQ ID NO:37; and/or the light chain variable region comprises a VLCDR1 region comprising SEQ ID NO:39, a VLCDR2 region comprising SEQ ID N0:40, and a VLCDR3 region comprising SEQ ID NO:41. In certain embodiments, the heavy chain variable region comprises SEQ ID NO: 34 and/or the light chain variable region comprises SEQ ID NO: 38.

Certain teachings include an isolated antibody, or antigen-binding fragment thereof, that binds to plazomicin, comprising a heavy chain variable region and a light chain variable region, wherein the heavy chain variable region comprises a VHCDR1 region comprising SEQ ID NO:43, a VHCDR2 region comprising SEQ ID NO:44, and a VHCDR3 region comprising SEQ ID NO:45; and/or the light chain variable region comprises a VLCDR1 region comprising SEQ ID NO:47, a VLCDR2 region comprising SEQ ID NO:48, and a VLCDR3 region comprising SEQ ID NO:49. In certain embodiments, the heavy chain variable region comprises SEQ ID NO: 42 and/or the light chain variable region comprises SEQ ID NO: 46.

Further teachings include an isolated antibody, or antigen-binding fragment thereof, that binds to plazomicin, comprising a heavy chain variable region and a light chain variable region, wherein the heavy chain variable region comprises a VHCDR1 region comprising SEQ ID NO:51, a VHCDR2 region comprising SEQ ID NO:52, and a VHCDR3 region comprising SEQ ID NO:53; and/or the light chain variable region comprises a VLCDR1 region comprising SEQ ID NO:55, a VLCDR2 region comprising SEQ ID NO:56, and a VLCDR3 region comprising SEQ ID NO:57. In certain embodiments, the heavy chain variable region comprises SEQ ID NO: 50 and/or the light chain variable region comprises SEQ ID NO: 54.

Further teachings include an isolated antibody, or antigen-binding fragment thereof, that binds to plazomicin, comprising a heavy chain variable region and a light chain variable region, wherein the heavy chain variable region comprises a VHCDR1 region comprising SEQ ID NO:59, a VHCDR2 region comprising SEQ ID NO:60, and a VHCDR3 region comprising SEQ ID NO:61; and/or the light chain variable region comprises a VLCDR1 region comprising SEQ ID NO:63, a VLCDR2 region comprising SEQ ID NO:64, and a VLCDR3 region comprising SEQ ID NO:65. In certain embodiments, the heavy chain variable region comprises SEQ ID NO: 58 and/or the light chain variable region comprises SEQ ID NO: 62.

Certain teachings include an isolated antibody, or antigen-binding fragment thereof, that binds to plazomicin, comprising a heavy chain variable region and a light chain variable region, wherein the heavy chain variable region comprises a VHCDR1 region comprising SEQ ID NO:67, a VHCDR2 region comprising SEQ ID NO:68, and a VHCDR3 region comprising SEQ ID NO:69; and/or the light chain variable region comprises a VLCDR1 region comprising SEQ ID NO:71, a VLCDR2 region comprising SEQ ID NO:72, and a VLCDR3 region comprising SEQ ID NO:73.In certain embodiments, the heavy chain variable region comprises SEQ ID NO:66 and/or the light chain variable region comprises SEQ ID NO:70.

Further teachings include an isolated antibody, or antigen-binding fragment thereof, that binds to plazomicin, comprising a heavy chain variable region and a light chain variable region, wherein the heavy chain variable region comprises a VHCDR1 region comprising SEQ ID NO:75, a VHCDR2 region comprising SEQ ID NO:76, and a VHCDR3 region comprising SEQ ID NO:77; and/or the light chain variable region comprises a VLCDR1 region comprising SEQ ID NO:79, a VLCDR2 region comprising SEQ ID NO:80, and a VLCDR3 region comprising SEQ ID NO:81. In certain embodiments, the heavy chain variable region comprises SEQ ID NO: 74 and/or the light chain variable region comprises SEQ ID NO: 78.

Some teachings include an isolated antibody, or antigen-binding fragment thereof, that binds to plazomicin, comprising a heavy chain variable region and a light chain variable region, wherein the heavy chain variable region comprises a VHCDR1 region comprising SEQ ID NO:83, a VHCDR2 region comprising SEQ ID NO:84, and a VHCDR3 region comprising SEQ ID NO:85; and/or the light chain variable region comprises a VLCDR1 region comprising SEQ ID NO:87, a VLCDR2 region comprising SEQ ID NO:88, and a VLCDR3 region comprising SEQ ID NO:89. In certain embodiments, the heavy chain variable region comprises SEQ ID NO: 82 and/or the light chain variable region comprises SEQ ID NO: 86.

Further teachings include an isolated antibody, or antigen-binding fragment thereof, that binds to plazomicin, comprising a heavy chain variable region and a light chain variable region, wherein the heavy chain variable region comprises a VHCDR1 region comprising SEQ ID NO:91, a VHCDR2 region comprising SEQ ID NO:92, and a VHCDR3 region comprising SEQ ID NO:93; and/or the light chain variable region comprises a VLCDR1 region comprising SEQ ID NO:95, a VLCDR2 region comprising SEQ ID NO:96, and a VLCDR3 region comprising SEQ ID NO:97. In certain embodiments, the heavy chain variable region comprises SEQ ID NO: 90 and/or the light chain variable region comprises SEQ ID NO: 94.

Further teachings include an isolated antibody, or antigen-binding fragment thereof, that binds to plazomicin, comprising a heavy chain variable region and a light chain variable region, wherein the heavy chain variable region comprises a VHCDR1 region comprising SEQ ID NO:99, a VHCDR2 region comprising SEQ ID NO: 100, and a VHCDR3 region comprising SEQ ID NO: 101; and/or the light chain variable region comprises a VLCDR1 region comprising SEQ ID NO:103, a VLCDR2 region comprising SEQ ID NO:104, and a VLCDR3 region comprising SEQ ID NO:105. In certain embodiments, the heavy chain variable region comprises SEQ ID NO: 98 and/or the light chain variable region comprises SEQ ID NO: 102.

Certain aspect of this disclosure are directed to isolated antibodies, or an antigen-binding fragments thereof, that bind to plazomicin, wherein the antibody is selected from the group comprising a scFv or a univalent antibody lacking a hinge region. Further embodiments of this aspect of the disclosure relate to isolated antibodies, or antigen-binding fragments thereof, that bind to plazomicin, wherein the antibody is a Fab or Fab' fragment. Further embodiments of this aspect of the disclosure relate to isolated antibodies, or antigen-binding fragments thereof, that bind to plazomicin, wherein the antibody is a F(ab')₂ fragment. Still further embodiments of this aspect of the disclosure relate to isolated antibodies, or antigen-binding fragments thereof, that bind to plazomicin, wherein the antibody is a whole antibody. In some embodiments, the isolated antibody or antigen binding fragment thereof comprises a variable region and a constant region.

Certain embodiments of the present disclosure are directed to a method of determining a level of plazomicin in a biological sample comprising (i) contacting the sample with an isolated antibody, or antigen-binding fragment thereof, disclosed herein that binds plazomicin, and (ii) detecting the presence or absence of, or an amount of, the antibody bound to plazomicin by an immunoassay, and thereby determining the level of plazomicin. In one embodiment of this aspect of the disclosure, the sample is a liquid matrix and is selected from the group comprising serum, plasma, blood, urine, or sputum. In a further embodiment of this aspect of the disclosure, the sample was obtained from a subject to whom plazomicin has been administered. In a further embodiment of this aspect of the disclosure, the immunoassay is selected from the group comprising cloned enzyme donor immunoassay (CEDIA), turbidity assay, and competitive ELISA. In certain embodiments, the anti-plazomicin antibodies are bound to a detectable marker.

Some teachings of the present disclosure are directed to a method of treating a bacterial infection in a subject in need thereof, comprising (i) providing to the subject an effective amount of plazomicin, (ii) waiting for a first period of time, and (iii) determining the amount of or level of plazomicin in a biological sample obtained from the subject according to the methods described herein. Certain teachings of this aspect of the disclosure further comprise (i) providing additional plazomicin to the subject, if the determined level of plazomicin is below or equal to a cut-off value or (ii) not providing additional plazomicin to the subject, if the determined level of plazomicin is above a cut-off value. In a further teaching of this aspect of the disclosure, the bacterium is a gram-negative bacterium. In a further teaching of this aspect of the disclosure, the bacteria are a multi-drug resistant (MDR) bacteria. In a further teaching of this aspect of the disclosure, the infection is a urinary tract infection.

Certain embodiments of the present disclosure are directed to an isolated polynucleotide encoding any of the antibodies, or antigen-binding fragments thereof, that bind plazomicin as described herein. Further embodiments of this aspect of the disclosure are related to expression vectors comprising the isolated polynucleotides. Further embodiments of this aspect of the disclosure are directed to a host cell comprising said expression vector.

Also provided herein is a plazomicin-hapten conjugate. In some teachings, the hapten is keyhold limpet hemocyanin (KLH), fluorescein, dinitrophenol, biotin, digoxigenin, urushiol, hydralazine, aniline, o-amino-benzoic acid, m-aminobenzoic acid, and p-aminobenzoic acid. In some teachings, the plazomicin-hapten conjugate is a plazomicin-KLH conjugate. Also described herein is a method of generating an antibody that specifically binds to plazomicin, comprising administering the plazomicin-conjugate to a mammal. In some teachings, the mammal is a mouse.

### DETAILED DESCRIPTION

The present invention provides reagents and methods for determining plazomicin levels in biological samples, including plazomicin-specific antibodies that allow for the specific detection of plazomicin or a pharmaceutically acceptable salt thereof and their use in therapeutic drug monitoring (TDM) during plazomicin treatment. Several aminoglycoside antibiotics demonstrate a high degree of chemical similarity and it is possible that antibodies raised against a particular aminoglycoside, such as plazomicin, would have high cross reactivity with other aminoglycosides (Shalev, Moran et al. Journal of pharmaceutical and biomedical analysis 75 (2013): 33-40). An antibody that is cross-reactive to multiple aminoglycosides may not enable an assay to accurately measure concentrations of one particular compound in samples that contain two or more structurally similar antibiotics. US2003/060430 discloses antibodies that bind aminoglycosides such as tobramycin, amikacin and gentamicin.

The present disclosure relates to antibodies and antigen-binding fragments thereof that specifically bind to plazomicin or a pharmaceutically acceptable salt thereof, in particular antibodies having specific epitopic specificity. In particular embodiments, the antibodies and antigen-binding fragments thereof disclosed herein preferentially bind to plazomicin over other aminoglycosides, such that the binding affinity of the antibodies for plazomicin is at least 5 fold, at least 10 fold, at least 100 fold, or at least 1000 fold greater than the binding affinity of the antibodies for other aminoglycosides. Other aminoglycosides can include, but are not limited to, amikacin, sisomicin, or gentamicin. In further embodiments, the antibodies, and antigen-binding fragments thereof, disclosed herein bind to plazomicin with very high affinity. In certain embodiments, the IC50, as determined by the assay described in Example 1 (for example, a competitive ELISA assay) is less than 0.1µg/mL. In some embodiments, the antibody or antigen binding fragment thereof specifically binds to plazomicin or a pharmaceutically acceptable salt thereof with an IC50 of about 100 µg/mL or less (such as about 50 µg/mL or less, about 25 µg/mL or less, about 20 µg/mL or less, about 19 µg/mL or less, about 15 µg/mL or less, about 10 µg/mL or less, about 5 µg/mL or less, about 2 µg/mL or less, about 1 µg/mL or less, about 0.5 µg/mL or less, about 0.2 µg/mL or less, about 0.1 µg/mL or less, about 0.05 µg/mL or less, or about 0.02 µg/mL or less. In certain embodiments, the IC50 is less than 0.05 µg/mL. In certain embodiments, the half maximal inhibitory concentration (IC50) is about 0.02 µg/mL. In certain embodiments, the IC50 is about 0.012 µg/mL. In some embodiments, the IC50 is about 0.02 µg/mL or more.

Embodiments of the invention also pertain to the use of anti-plazomicin antibodies, or antigen-binding fragments thereof, in immunoassays designed for the therapeutic drug monitoring (TDM) of plazomicin treatment regimens. Certain embodiments provide a method of selecting a subject for further treatment after having received administration of plazomicin wherein the levels of plazomicin are determined from a biological sample taken from a subject after administration of plazomicin or a pharmaceutically acceptable salt thereof. Certain teachings provide a method of treating a bacterial infection wherein the levels of plazomicin are determined from a biological sample taken from a subject after administration of plazomicin treatment, such as after a first time period of plazomicin treatment. Further teachings comprise providing additional plazomicin to the subject if the determined level of plazomicin is below or above a defined cut-off value. In further teachings, additional plazomicin is not provided to the subject, or future dosage levels are decreased, if the determined level of plazomicin is above a defined cut-off value. In certain teachings, the bacteria are gram-negative bacteria. In further teachings, the bacteria are multidrug resistant bacteria. In further teachings, the infection is a urinary tract infection.

Each embodiment in this specification is to be applied *mutatis mutandis* to every other embodiment unless expressly stated otherwise.

The details of the disclosure are set forth in the accompanying description below. Although methods and materials similar or equivalent to those described herein can be used in the practice or testing of the present disclosure, illustrative methods and materials are now described. Other features, objects, and advantages of the disclosure will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this disclosure belongs.

The practice of the present invention will employ, unless indicated specifically to the contrary, conventional methods of virology, immunology, microbiology, molecular biology, and recombinant DNA techniques within the skill of the art, many of which are described below for the purpose of illustration. Such techniques are explained fully in the literature. See, *e.g.,* Current Protocols in Molecular Biology or Current Protocols in Immunology, John Wiley & Sons, New York, N.Y.(2009); Ausubel et al., Short Protocols in Molecular Biology, 3rd ed., Wiley & Sons, 1995; Sambrook and Russell, Molecular Cloning: A Laboratory Manual (3rd Edition, 2001); Maniatis et al. Molecular Cloning: A Laboratory Manual (1982); DNA Cloning: A Practical Approach, vol. I & II (D. Glover, ed.); Oligonucleotide Synthesis (N. Gait, ed., 1984); Nucleic Acid Hybridization (B. Hames & S. Higgins, eds., 1985); Transcription and Translation (B. Hames & S. Higgins, eds., 1984); Animal Cell Culture (R. Freshney, ed., 1986); Perbal, A Practical Guide to Molecular Cloning (1984) and other like references.

Standard techniques may be used for recombinant DNA, oligonucleotide synthesis, and tissue culture and transformation (*e.g*., electroporation, lipofection). Enzymatic reactions and purification techniques may be performed according to manufacturer's specifications or as commonly accomplished in the art or as described herein. These and related techniques and procedures may be generally performed according to conventional methods well known in the art and as described in various general and more specific references that are cited and discussed throughout the present specification. Unless specific definitions are provided, the nomenclature utilized in connection with, and the laboratory procedures and techniques of, molecular biology, analytical chemistry, synthetic organic chemistry, and medicinal and pharmaceutical chemistry described herein are those well-known and commonly used in the art. Standard techniques may be used for recombinant technology, molecular biological, microbiological, chemical syntheses, chemical analyses, pharmaceutical preparation, formulation, and delivery, and treatment of patients.

As used in this specification and the appended claims, the singular forms "a," "an" and "the" include plural references unless the content clearly dictates otherwise.

As used in this specification, the term "and/or" is used in this disclosure to either "and" or "or" unless indicated otherwise.

The term "about" as used herein refers to the usual error range for the respective value readily known to the skilled person in this technical field. Reference to "about" a value or parameter herein includes (and describes) embodiments that are directed to that value or parameter *per se.* For example, description referring to "about X" includes description of "X".

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

### Antibodies and antigen-binding fragments

In accordance with the invention, the antibodies or antigen-binding agents bind to plazomicin or a pharmaceutically acceptable salt thereof. The present invention provides antibodies and antigen-binding fragments thereof that specifically bind to plazomicin. In some embodiments, the antibodies or antigen-binding fragments thereof specifically bind to a pharmaceutically acceptable plazomicin salt.

Plazomicin (which can also be referred to a 6'-(hydroxylethyl)-1-(HABA)-sisomicin) is an aminoglycoside compound with antibacterial activity. Plazomicin was developed by chemically modifying an existing aminoglycoside, sisomicin, in order to overcome mechanisms of antibiotic resistance. This chemical modification comprises by appending a hydroxy-aminobutyric acid (HABA) substituent at position 1 and a hydroxyethyl substituent at position 6', resulting in the chemical structure of plazomicin pictured below. The inventions described herein refer to plazomicin and include stereoisomers, pharmaceutically acceptable salts, carriers, diluents, or excipients, and prodrugs thereof.

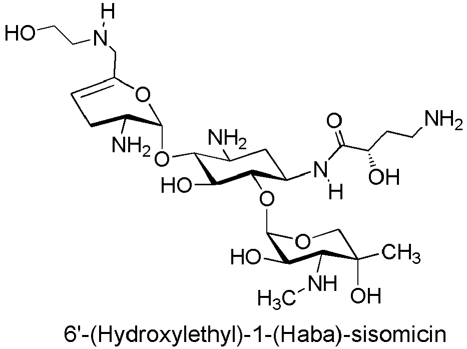

"Pharmaceutically acceptable salts" include both acid and base addition salts.

"Pharmaceutically acceptable acid addition salt" refers to those salts which retain the biological effectiveness and properties of the free bases, which are not biologically or otherwise undesirable, and which are formed with inorganic acids such as, but are not limited to, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, ptoluenesulfonic acid, trifluoroacetic acid, undecylenic acid, and the like.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, *N*-ethylpiperidine, polyamine resins and the like. Particularly preferred organic bases are isopropylamine, diethylamine, ethanolamine, trimethylamine, dicyclohexylamine, choline and caffeine.

The present invention also includes plazomicin-hapten conjugates,, *e.g*., plazomicin-keyhole limpet hemocyanin (KLH) as described in Example 1. As used herein, "hapten" refers to a small molecule that can elicit an immune response only when attached to a larger carrier, *e.g*., plazomicin. Non-limiting examples of haptens include KLH, fluorescein, dinitrophenol, biotin, digoxigenin, urushiol, hydralazine, aniline, o-aminobenzoic acid, m-aminobenzoic acid, and p-aminobenzoic acid.

As is well known in the art, an "antibody" is an immunoglobulin (Ig) molecule capable of specific binding to a target, such as a carbohydrate, polynucleotide, lipid, or polypeptide, through at least one epitope recognition site, located in the variable region of the Ig molecule. As used herein, the term encompasses not only intact polyclonal or monoclonal antibodies, but also fragments thereof, such as dAb, Fab, Fab', F(ab')₂, Fv), single chain (scFv), synthetic variants thereof, naturally occurring variants, fusion proteins comprising an antibody portion with an antigen-binding fragment of the required specificity, chimeric antibodies, nanobodies, and any other modified configuration of the immunoglobulin molecule that comprises an antigen-binding site or fragment (epitope recognition site) of the required specificity. Minibodies comprising a scFv joined to a CH3 domain are also included herein (S. Hu et al., Cancer Res., 56, 3055-3061, 1996). See *e.g.,* Ward, E. S. et al., Nature 341, 544-546 (1989); Bird et al., Science, 242, 423-426, 1988; Huston et al., PNAS USA, 85, 5879-5883, 1988); PCT/US92/09965; WO94/13804; P. Holliger et al., Proc. Natl. Acad. Sci. USA 90 6444-6448, 1993; Y. Reiter et al., Nature Biotech, 14, 1239-1245, 1996; S. Hu et al., Cancer Res., 56, 3055-3061, 1996.

In certain embodiments, antibodies and antigen-binding fragments thereof as described herein include a heavy chain and a light chain "complementarity determining region set" or "CDR set," respectively interposed between a heavy chain and a light chain "framework region (FR) set" which provide support to the CDRs and define the spatial relationship of the CDRs relative to each other. As used herein, the term "CDR set" refers to the three CDRs of a heavy or light chain variable (V) region. Proceeding from the N-terminus of a heavy or light chain, these regions are denoted as "CDR1," "CDR2," and "CDR3" respectively. An antigen-binding site, therefore, may include six CDRs, comprising the CDR set from each of a heavy and a light chain V region. A polypeptide comprising a single CDR, (*e.g*., a CDR1, CDR2 or CDR3) is referred to herein as a "molecular recognition unit." Crystallographic analysis of a number of antigen-antibody complexes has demonstrated that the amino acid residues of CDRs form extensive contact with bound antigen, wherein the most extensive antigen contact is with the heavy chain CDR3. Thus, the molecular recognition units are primarily responsible for the specificity of an antigen-binding site.

A number of CDR delineations are known in the art and are encompassed herein. A person of skill in the art can readily determine a CDR for a given delineation based on the sequence of the heavy or light chain variable region. The "Kabat" Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). "Chothia" CDRs refer to the location of the structural loops (Chothia & Lesk, Canonical structures for the hypervariable regions of immunoglobulins, J. Mol. Biol., vol. 196, pp. 901-917 (1987)). The "AbM" CDRs represent a compromise between the Kabat CDRs and Chothia structural loops, and are used by Oxford Molecular's AbM antibody modeling software. The "Contact" CDRs are based on an analysis of the available complex crystal structures. The residues from each of these CDRs are noted below in Table 1, in reference to common antibody numbering schemes.

Unless otherwise specified herein, numbering of CDRs and FRs refers to the Kabat numbering scheme as described in Kabat et al., *supra,* including when CDR delineations are made in reference to Kabat, Chothia, AbM, or Contact schemes. Using this numbering system, the actual linear amino acid sequence may contain fewer or additional amino acids corresponding to a shortening of, or insertion into, a FR or CDR of the variable domain. For example, a heavy-chain variable domain may include a single amino acid insert (residue 52a according to Kabat) after residue 52 of H2 and inserted residues (e.g. residues 82a, 82b, and 82c, etc. according to Kabat) after heavy-chain FR residue 82. The Kabat numbering of residues may be determined for a given antibody by alignment at regions of homology of the sequence of the antibody with a "standard" Kabat numbered sequence.

**Table 1: CDR Delineations According to Various Schemes**

| **CDR** | **Kabat** | **AbM** | **Chothia** | **Contact** |
|---|---|---|---|---|
| VL-CDR1 | L24-L34 | L24-L34 | L26-L32 | L30-L36 |
| VL-CDR2 | L50-L56 | L50-L56 | L50-L52 | L46-L55 |
| VL-CDR3 | L89-L97 | L89-L97 | L91-L96 | L89-L96 |
| VH-CDR1 (Kabat Numbering) | H31-H35B | H26-H35B | H26-H32 | H30-H35B |
| VH-CDR1 (Chothia Numbering) | H31-H35 | H26-H35 | H26-H32 | H30-H35 |
| VH-CDR2 | H50-H65 | H50-H58 | H53-H55 | H47-H58 |
| VH-CDR3 | H95-H102 | H95-H102 | H95-H101 | H93-H101 |

In some embodiments, the CDRs are "extended CDRs," and encompass a region that begins or terminates according to a different scheme. For example, an extended CDR can be as follows: L24-L36, L26-L34, or L26-L36 (VL-CDR1), L46-L52, L46-L56, or L50-L55 (VL-CDR2), L91-L97 (VL-CDR3), H47-H55, H47-H65, H50-H55, H53-H58, or H53-H65 (VH-CDR2), and/or H93-H102 (VH-CDR3).

As used herein, the term "FR set" refers to the four flanking amino acid sequences which frame the CDRs of a CDR set of a heavy or light chain V region. Some FR residues may contact bound antigen; however, FRs are primarily responsible for folding the V region into the antigen-binding site, particularly the FR residues directly adjacent to the CDRs. That is, the variable light chain and variable heavy chain are arranged according to the formula FR1-CDR1-FR2-CDR2-FR3-CDR3-FR4, wherein the FRs are defined according to the CDRs juxtaposed between them according to the CDR delineations discussed above. Within FRs, certain amino residues and certain structural features are highly conserved. In this regard, all V region sequences contain an internal disulfide loop of around 90 amino acid residues. When the V regions fold into a binding-site, the CDRs are displayed as projecting loop motifs which form an antigen-binding surface. It is generally recognized that there are conserved structural regions of FRs which influence the folded shape of the CDR loops into certain "canonical" structures-regardless of the precise CDR amino acid sequence. Further, certain FR residues are known to participate in non-covalent interdomain contacts which stabilize the interaction of the antibody heavy and light chains. The structures and locations of immunoglobulin variable domains may be determined by reference to Kabat, E. A. et al., Sequences of Proteins of Immunological Interest. 4th Edition. US Department of Health and Human Services. 1987, and updates thereof, now available on the Internet (immuno.bme.nwu.edu).

The variable region for both light and heavy chains of the antibodies and the antigen-binding fragments thereof can include a framework from an immunoglobulin of any species. In some embodiments, the variable region for the light chain and/or the variable region for the heavy chain comprise a murine framework or a framework derived from a murine framework. In some embodiments, the variable region for the light chain and/or the variable region for the heavy chain comprise a human framework or a framework derived from a human framework. In some embodiments, the framework is or is derived from a consensus framework, for example as described in Kabat et al., supra. A consensus framework is a framework that represents the most commonly occurring amino acid residues in a selection of immunoglobulin variable light or variable heavy chain framework sequences. The framework can be selected from a subgroup of variable domain sequences. Light chain variable framework subgroups include kappa or lambda subgroups, such as kappa I, kappa II, kappa III, kappa IV. Subgroups for the heavy chain variable framework include subgroup I, subgroup II, and subgroup III. Alternatively, a consensus framework can be derived from the above in which particular residues, such as when a framework residue is selected based on its homology to the donor framework by aligning the donor framework sequence with a collection of various framework sequences. An acceptor human framework "derived from" an immunoglobulin framework or a consensus framework may comprise the same amino acid sequence thereof, or it may contain pre-existing amino acid sequence changes. In some embodiments, the number of pre-existing amino acid changes are 10 or less, 9 or less, 8 or less, 7 or less, 6 or less, 5 or less, 4 or less, 3 or less, or 2 or less.

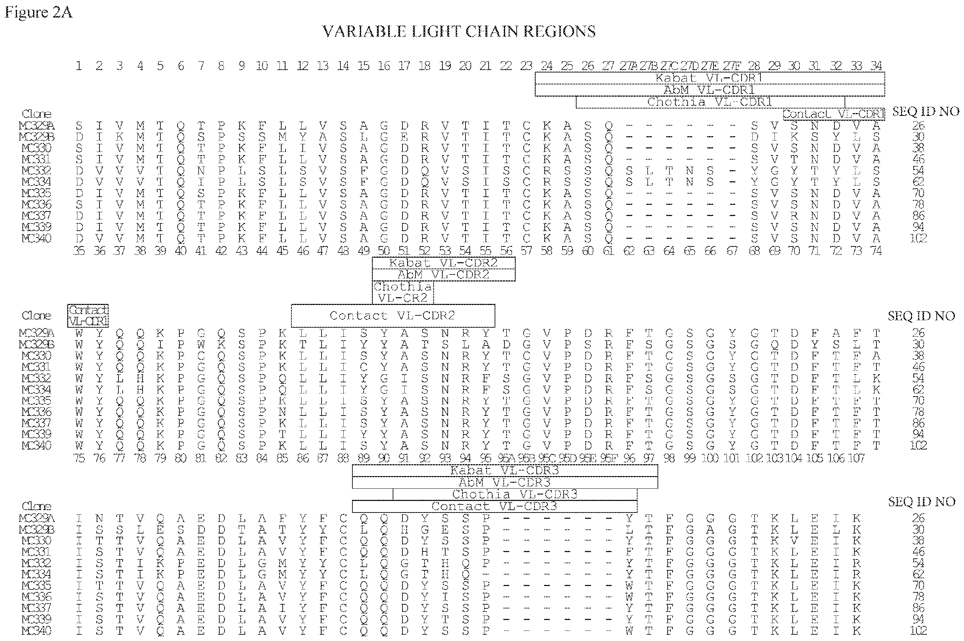

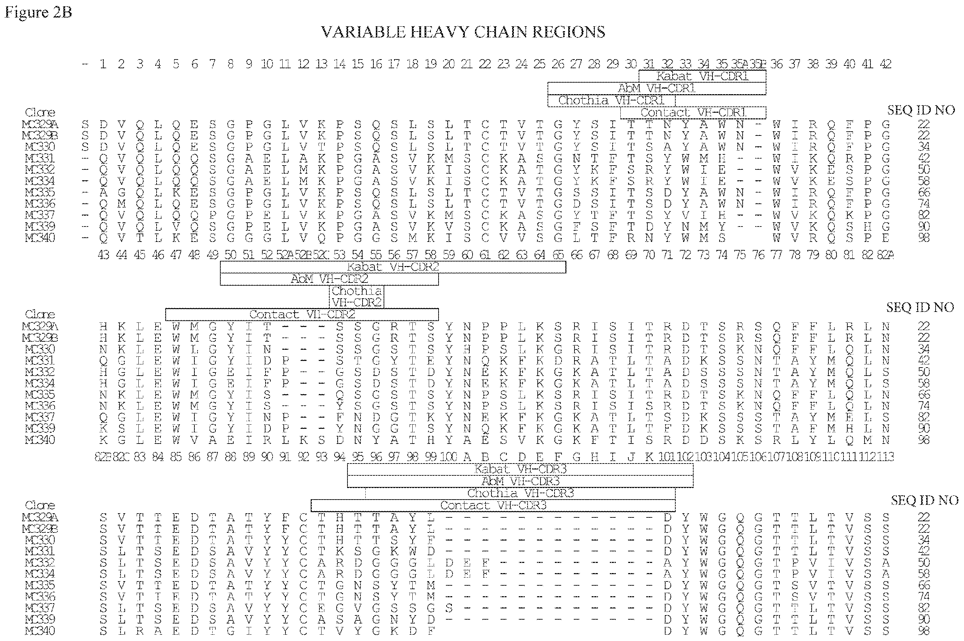

Variable region amino acid sequences of exemplary anti-plazomicin antibodies and CDR regions are shown in Table 2. In each amino acid sequence in Table 2, CDRs 1-3 on both the heavy and light chain are indicated by underlined text. Other CDR regions according to various CDR delineations are described in Table 3, in the sequence listing, and FIGS. 2A and 2B.

**Table 2: Exemplary anti-plazomicin antibody sequences**

| **Antibody** | **SEQ ID NO:** | **Chain** | **Amino acid sequence** |
|---|---|---|---|
| MC329-A/B | 22 | Heavy | |
| MC329-A | 26 | Light | |
| MC329-B | 30 | Light | |
| MC330 | 34 | Heavy | |
| MC330 | 38 | Light | |
| MC331 | 42 | Heavy | |
| MC331 | 46 | Light | |
| | | | |
| MC332 | 50 | Heavy | |
| MC332 | 54 | Light | |
| MC334 | 58 | Heavy | |
| MC334 | 62 | Light | |
| MC335 | 66 | Heavy | |
| MC335 | 70 | Light | |
| MC336 | 74 | Heavy | |
| MC336 | 78 | Light | |
| MC337 | 82 | Heavy | |
| MC337 | 86 | light | |
| MC339 | 90 | Heavy | |
| MC339 | 94 | Light | |
| MC340 | 98 | Heavy | |
| MC340 | 102 | Light | |
| | | | |

The term "antigen-binding fragment" as used herein refers to a polypeptide fragment that contains at least one CDR of an immunoglobulin heavy and/or light chain that binds to at least one epitope of the antigen of interest. In this regard, an antigen-binding fragment of the herein described antibodies may comprise 1, 2, 3, 4, 5, or all 6 CDRs of a variable heavy chain (VH) and variable light chain (VL) sequence from antibodies that bind plazomicin or a pharmaceutically acceptable salt thereof, set forth herein. An antigen-binding fragment of the plazomicin-specific antibodies described herein is capable of binding to plazomicin or a pharmaceutically acceptable salt thereof. Exemplary antigen-binding fragments include Fab, Fab', F(ab')₂ and Fv fragments; diabodies; linear antibodies (see U.S. Patent 5,641,870, Example 2; Zapata et al, Protein Eng. 8(10): 1057-1062 [1995]); single-chain antibody molecules, and multispecific antibodies formed from antibody fragments. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Each Fab fragment is monovalent with respect to antigen binding, i.e., it has a single antigen-binding site. Pepsin treatment of an antibody yields a single large F(ab')2 fragment which roughly corresponds to two disulfide linked Fab fragments having different antigen-binding activity and is still capable of cross-linking antigen. Fab' fragments differ from Fab fragments by having a few additional residues at the carboxy terminus of the CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The term "antigen" refers to a molecule or a portion of a molecule capable of being bound by a selective binding agent, such as an antibody, and additionally capable of being used in an animal to produce antibodies capable of binding to an epitope of that antigen. An antigen may have one or more epitopes. Teachings herein contemplate the use of plazomicin, or a pharmaceutically acceptable salt thereof, or plazomicin conjugated to a hapten, as an antigen.

The term "epitope" includes any determinant capable of specific binding to an immunoglobulin or T-cell receptor. An epitope is a region of an antigen that is bound by an antibody. In certain embodiments, epitope determinants include chemically active surface groupings of molecules such as amino acids, sugar side chains, phosphoryl or sulfonyl, and may in certain embodiments have specific three-dimensional structural characteristics, and/or specific charge characteristics. In certain embodiments, an antibody is said to specifically bind an antigen when it preferentially recognizes its target antigen in a complex mixture of proteins and/or macromolecules. An antibody is said to specifically bind an antigen when the equilibrium dissociation constant is ≤10⁻⁷ or 10⁻⁸ M. In some embodiments, the equilibrium dissociation constant may be ≤10⁻⁹ M or ≤10⁻¹⁰ M. In further embodiments, the equilibrium dissociation constant may be ≤10⁻¹¹ M or less.

An epitope that "specifically binds" or "preferentially binds" (used interchangeably herein) to an antibody or a polypeptide is a term well understood in the art. A molecule is said to exhibit "specific binding" or "preferential binding" if it reacts or associates more frequently, more rapidly, with greater duration and/or with greater affinity with a particular cell or substance than it does with alternative cells or substances. An antibody specifically binds or preferentially binds to a target if it binds with greater affinity, avidity, more readily, and/or with greater duration than it binds to other substances. For example, an antibody that specifically or preferentially binds to a plazomicin epitope is an antibody that binds one plazomicin epitope with greater affinity, avidity, more readily, and/or with greater duration than it binds to other plazomicin epitopes or non- plazomicin epitopes. It is also understood by reading this definition that specific binding or preferential binding does not necessarily require (although it can include) exclusive binding. Methods to determine such specific or preferential binding are also well known in the art, *e.g*., an immunoassay. In certain embodiments described herein, the anti-plazomicin antibodies specifically or preferentially bind to epitopes of plazomicin with greater affinity/avidity than to other aminoglycosides, such as amikacin, sisomicin, or gentamicin.

"Immunological binding" generally refers to the non-covalent interactions of the type which occur between an immunoglobulin molecule and an antigen for which the immunoglobulin is specific, for example by way of illustration and not limitation, as a result of electrostatic, ionic, hydrophilic and/or hydrophobic attractions or repulsion, steric forces, hydrogen bonding, van der Waals forces, and other interactions. The strength, or affinity of immunological binding interactions can be expressed in terms of the dissociation constant (K_{d}) of the interaction, wherein a smaller K_{d} represents a greater affinity. Immunological binding properties of selected polypeptides can be quantified using methods well known in the art. One such method entails measuring the rates of antigen-binding site/antigen complex formation and dissociation, wherein those rates depend on the concentrations of the complex partners, the affinity of the interaction, and on geometric parameters that equally influence the rate in both directions. Thus, both the "on rate constant" (Kₒₙ) and the "off rate constant" (K_{off}) can be determined by calculation of the concentrations and the actual rates of association and dissociation (See Nature 361:186-87 (1993)). The ratio of K_{off} /Kₒₙ enables cancellation of all parameters not related to affinity, and is thus equal to the dissociation constant K_{d} (See, generally, Davies et al. (1990) Annual Rev. Biochem. 59:439-473).

The term "immunologically active", with reference to an epitope or "remaining immunologically active," refers to the ability of an antibody (e.g., an anti-plazomicin antibody) to bind to the epitope under different conditions, for example, after the epitope has been subjected to reducing and denaturing conditions.

The term "F(ab)" refers to two of the protein fragments resulting from proteolytic cleavage of immunoglobulin G (IgG) molecules by the enzyme papain. Each F(ab) comprises a covalent heterodimer of the VH chain and VL chain and includes an intact antigen-binding site.

The term "F(ab)₂" refers to a protein fragment of IgG generated by proteolytic cleavage by the enzyme pepsin. Each F(ab')₂ fragment comprises two F(ab) fragments, thus comprising both antigen-binding sites.

An "Fv fragment" for use according to certain embodiments of the present invention can be produced by preferential proteolytic cleavage of an IgM, and on rare occasions of an IgG or IgA immunoglobulin molecule. Fv fragments are, however, more commonly derived using recombinant techniques known in the art. The Fv fragment includes a non-covalent VH::VL heterodimer including an antigen-binding site which retains much of the antigen recognition and binding capabilities of the native antibody molecule, but lacking the CH1 and CL domains contained within a Fab. Inbar et al. (1972) Proc. Nat. Acad. Sci. USA 69:2659-2662; Hochman et al. (1976) Biochem 15:2706-2710; and Ehrlich et al. (1980) Biochem 19:4091-4096.

In certain embodiments, single chain Fv (scFv) antibodies are contemplated and may be prepared using standard molecular biology techniques following the teachings of the present application with regard to selecting antibodies having the desired specificity.

In still further embodiments, chimeric antibodies may be made. For example, a chimeric antibody may comprise CDRs and framework regions from different antibodies. These antibodies may be produced through recombinant molecular biological techniques or may be physically conjugated together.

A scFv polypeptide is a covalently linked VH::VL heterodimer which is expressed from a gene fusion including VH- and VL-encoding genes linked by a peptide-encoding linker. Huston et al. (1988) Proc. Nat. Acad. Sci. USA 85(16):5879-5883. A number of methods have been described to discern chemical structures for converting the naturally aggregated-but chemically separated-light and heavy polypeptide chains from an antibody V region into an scFv molecule which will fold into a three dimensional structure substantially similar to the structure of an antigen-binding site. See, *e.g*., U.S. Pat. Nos. 5,091,513 and 5,132,405, to Huston et al.; and U.S. Pat. No. 4,946,778, to Ladner et al.

In certain embodiments, the antibodies described herein may be provided in the form of a UniBody^{®}. A UniBody^{®} is an IgG4 antibody with the hinge region removed (see GenMab Utrecht, The Netherlands; see also, *e.g*., US20090226421). This antibody technology creates a stable, smaller antibody format with an anticipated longer therapeutic window than current small antibody formats. IgG4 antibodies are considered inert and thus do not interact with the immune system. Fully human IgG4 antibodies may be modified by eliminating the hinge region of the antibody to obtain half-molecule fragments having distinct stability properties relative to the corresponding intact IgG4 (GenMab, Utrecht). Halving the IgG4 molecule leaves only one area on the UniBody^{®} that can bind to cognate antigens (*e.g*., disease targets) and the UniBody^{®} therefore binds univalently to only one site on target cells. For certain cancer cell surface antigens, this univalent binding may not stimulate the cancer cells to grow as may be seen using bivalent antibodies having the same antigen specificity, and hence UniBody^{®} technology may afford treatment options for some types of cancer that may be refractory to treatment with conventional antibodies. The small size of the UniBody^{®} can be a great benefit when treating some forms of cancer, allowing for better distribution of the molecule over larger solid tumors and potentially increasing efficacy.

In certain embodiments, the antibodies of the present disclosure may be chimeric antibodies. In this regard, a chimeric antibody is comprised of an antigen-binding fragment of an anti-plazomicin antibody operably linked or otherwise fused to a heterologous Fc portion of a different antibody. In certain embodiments, the heterologous Fc domain is of human origin. In further embodiments, the heterologous Fc domain may be from a different Ig class from the parent antibody, including IgA (including subclasses IgAl and IgA2), IgD, IgE, IgG (including subclasses IgG1, IgG2, IgG3, and IgG4), and IgM. In further embodiments, the heterologous Fc domain may be comprised of CH2 and CH3 domains from one or more of the different Ig classes. The anti-plazomicin antigen-binding fragment of a chimeric antibody may comprise only one or more of the CDRs of the antibodies described herein (*e.g*., 1, 2, 3, 4, 5, or 6 CDRs of the antibodies described herein), or may comprise an entire variable domain (VL, VH or both).

In certain embodiments, the antibodies of the present disclosure may be "non-naturally occurring" antibodies. Non-naturally occurring antibodies can refer to antibodies that comprise one or more amino acid modifications, such that the resultant antibody is substantially non-naturally occurring (*e.g*., does not exists in nature). These amino acid modifications can include point mutations, wherein a naturally occurring amino acid is substituted for another naturally occurring amino acid. In some embodiments, the amino acid modifications can include point mutations wherein a non-naturally occurring amino acid is substituted for a naturally occurring amino acid. Non-naturally occurring antibodies can also refer to antibodies that are conjugated to a heterologous protein or compound, such as a detectable marker.

A "monoclonal antibody" (mAb) refers to a homogeneous antibody population wherein the monoclonal antibody is comprised of amino acids (naturally occurring and non-naturally occurring) that are involved in the selective binding of an epitope. In particular, the CDRs of the monoclonal antibody are identical in all the molecules of the population. MAbs contain an antigen binding site capable of immunoreacting with a particular epitope of the antigen characterized by a unique binding affinity for it. The term "monoclonal antibody" encompasses not only intact monoclonal antibodies and full-length monoclonal antibodies, but also fragments thereof as described above.

In some embodiments, the antibody is a murine antibody. In some embodiments, the antibody or antigen binding fragment described herein is a human antibody, a chimeric antibody, or a humanized antibody. A chimeric antibody may comprise CDRs and framework regions from different antibodies. These antibodies may be produced through recombinant molecular biological techniques or may be physically conjugated together.

"Humanized" forms of non-human (e.g., murine) antibodies are chimeric antibodies that contain a portion of the sequence derived from non-human immunoglobulin and a portion derived from human immunoglobulin. In one embodiment, a humanized antibody is a human immunoglobulin (recipient antibody) in which residues from one or more CDRs of the recipient are replaced by residues from a CDR of a non-human species (donor antibody) such as mouse, rat, rabbit or non- human primate having the desired specificity, affinity, and/or capacity. In some instances, framework ("FR") residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity. In general, a humanized antibody will comprise substantially all of at least one, and typically two, variable domains, in which all or substantially all of the CDRs correspond to those of a non-human immunoglobulin sequence, and all or substantially all of the FR regions are those of a human immunoglobulin sequence, although the FR regions may include one or more individual FR residue substitutions that improve antibody performance, such as binding affinity, isomerization, immunogenicity, etc. The number of these amino acid substitutions in the FR is typically no more than 6 in the heavy chain and no more than 3 in the light chain. The humanized antibody optionally will also comprise at least a portion of an immunoglobulin constant region (Fc), typically that of a human immunoglobulin. For further details, see, e.g., Jones et al, Nature vol. 321, pp. 522-525 (1986); Riechmann et al, Nature vol. 332, pp. 323-329 (1988); and Presta, Curr. Op. Struct. Biol. vol. 2, pp. 593-596 (1992). See also, for example, Vaswani & Hamilton, Ann. Allergy, Asthma & Immunol., vol. 1, pp. 105-115 (1998); Harris, Biochem. Soc. Transactions vol. 23, pp. 1035-1038 (1995); Hurle & Gross, Curr. Op. Biotech., vol. 5, pp. 428-433 (1994); and U.S. Pat. Nos. 6,982,321 and 7,087,409.

In some aspects, the antibody or the antigen-binding fragments of the antibody is isolated. The isolated antibody or antigen-binding fragment thereof specifically binds plazomicin or a pharmaceutically acceptable salt thereof.

The present disclosure contemplates variants of the antibodies disclosed herein. In certain teachings, such variant antibodies or antigen-binding fragments, or CDRs thereof, bind to plazomicin at least about 50%, at least about 70%, at least about 80%, at least about 85%, at least about 90% and in certain teachings, at least about 95% as well as an antibody sequence specifically set forth herein. In further teachings, such variant antibodies or antigen-binding fragments, or CDRs thereof, bind to plazomicin with greater affinity than the antibodies set forth herein, for example, that bind quantitatively at least about 105%, 106%, 107%, 108%, 109%, or 110% as well as an antibody sequence specifically set forth herein.

In particular embodiments, a subject antibody may have: a) a heavy chain variable region having an amino acid sequence that is at least 80% identical, at least 85% identical, at least 90%, at least 95%, at least 96%, at least 97%, at least 98%, or 99% or 100% identical, to a heavy chain variable region of an anti-plazomicin antibody described herein; and b) a light chain variable region having an amino acid sequence that is at least 80% identical, at least 85%, at least 90%, at least 95%, at least 96%, at least 97%,at least 98%, or 99% or 100% identical, to a light chain variable region of an anti-plazomicin antibody described herein. Amino acid sequences of illustrative heavy chains are set forth in SEQ ID NOs: 22, 34, 42, 50, 58, 66, 74, 82, 90, and 98. Amino acid sequence of illustrative light chain regions are set forth in SEQ ID NOs: 26, 30, 38, 46, 54, 62, 70, 78, 86, 94, and 102.

In some embodiments, an isolated antibody or antigen binding fragment thereof comprises a heavy chain variable region having an amino acid sequence that is at least 80% identical, at least 85% identical, at least 90%, at least 95%, at least 96%, at least 97%, at least 98%, at least 99% or 100% identical to any of SEQ ID NOs: 22, 34, 42, 50, 58, 66, 74, 82, 90, or 98. In some embodiments, an isolated antibody or antigen binding fragment thereof comprises a light chain variable region having an amino acid sequence that is at least 80% identical, at least 85% identical, at least 90%, at least 95%, at least 96%, at least 97%, at least 98%, at least 99% or 100% identical to any of SEQ ID NOs: 26, 30, 38, 46, 54, 62, 70, 78, 86, 94, or 102. In some embodiments, an anti-plazomicin may have: a) a heavy chain variable region having an amino acid sequence that is at least 80% identical, at least 85% identical, at least 90%, at least 95%, at least 96%, at least 97%, at least 98%, or 99% or 100% identical to any of SEQ ID NOs: 22, 34, 42, 50, 58, 66, 74, 82, 90, or 98 and b) a light chain variable region having an amino acid sequence that is at least 80% identical, at least 85%, at least 90%, at least 95%, at least 98%, or 99% or 100% identical, to the light chain variable region of any of SEQ ID NOs: 26, 30, 38, 46, 54, 62, 70, 78, 86, 94, or 102. The antibody or antigen-binding fragment thereof specifically binds plazomicin or a pharmaceutically acceptable salt thereof.

In some embodiments, the antibody or antigen-binding fragment thereof comprises a light chain variable region comprising a VL-CDR1, a VL-CDR2, and/or a VL-CDR3 comprising an amino acid sequence having at least 80%, at least 85%, at least 90%, at least 92%, at least 95%, at least 96%, at least 97%, at least 98%, at least 99%, or 100% identity to any of the VL-CDR1s, VL-CDR2s, and/or VL-CDR3s described herein (e.g, according to Kabat, Chothia, AbM, Contact, extended, or any other scheme).The antibody or antigen-binding fragment thereof specifically binds plazomicin or a pharmaceutically acceptable salt thereof.

For example, in some embodiments, the isolated antibody or antigen binding fragment thereof comprises a VL-CDR1 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 27, 31, 39, 47, 55, 63, 71, 79, 87, 95, 103, 196, 197, 198, 203, 204, 205, 211, 212, 213, 218, 219, 220, 226, 227, 228, 233, 234, 235, 240, 241, 242, 247, 248, 249, 254, 255, 256, 261, 262, 263, 268, 269, or 270. In some embodiments, the antibody or antigen binding fragment thereof comprises a VL-CDR1 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 196, 203, 218, 226, or 254. The antibody or antigen-binding fragment thereof specifically binds plazomicin or a pharmaceutically acceptable salt thereof.

In some embodiments, the isolated antibody or antigen binding fragment thereof comprises a VL-CDR2 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 28, 32, 40, 48, 56, 64, 72, 80, 88, 96, 104, 199, 200, 206, 207, 214, 215, 221, 222, 229, 230, 236, 237, 243, 244, 250, 251, 257, 258, 264, 256, 271, or 272. In some embodiments, the antibody or antigen binding fragment thereof comprises a VL-CDR2 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 199, 206, or 229. The antibody or antigen-binding fragment thereof specifically binds plazomicin or a pharmaceutically acceptable salt thereof.

In some embodiments, the isolated antibody or antigen binding fragment thereof comprises a VL-CDR3 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 29, 33, 41, 49, 57, 65, 73, 81, 89, 97, 105, 201, 202, 208, 209, 210, 216, 217, 223, 224, 225, 231, 232, 238, 239, 245, 246, 252, 253, 259, 260, 266, 267, 273, or 274. In some embodiments, the antibody or antigen binding fragment thereof comprises a VL-CDR3 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 29, 57, 73, 81, 97, 208, or 223.The antibody or antigen-binding fragment thereof specifically binds plazomicin or a pharmaceutically acceptable salt thereof.

In some embodiments, the isolated antibody or antigen binding fragment includes a VL-CDR1, a VL-CDR2, and a VL-CDR3 according the above-described VL-CDR1s, VL-CDR2s, and VL-CDR3s. For example, in some embodiments, the isolated antibody or antigen-binding fragment thereof comprises a variable light chain comprising a VL-CDR1 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 27, 31, 39, 47, 55, 63, 71, 79, 87, 95, 103, 196, 197, 198, 203, 204, 205, 211, 212, 213, 218, 219, 220, 226, 227, 228, 233, 234, 235, 240, 241, 242, 247, 248, 249, 254, 255, 256, 261, 262, 263, 268, 269, or 270; a VL-CDR2 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 28, 32, 40, 48, 56, 64, 72, 80, 88, 96, 104, 199, 200, 206, 207, 214, 215, 221, 222, 229, 230, 236, 237, 243, 244, 250, 251, 257, 258, 264, 256, 271, or 272; and a VL-CDR3 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 29, 33, 41, 49, 57, 65, 73, 81, 89, 97, 105, 201, 202, 208, 209, 210, 216, 217, 223, 224, 225, 231, 232, 238, 239, 245, 246, 252, 253, 259, 260, 266, 267, 273, or 274. The antibody or antigen-binding fragment thereof specifically binds plazomicin or a pharmaceutically acceptable salt thereof.

The isolated antibody or antigen-binding fragment thereof comprises a variable light chain comprising a VL-CDR1 comprising the amino acid sequence selected from any one of SEQ ID NOs: 196, 203, 218, 226, or 254; a VL-CDR2 comprising the amino acid sequence selected from any one of SEQ ID NOs: 199, 206, or 229; and a VL-CDR3 comprising the amino acid sequence selected from any one of SEQ ID NOs: 29, 57, 73, 81, 97, 208, or 223.The antibody or antigen-binding fragment thereof specifically binds plazomicin or a pharmaceutically acceptable salt thereof.

In some embodiments, the isolated antibody or antigen-binding fragment thereof comprises a light chain variable region comprising (a) a VL-CDR1 comprising the amino acid sequence of SEQ ID NO: 196; a VL-CDR2 comprising the amino acid sequence of SEQ ID NO: 199; and a VL-CDR3 comprising the amino acid sequence of SEQ ID NO: 29; (b) a VL-CDR1 comprising the amino acid sequence of SEQ ID NO: 203; a VL-CDR2 comprising the amino acid sequence of SEQ ID NO: 206; and a VL-CDR3 comprising the amino acid sequence of SEQ ID NO: 208; (c) a VL-CDR1 comprising the amino acid sequence of SEQ ID NO: 218; a VL-CDR2 comprising the amino acid sequence of SEQ ID NO: 221; and a VL-CDR3 comprising the amino acid sequence of SEQ ID NO: 223; (d) a VL-CDR1 comprising the amino acid sequence of SEQ ID NO: 226; a VL-CDR2 comprising the amino acid sequence of SEQ ID NO: 229; and a VL-CDR3 comprising the amino acid sequence of SEQ ID NO: 57; (e) a VL-CDR1 comprising the amino acid sequence of SEQ ID NO: 240; a VL-CDR2 comprising the amino acid sequence of SEQ ID NO: 243; and a VL-CDR3 comprising the amino acid sequence of SEQ ID NO: 73; (f) a VL-CDR1 comprising the amino acid sequence of SEQ ID NO: 247; a VL-CDR2 comprising the amino acid sequence of SEQ ID NO: 250; and a VL-CDR3 comprising the amino acid sequence of SEQ ID NO: 81; (g) a VL-CDR1 comprising the amino acid sequence of SEQ ID NO: 254; a VL-CDR2 comprising the amino acid sequence of SEQ ID NO: 257; and a VL-CDR3 comprising the amino acid sequence of SEQ ID NO: 89; or (h) a VL-CDR1 comprising the amino acid sequence of SEQ ID NO: 261; a VL-CDR2 comprising the amino acid sequence of SEQ ID NO: 264; and a VL-CDR3 comprising the amino acid sequence of SEQ ID NO: 97. The antibody or antigen-binding fragment thereof specifically binds plazomicin or a pharmaceutically acceptable salt thereof.

In some embodiments, the isolated antibody or antigen-binding fragment thereof comprises a light chain variable region comprising (a) a VL-CDR1 comprising the amino acid sequence of SEQ ID NO: 27; a VL-CDR2 comprising the amino acid sequence of SEQ ID NO: 28; and a VL-CDR3 comprising the amino acid sequence of SEQ ID NO: 29; (b) a VL-CDR1 comprising the amino acid sequence of SEQ ID NO: 31; a VL-CDR2 comprising the amino acid sequence of SEQ ID NO: 32; and a VL-CDR3 comprising the amino acid sequence of SEQ ID NO: 33; (c) a VL-CDR1 comprising the amino acid sequence of SEQ ID NO: 39; a VL-CDR2 comprising the amino acid sequence of SEQ ID NO: 40; and a VL-CDR3 comprising the amino acid sequence of SEQ ID NO: 41; (d) a VL-CDR1 comprising the amino acid sequence of SEQ ID NO: 47; a VL-CDR2 comprising the amino acid sequence of SEQ ID NO: 48; and a VL-CDR3 comprising the amino acid sequence of SEQ ID NO: 49; (e) a VL-CDR1 comprising the amino acid sequence of SEQ ID NO: 55; a VL-CDR2 comprising the amino acid sequence of SEQ ID NO: 56; and a VL-CDR3 comprising the amino acid sequence of SEQ ID NO: 57; (f) a VL-CDR1 comprising the amino acid sequence of SEQ ID NO: 63; a VL-CDR2 comprising the amino acid sequence of SEQ ID NO: 64; and a VL-CDR3 comprising the amino acid sequence of SEQ ID NO: 65; (g) a VL-CDR1 comprising the amino acid sequence of SEQ ID NO: 71; a VL-CDR2 comprising the amino acid sequence of SEQ ID NO: 72; and a VL-CDR3 comprising the amino acid sequence of SEQ ID NO: 73; or (h) a VL-CDR1 comprising the amino acid sequence of SEQ ID NO: 79; a VL-CDR2 comprising the amino acid sequence of SEQ ID NO: 80; and a VL-CDR3 comprising the amino acid sequence of SEQ ID NO: 81; (i) a VL-CDR1 comprising the amino acid sequence of SEQ ID NO: 87; a VL-CDR2 comprising the amino acid sequence of SEQ ID NO: 88; and a VL-CDR3 comprising the amino acid sequence of SEQ ID NO: 89; (j) a VL-CDR1 comprising the amino acid sequence of SEQ ID NO: 95; a VL-CDR2 comprising the amino acid sequence of SEQ ID NO: 96; and a VL-CDR3 comprising the amino acid sequence of SEQ ID NO: 97; or (k) a VL-CDR1 comprising the amino acid sequence of SEQ ID NO: 103; a VL-CDR2 comprising the amino acid sequence of SEQ ID NO: 104; and a VL-CDR3 comprising the amino acid sequence of SEQ ID NO: 105. The antibody or antigen-binding fragment thereof specifically binds plazomicin or a pharmaceutically acceptable salt thereof.

In some embodiments, the isolated antibody or antigen-binding fragment thereof comprises a light chain variable region comprising (a) a VL-CDR1 comprising the amino acid sequence of SEQ ID NO: 197; a VL-CDR2 comprising the amino acid sequence of SEQ ID NO: 28; and a VL-CDR3 comprising the amino acid sequence of SEQ ID NO: 201; (b) a VL-CDR1 comprising the amino acid sequence of SEQ ID NO: 204; a VL-CDR2 comprising the amino acid sequence of SEQ ID NO: 32; and a VL-CDR3 comprising the amino acid sequence of SEQ ID NO: 209; (c) a VL-CDR1 comprising the amino acid sequence of SEQ ID NO: 212; a VL-CDR2 comprising the amino acid sequence of SEQ ID NO: 40; and a VL-CDR3 comprising the amino acid sequence of SEQ ID NO: 216; (d) a VL-CDR1 comprising the amino acid sequence of SEQ ID NO: 219; a VL-CDR2 comprising the amino acid sequence of SEQ ID NO: 48; and a VL-CDR3 comprising the amino acid sequence of SEQ ID NO: 224; (e) a VL-CDR1 comprising the amino acid sequence of SEQ ID NO: 227; a VL-CDR2 comprising the amino acid sequence of SEQ ID NO: 56; and a VL-CDR3 comprising the amino acid sequence of SEQ ID NO: 231; (f) a VL-CDR1 comprising the amino acid sequence of SEQ ID NO: 234; a VL-CDR2 comprising the amino acid sequence of SEQ ID NO: 64; and a VL-CDR3 comprising the amino acid sequence of SEQ ID NO: 238; (g) a VL-CDR1 comprising the amino acid sequence of SEQ ID NO: 241; a VL-CDR2 comprising the amino acid sequence of SEQ ID NO: 72; and a VL-CDR3 comprising the amino acid sequence of SEQ ID NO: 245; or (h) a VL-CDR1 comprising the amino acid sequence of SEQ ID NO: 248; a VL-CDR2 comprising the amino acid sequence of SEQ ID NO: 80; and a VL-CDR3 comprising the amino acid sequence of SEQ ID NO: 252; (i) a VL-CDR1 comprising the amino acid sequence of SEQ ID NO: 255; a VL-CDR2 comprising the amino acid sequence of SEQ ID NO: 88; and a VL-CDR3 comprising the amino acid sequence of SEQ ID NO: 259; (j) a VL-CDR1 comprising the amino acid sequence of SEQ ID NO: 262; a VL-CDR2 comprising the amino acid sequence of SEQ ID NO: 96; and a VL-CDR3 comprising the amino acid sequence of SEQ ID NO: 266; or (k) a VL-CDR1 comprising the amino acid sequence of SEQ ID NO: 269; a VL-CDR2 comprising the amino acid sequence of SEQ ID NO: 104; and a VL-CDR3 comprising the amino acid sequence of SEQ ID NO: 273. The antibody or antigen-binding fragment thereof specifically binds plazomicin or a pharmaceutically acceptable salt thereof.

In some embodiments, the isolated antibody or antigen-binding fragment thereof comprises a light chain variable region comprising (a) a VL-CDR1 comprising the amino acid sequence of SEQ ID NO: 198; a VL-CDR2 comprising the amino acid sequence of SEQ ID NO: 200; and a VL-CDR3 comprising the amino acid sequence of SEQ ID NO: 202; (b) a VL-CDR1 comprising the amino acid sequence of SEQ ID NO: 205; a VL-CDR2 comprising the amino acid sequence of SEQ ID NO: 207; and a VL-CDR3 comprising the amino acid sequence of SEQ ID NO: 210; (c) a VL-CDR1 comprising the amino acid sequence of SEQ ID NO: 213; a VL-CDR2 comprising the amino acid sequence of SEQ ID NO: 215; and a VL-CDR3 comprising the amino acid sequence of SEQ ID NO: 217; (d) a VL-CDR1 comprising the amino acid sequence of SEQ ID NO: 220; a VL-CDR2 comprising the amino acid sequence of SEQ ID NO: 222; and a VL-CDR3 comprising the amino acid sequence of SEQ ID NO: 225; (e) a VL-CDR1 comprising the amino acid sequence of SEQ ID NO: 228; a VL-CDR2 comprising the amino acid sequence of SEQ ID NO: 230; and a VL-CDR3 comprising the amino acid sequence of SEQ ID NO: 232; (f) a VL-CDR1 comprising the amino acid sequence of SEQ ID NO: 235; a VL-CDR2 comprising the amino acid sequence of SEQ ID NO: 237; and a VL-CDR3 comprising the amino acid sequence of SEQ ID NO: 239; (g) a VL-CDR1 comprising the amino acid sequence of SEQ ID NO: 242; a VL-CDR2 comprising the amino acid sequence of SEQ ID NO: 244; and a VL-CDR3 comprising the amino acid sequence of SEQ ID NO: 246; or (h) a VL-CDR1 comprising the amino acid sequence of SEQ ID NO: 249; a VL-CDR2 comprising the amino acid sequence of SEQ ID NO: 251; and a VL-CDR3 comprising the amino acid sequence of SEQ ID NO: 253; (i) a VL-CDR1 comprising the amino acid sequence of SEQ ID NO: 256; a VL-CDR2 comprising the amino acid sequence of SEQ ID NO: 258; and a VL-CDR3 comprising the amino acid sequence of SEQ ID NO: 260; (j) a VL-CDR1 comprising the amino acid sequence of SEQ ID NO: 263; a VL-CDR2 comprising the amino acid sequence of SEQ ID NO: 265; and a VL-CDR3 comprising the amino acid sequence of SEQ ID NO: 267; or (k) a VL-CDR1 comprising the amino acid sequence of SEQ ID NO: 270; a VL-CDR2 comprising the amino acid sequence of SEQ ID NO: 272; and a VL-CDR3 comprising the amino acid sequence of SEQ ID NO: 274.The antibody or antigen-binding fragment thereof specifically binds plazomicin or a pharmaceutically acceptable salt thereof.

In some embodiments, the isolated antibody or antigen-binding fragment thereof comprises a heavy chain variable region comprising a VH-CDR1, a VH-CDR2, and/or a VH-CDR3 comprising an amino acid sequence having at least 80%, at least 85%, at least 90%, at least 92%, at least 95%, at least 96%, at least 97%, at least 98%, at least 99%, or 100% identity to any of the VH-CDR1s, VH-CDR2s, and/or VH-CDR3s described herein (e.g, according to Kabat, Chothia, AbM, Contact, extended, or any other scheme). The antibody or antigen-binding fragment thereof specifically binds plazomicin or a pharmaceutically acceptable salt thereof.

For example, in some embodiments, the isolated antibody or antigen binding fragment thereof comprises a VH-CDR1 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 23, 35, 43, 51, 59, 67, 75, 83, 91, 99, 106, 107, 108, 115, 116, 117, 124, 125, 126, 133, 134, 135, 142, 143, 144, 151, 152, 153, 160, 160, 162, 169, 170, 171, 178, 17, 180, 187, 188, or 189. In some embodiments, the antibody or antigen binding fragment thereof comprises a VH-CDR1 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 106, 115, 124, 133, 151, 169, 178, or 187. The antibody or antigen-binding fragment thereof specifically binds plazomicin or a pharmaceutically acceptable salt thereof.

In some embodiments, the isolated antibody or antigen binding fragment thereof comprises a VH-CDR2 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 24, 36, 44, 52, 60, 68, 76, 84, 92, 100, 109, 110, 111, 118, 119, 120, 127, 128, 129, 136, 137, 138, 145, 146, 147, 154, 155, 156, 163, 164, 165, 172, 173, 174, 181, 182, 183, 190, 191, or 192. In some embodiments, the antibody or antigen binding fragment thereof comprises a VH-CDR2 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 109, 118, 127, 136, 154, 163, 172, 181, or 190. The antibody or antigen-binding fragment thereof specifically binds plazomicin or a pharmaceutically acceptable salt thereof.

In some embodiments, the isolated antibody or antigen binding fragment thereof comprises a VH-CDR3 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 25, 37, 45, 53, 61, 69, 77, 85, 93, 101, 112, 113, 114, 121, 122, 123, 130, 131, 132, 139, 140, 141, 148, 149, 150, 157, 158, 159, 166, 167, 168, 175, 176, 177, 184, 185, 186, 193, 194 or 195. In some embodiments, the antibody or antigen binding fragment thereof comprises a VH-CDR3 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 112, 121, 130 ,139, 157, 175, 184, or 193. The antibody or antigen-binding fragment thereof specifically binds plazomicin or a pharmaceutically acceptable salt thereof.

In some embodiments, the isolated antibody or antigen-binding fragment thereof comprises a heavy chain variable region comprising a VH-CDR1, VH-CDR2, and a VH-CDR3 according to any of the VH-CDR1s, VH-CDR2s, and VH-CDR3s described above. For example, in some embodiments, the isolated antibody or antigen-binding fragment thereof comprises a heavy chain variable region comprising a VH-CDR1 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 23, 35, 43, 51, 59, 67, 75, 83, 91, 99, 106, 107, 108, 115, 116, 117, 124, 125, 126, 133, 134, 135, 142, 143, 144, 151, 152, 153, 160, 160, 162, 169, 170, 171, 178, 17, 180, 187, 188, or 189; a VH-CDR2 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 24, 36, 44, 52, 60, 68, 76, 84, 92, 100, 109, 110, 111, 118, 119, 120, 127, 128, 129, 136, 137, 138, 145, 146, 147, 154, 155, 156, 163, 164, 165, 172, 173, 174, 181, 182, 183, 190, 191, or 192; and a VH-CDR3 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 25, 37, 45, 53, 61, 69, 77, 85, 93, 101, 112, 113, 114, 121, 122, 123, 130, 131, 132, 139, 140, 141, 148, 149, 150, 157, 158, 159, 166, 167, 168, 175, 176, 177, 184, 185, 186, 193, 194 or 195. In some embodiments, the isolated antibody or antigen-binding fragment thereof comprises a heavy chain variable region comprising a VH-CDR1 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 106, 115, 124, 133, 151, 169, 178, or 187; a VH-CDR2 comprising an amino acid sequence having at least 80%, at least 85%, at least 90%, at least 92%, at least 95%, at least 96%, at least 97%, at least 98%, at least 99%, or 100% identity to theamino acid sequence selected from any one of SEQ ID NOs: 109, 118, 127, 136, 154, 163, 172, 181, or 190; and a VH-CDR3 comprising an amino acid sequence having at least 80%, at least 85%, at least 90%, at least 92%, at least 95%, at least 96%, at least 97%, at least 98%, at least 99%, or 100% identity to the amino acid sequence selected from any one of SEQ ID NOs: 112, 121, 130 ,139, 157, 175, 184, or 193. The antibody or antigen-binding fragment thereof specifically binds plazomicin or a pharmaceutically acceptable salt thereof.

In some embodiments, the isolated antibody or antigen-binding fragment thereof comprises a heavy chain variable region comprising: (a) a VH-CDR1 comprising the amino acid sequence of SEQ ID NO: 106; a VH-CDR2 comprising the amino acid sequence of SEQ ID NO: 109; and a VH-CDR3 comprising the amino acid sequence of SEQ ID NO: 112; (b) a VH-CDR1 comprising the amino acid sequence of SEQ ID NO: 115; a VH-CDR2 comprising the amino acid sequence of SEQ ID NO: 118; and a VH-CDR3 comprising the amino acid sequence of SEQ ID NO: 121; (c) a VH-CDR1 comprising the amino acid sequence of SEQ ID NO: 124; a VH-CDR2 comprising the amino acid sequence of SEQ ID NO: 127; and a VH-CDR3 comprising the amino acid sequence of SEQ ID NO: 130; (d) a VH-CDR1 comprising the amino acid sequence of SEQ ID NO: 133; a VH-CDR2 comprising the amino acid sequence of SEQ ID NO: 136; and a VH-CDR3 comprising the amino acid sequence of SEQ ID NO: 139; (e) a VH-CDR1 comprising the amino acid sequence of SEQ ID NO: 151; a VH-CDR2 comprising the amino acid sequence of SEQ ID NO: 154; and a VH-CDR3 comprising the amino acid sequence of SEQ ID NO: 157; (f) a VH-CDR1 comprising the amino acid sequence of SEQ ID NO: 160; a VH-CDR2 comprising the amino acid sequence of SEQ ID NO: 163; and a VH-CDR3 comprising the amino acid sequence of SEQ ID NO: 166; (g) a VH-CDR1 comprising the amino acid sequence of SEQ ID NO: 169; a VH-CDR2 comprising the amino acid sequence of SEQ ID NO: 172; and a VH-CDR3 comprising the amino acid sequence of SEQ ID NO: 175; (h) a VH-CDR1 comprising the amino acid sequence of SEQ ID NO: 178; a VH-CDR2 comprising the amino acid sequence of SEQ ID NO: 181; and a VH-CDR3 comprising the amino acid sequence of SEQ ID NO: 184; or (i) a VH-CDR1 comprising the amino acid sequence of SEQ ID NO: 187; a VH-CDR2 comprising the amino acid sequence of SEQ ID NO: 190; and a VH-CDR3 comprising the amino acid sequence of SEQ ID NO: 193. The antibody or antigen-binding fragment thereof specifically binds plazomicin or a pharmaceutically acceptable salt thereof.

In some embodiments, the isolated antibody or antigen-binding fragment thereof comprises a heavy chain variable region comprising: (a) a VH-CDR1 comprising the amino acid sequence of SEQ ID NO: 23; a VH-CDR2 comprising the amino acid sequence of SEQ ID NO: 24; and a VH-CDR3 comprising the amino acid sequence of SEQ ID NO: 25; (b) a VH-CDR1 comprising the amino acid sequence of SEQ ID NO: 35; a VH-CDR2 comprising the amino acid sequence of SEQ ID NO: 36; and a VH-CDR3 comprising the amino acid sequence of SEQ ID NO: 37; (c) a VH-CDR1 comprising the amino acid sequence of SEQ ID NO: 43; a VH-CDR2 comprising the amino acid sequence of SEQ ID NO: 44; and a VH-CDR3 comprising the amino acid sequence of SEQ ID NO: 45; (d) a VH-CDR1 comprising the amino acid sequence of SEQ ID NO: 51; a VH-CDR2 comprising the amino acid sequence of SEQ ID NO: 52; and a VH-CDR3 comprising the amino acid sequence of SEQ ID NO: 53; (e) a VH-CDR1 comprising the amino acid sequence of SEQ ID NO: 59; a VH-CDR2 comprising the amino acid sequence of SEQ ID NO: 60; and a VH-CDR3 comprising the amino acid sequence of SEQ ID NO: 61; (f) a VH-CDR1 comprising the amino acid sequence of SEQ ID NO: 67; a VH-CDR2 comprising the amino acid sequence of SEQ ID NO: 68; and a VH-CDR3 comprising the amino acid sequence of SEQ ID NO: 69; (g) a VH-CDR1 comprising the amino acid sequence of SEQ ID NO: 75; a VH-CDR2 comprising the amino acid sequence of SEQ ID NO: 76; and a VH-CDR3 comprising the amino acid sequence of SEQ ID NO: 77; (h) a VH-CDR1 comprising the amino acid sequence of SEQ ID NO: 83; a VH-CDR2 comprising the amino acid sequence of SEQ ID NO: 84; and a VH-CDR3 comprising the amino acid sequence of SEQ ID NO: 85; (i) a VH-CDR1 comprising the amino acid sequence of SEQ ID NO: 91; a VH-CDR2 comprising the amino acid sequence of SEQ ID NO: 92; and a VH-CDR3 comprising the amino acid sequence of SEQ ID NO: 93; or (j) a VH-CDR1 comprising the amino acid sequence of SEQ ID NO: 99; a VH-CDR2 comprising the amino acid sequence of SEQ ID NO: 100; and a VH-CDR3 comprising the amino acid sequence of SEQ ID NO: 101. The antibody or antigen-binding fragment thereof specifically binds plazomicin or a pharmaceutically acceptable salt thereof.

In some embodiments, the isolated antibody or antigen-binding fragment thereof comprises a heavy chain variable region comprising: (a) a VH-CDR1 comprising the amino acid sequence of SEQ ID NO: 107; a VH-CDR2 comprising the amino acid sequence of SEQ ID NO: 110; and a VH-CDR3 comprising the amino acid sequence of SEQ ID NO: 113; (b) a VH-CDR1 comprising the amino acid sequence of SEQ ID NO: 116; a VH-CDR2 comprising the amino acid sequence of SEQ ID NO: 119; and a VH-CDR3 comprising the amino acid sequence of SEQ ID NO: 122; (c) a VH-CDR1 comprising the amino acid sequence of SEQ ID NO: 125; a VH-CDR2 comprising the amino acid sequence of SEQ ID NO: 128; and a VH-CDR3 comprising the amino acid sequence of SEQ ID NO: 131; (d) a VH-CDR1 comprising the amino acid sequence of SEQ ID NO: 134; a VH-CDR2 comprising the amino acid sequence of SEQ ID NO: 137; and a VH-CDR3 comprising the amino acid sequence of SEQ ID NO: 140; (e) a VH-CDR1 comprising the amino acid sequence of SEQ ID NO: 143; a VH-CDR2 comprising the amino acid sequence of SEQ ID NO: 146; and a VH-CDR3 comprising the amino acid sequence of SEQ ID NO: 149; (f) a VH-CDR1 comprising the amino acid sequence of SEQ ID NO: 152; a VH-CDR2 comprising the amino acid sequence of SEQ ID NO: 155; and a VH-CDR3 comprising the amino acid sequence of SEQ ID NO: 158; (g) a VH-CDR1 comprising the amino acid sequence of SEQ ID NO: 161; a VH-CDR2 comprising the amino acid sequence of SEQ ID NO: 164; and a VH-CDR3 comprising the amino acid sequence of SEQ ID NO: 167; (h) a VH-CDR1 comprising the amino acid sequence of SEQ ID NO: 170; a VH-CDR2 comprising the amino acid sequence of SEQ ID NO: 173; and a VH-CDR3 comprising the amino acid sequence of SEQ ID NO: 176; (i) a VH-CDR1 comprising the amino acid sequence of SEQ ID NO: 179; a VH-CDR2 comprising the amino acid sequence of SEQ ID NO: 182; and a VH-CDR3 comprising the amino acid sequence of SEQ ID NO: 185; or (j) a VH-CDR1 comprising the amino acid sequence of SEQ ID NO: 188; a VH-CDR2 comprising the amino acid sequence of SEQ ID NO: 191; and a VH-CDR3 comprising the amino acid sequence of SEQ ID NO: 194. The antibody or antigen-binding fragment thereof specifically binds plazomicin or a pharmaceutically acceptable salt thereof.

In some embodiments, the isolated antibody or antigen-binding fragment thereof comprises a heavy chain variable region comprising: (a) a VH-CDR1 comprising the amino acid sequence of SEQ ID NO: 108; a VH-CDR2 comprising the amino acid sequence of SEQ ID NO: 111; and a VH-CDR3 comprising the amino acid sequence of SEQ ID NO: 114; (b) a VH-CDR1 comprising the amino acid sequence of SEQ ID NO: 117; a VH-CDR2 comprising the amino acid sequence of SEQ ID NO: 120; and a VH-CDR3 comprising the amino acid sequence of SEQ ID NO: 123; (c) a VH-CDR1 comprising the amino acid sequence of SEQ ID NO: 126; a VH-CDR2 comprising the amino acid sequence of SEQ ID NO: 129; and a VH-CDR3 comprising the amino acid sequence of SEQ ID NO: 132; (d) a VH-CDR1 comprising the amino acid sequence of SEQ ID NO: 135; a VH-CDR2 comprising the amino acid sequence of SEQ ID NO: 138; and a VH-CDR3 comprising the amino acid sequence of SEQ ID NO: 141; (e) a VH-CDR1 comprising the amino acid sequence of SEQ ID NO: 144; a VH-CDR2 comprising the amino acid sequence of SEQ ID NO: 147; and a VH-CDR3 comprising the amino acid sequence of SEQ ID NO: 150; (f) a VH-CDR1 comprising the amino acid sequence of SEQ ID NO: 153; a VH-CDR2 comprising the amino acid sequence of SEQ ID NO: 156; and a VH-CDR3 comprising the amino acid sequence of SEQ ID NO: 159; (g) a VH-CDR1 comprising the amino acid sequence of SEQ ID NO: 162; a VH-CDR2 comprising the amino acid sequence of SEQ ID NO: 165; and a VH-CDR3 comprising the amino acid sequence of SEQ ID NO: 168; (h) a VH-CDR1 comprising the amino acid sequence of SEQ ID NO: 171; a VH-CDR2 comprising the amino acid sequence of SEQ ID NO: 174; and a VH-CDR3 comprising the amino acid sequence of SEQ ID NO: 177; (i) a VH-CDR1 comprising the amino acid sequence of SEQ ID NO: 180; a VH-CDR2 comprising the amino acid sequence of SEQ ID NO: 183; and a VH-CDR3 comprising the amino acid sequence of SEQ ID NO: 186; or (j) a VH-CDR1 comprising the amino acid sequence of SEQ ID NO: 189; a VH-CDR2 comprising the amino acid sequence of SEQ ID NO: 192; and a VH-CDR3 comprising the amino acid sequence of SEQ ID NO: 195. The antibody or antigen-binding fragment thereof specifically binds plazomicin or a pharmaceutically acceptable salt thereof.

In some embodiments, the isolated antibody or antigen binding fragment thereof comprises a VH-CDR1, a VH-CDR2, a VH-CDR3, a VL-CDR1, a VL-CDR2, and a VL-CDR3 according to any one of the VH-CDR1s, VH-CDR2s, VH-CDR3s, VL-CDR1s, VL-CDR2s, and VL-CDR3s described above. For example, in some embodiments, the isolated antibody or antigen binding fragment thereof comprises a light chain variable region comprising a VL-CDR1 comprising the amino acid sequence selected from any one of SEQ ID NOs: 27, 31, 39, 47, 55, 63, 71, 79, 87, 95, 103, 196, 197, 198, 203, 204, 205, 211, 212, 213, 218, 219, 220, 226, 227, 228, 233, 234, 235, 240, 241, 242, 247, 248, 249, 254, 255, 256, 261, 262, 263, 268, 269, or 270; a VL-CDR2 comprising the amino acid sequence selected from any one of SEQ ID NOs: 28, 32, 40, 48, 56, 64, 72, 80, 88, 96, 104, 199, 200, 206, 207, 214, 215, 221, 222, 229, 230, 236, 237, 243, 244, 250, 251, 257, 258, 264, 256, 271, or 272; and a VL-CDR3 comprising the amino acid sequence selected from any one of SEQ ID NOs: 29, 33, 41, 49, 57, 65, 73, 81, 89, 97, 105, 201, 202, 208, 209, 210, 216, 217, 223, 224, 225, 231, 232, 238, 239, 245, 246, 252, 253, 259, 260, 266, 267, 273, or 274; and a heavy chain variable region comprising a VH-CDR1 comprising the amino acid sequence selected from any one of SEQ ID NOs: 23, 35, 43, 51, 59, 67, 75, 83, 91, 99, 106, 107, 108, 115, 116, 117, 124, 125, 126, 133, 134, 135, 142, 143, 144, 151, 152, 153, 160, 160, 162, 169, 170, 171, 178, 17, 180, 187, 188, or 189; a VH-CDR2 comprising the amino acid sequence selected from any one of SEQ ID NOs: 24, 36, 44, 52, 60, 68, 76, 84, 92, 100, 109, 110, 111, 118, 119, 120, 127, 128, 129, 136, 137, 138, 145, 146, 147, 154, 155, 156, 163, 164, 165, 172, 173, 174, 181, 182, 183, 190, 191, or 192; and a VH-CDR3 comprising the amino acid sequence selected from any one of SEQ ID NOs: 25, 37, 45, 53, 61, 69, 77, 85, 93, 101, 112, 113, 114, 121, 122, 123, 130, 131, 132, 139, 140, 141, 148, 149, 150, 157, 158, 159, 166, 167, 168, 175, 176, 177, 184, 185, 186, 193, 194 or 195. In some embodiments, the antibody or antigen-binding fragment thereof specifically binds plazomicin or a pharmaceutically acceptable salt thereof.

In some embodiments, the isolated antibody or antigen binding fragment thereof comprises a light chain variable region comprising (i) a VH-CDR1 comprising the amino acid sequence selected from any one of SEQ ID NOs: 106, 115, 124, 133, 151, 169, 178, or 187, (ii) a VH-CDR2 comprising the amino acid sequence selected from any one of SEQ ID NOs: 109, 118, 127, 136, 154, 163, 172, 181, or 190, and (iii) a VH-CDR3 comprising the amino acid sequence selected from any one of SEQ ID NOs: 112, 121, 130, 139, 157, 175, 184, 193; and a light chain variable region comprising (i) a VL-CDR1 comprising the amino acid sequence selected from any one of SEQ ID NOs: 196, 203, 218, 226, or 254, (ii) a VL-CDR2 comprising the amino acid sequence selected from any one of SEQ ID NOs: 199, 206, or 229, and (iii) a VL-CDR3 comprising the amino acid sequence selected from any one of SEQ ID NOs: 29, 57, 73, 81, 97, 208, or 223. The antibody or antigen-binding fragment thereof specifically binds plazomicin or a pharmaceutically acceptable salt thereof.

In particular embodiments, the antibody may comprise: a) a heavy chain variable region comprising: i) a CDR1 region that is identical in amino acid sequence to the heavy chain CDR1 region of a selected antibody described herein; ii) a CDR2 region that is identical in amino acid sequence to the heavy chain CDR2 region of the selected antibody; and iii) a CDR3 region that is identical in amino acid sequence to the heavy chain CDR3 region of the selected antibody; and b) a light chain variable domain comprising: i) a CDR1 region that is identical in amino acid sequence to the light chain CDR1 region of the selected antibody; ii) a CDR2 region that is identical in amino acid sequence to the light chain CDR2 region of the selected antibody; and iii) a CDR3 region that is identical in amino acid sequence to the light chain CDR3 region of the selected antibody; wherein the antibody specifically binds a selected target (e.g., plazomicin).

In some embodiments, the isolated antibody or antigen-binding fragment thereof comprises a light chain variable region comprising a VL-CDR1, a VL-CDR2, and a VL-CDR3, respectively, comprising the amino acid sequence of a VL-CDR1, a VL-CDR2, and a VL-CDR3 contained within the light chain variable region set forth in SEQ ID NO: 26, SEQ ID NO: 30, SEQ ID NO: 38, SEQ ID NO: 46, SEQ ID NO: 54, SEQ ID NO: 62, SEQ ID NO: 70, SEQ ID NO: 78, SEQ ID NO: 86, SEQ ID NO: 94, or SEQ ID NO: 102; and/or a heavy chain variable region comprising a VH-CDR1, a VH-CDR2, and a VH-CDR3, respectively, comprising the amino acid sequence of a VH-CDR1, a VH-CDR2, and a VH-CDR3 contained within the heavy chain variable region set forth in SEQ ID NO: 22, SEQ ID NO: 34, SEQ ID NO: 42, SEQ ID NO: 50, SEQ ID NO: 58, SEQ ID NO: 66, SEQ ID NO: 74: SEQ ID NO: 82; SEQ ID NO: 90, and SEQ ID NO: 98. The antibody or antigen-binding fragment thereof specifically binds plazomicin or a pharmaceutically acceptable salt thereof.

In some embodiments, the isolated antibody or the antigen-binding fragment thereof, comprises a light chain variable region comprising a VL-CDR1 set forth in SEQ ID NO: SEQ ID NO: 196, 203, 218, 226, or 254; a VL-CDR2 set forth in SEQ ID NO: 199, 206 or 229; and a VL-CDR3 set forth in 29, 57, 73, 81, 97, 208, or 223; and/or a heavy chain variable region comprising a VH-CDR1 set forth in 106, 115, 124, 133, 151, 169, 178, or 187; a VH-CDR2 set forth in SEQ ID NO: 109, 118, 127, 136, 154, 163, 172, 181, or 190; and a VL-CDR3 set forth in SEQ ID NOs: 29, 57, 73, 81, 97, 208, or 223. The antibody or antigen-binding fragment thereof specifically binds plazomicin or a pharmaceutically acceptable salt thereof.

In some embodiments, the antibody or antigen-binding fragment thereof comprises (a) a light chain variable region comprising VL-CDR1, a VL-CDR2 and VL-CDR3 set forth in SEQ ID NO: 196, SEQ ID NO: 199, and SEQ ID NO: 29, respectively; and; a heavy chain variable region comprising VH-CDR1, VH-CDR2 and VH-CDR3 set forth in SEQ ID NO: 106, SEQ ID NO: 109, and SEQ ID NO: 112, respectively; (b) a light chain variable region comprising VL-CDR1, a VL-CDR2 and VL-CDR3 set forth in SEQ ID NO: 203, SEQ ID NO: 206, and SEQ ID NO: 208, respectively; and; a heavy chain variable region comprising VH-CDR1, VH-CDR2 and VH-CDR3 set forth in SEQ ID NO: 106, SEQ ID NO: 109, and SEQ ID NO: 112, respectively; (c) a light chain variable region comprising VL-CDR1, a VL-CDR2 and VL-CDR3 set forth in SEQ ID NO: 211, SEQ ID NO: 214, and SEQ ID NO: 41, respectively; and; a heavy chain variable region comprising VH-CDR1, VH-CDR2 and VH-CDR3 set forth in SEQ ID NO: 115, SEQ ID NO: 118, and SEQ ID NO: 121, respectively; (d) a light chain variable region comprising VL-CDR1, a VL-CDR2 and VL-CDR3 set forth in SEQ ID NO: 218, SEQ ID NO: 221, and SEQ ID NO: 223, respectively; and; a heavy chain variable region comprising VH-CDR1, VH-CDR2 and VH-CDR3 set forth in SEQ ID NO: 124, SEQ ID NO: 127, and SEQ ID NO: 130, respectively; (e) a light chain variable region comprising VL-CDR1, a VL-CDR2 and VL-CDR3 set forth in SEQ ID NO: 226, SEQ ID NO: 229, and SEQ ID NO: 57, respectively; and; a heavy chain variable region comprising VH-CDR1, VH-CDR2 and VH-CDR3 set forth in SEQ ID NO: 133, SEQ ID NO: 136, and SEQ ID NO: 139, respectively; (f) a light chain variable region comprising VL-CDR1, a VL-CDR2 and VL-CDR3 set forth in SEQ ID NO: 240, SEQ ID NO: 243, and SEQ ID NO: 73, respectively; and; a heavy chain variable region comprising VH-CDR1, VH-CDR2 and VH-CDR3 set forth in SEQ ID NO: 151, SEQ ID NO: 154, and SEQ ID NO: 157, respectively; (g) a light chain variable region comprising VL-CDR1, a VL-CDR2 and VL-CDR3 set forth in SEQ ID NO: 247, SEQ ID NO: 250, and SEQ ID NO: 81, respectively; and; a heavy chain variable region comprising VH-CDR1, VH-CDR2 and VH-CDR3 set forth in SEQ ID NO: 160, SEQ ID NO: 163, and SEQ ID NO: 166, respectively; (h) a light chain variable region comprising VL-CDR1, a VL-CDR2 and VL-CDR3 set forth in SEQ ID NO: 254, SEQ ID NO: 257, and SEQ ID NO: 89, respectively; and; a heavy chain variable region comprising VH-CDR1, VH-CDR2 and VH-CDR3 set forth in SEQ ID NO: 169, SEQ ID NO: 172, and SEQ ID NO: 175, respectively; (i) a light chain variable region comprising VL-CDR1, a VL-CDR2 and VL-CDR3 set forth in SEQ ID NO: 261, SEQ ID NO: 264, and SEQ ID NO: 97, respectively; and; a heavy chain variable region comprising VH-CDR1, VH-CDR2 and VH-CDR3 set forth in SEQ ID NO: 178, SEQ ID NO: 181, and SEQ ID NO: 184, respectively; or (j) a light chain variable region comprising VL-CDR1, a VL-CDR2 and VL-CDR3 set forth in SEQ ID NO: 268, SEQ ID NO: 271, and SEQ ID NO: 105, respectively; and; a heavy chain variable region comprising VH-CDR1, VH-CDR2 and VH-CDR3 set forth in SEQ ID NO: 187, SEQ ID NO: 190, and SEQ ID NO: 193, respectively. IThe antibody or antigen-binding fragment thereof specifically binds plazomicin or a pharmaceutically acceptable salt thereof.

In some embodiments, the antibody or antigen-binding fragment thereof comprises (a) a variable light chain region that is, at least 85%, at least 90%, at least 95%, at least 96%, at least 97%, at least 98%, at least 99%, or 100% identical to SEQ ID NO: 26, and a variable heavy chain region that is at least 85%, at least 90%, at least 95%, at least 96%, at least 97%, at least 98%, at least 99%, or 100% identical to SEQ ID NO: 22; (b) a variable light chain region that is at least 85%, at least 90%, at least 95%, at least 96%, at least 97%, at least 98%, at least 99%, or 100% identical to SEQ ID NO: 30, and a variable heavy chain region that is at least 85%, at least 90%, at least 95%, at least 96%, at least 97%, at least 98%, at least 99%, or 100% identical to SEQ ID NO: 22; (c) a variable light chain region that is at least 85%, at least 90%, at least 95%, at least 96%, at least 97%, at least 98%, at least 99%, or 100% identical to SEQ ID NO: 38, and a variable heavy chain region that is at least 85%, at least 90%, at least 95%, at least 96%, at least 97%, at least 98%, at least 99%, or 100% identical to SEQ ID NO: 34; (d) a variable light chain region that is at least 85%, at least 90%, at least 95%, at least 96%, at least 97%, at least 98%, at least 99%, or 100% identical to SEQ ID NO: 46, and a variable heavy chain region that is at least 85%, at least 90%, at least 95%, at least 96%, at least 97%, at least 98%, at least 99%, or 100% identical to SEQ ID NO: 42; (e) a variable light chain region that is at least 85%, at least 90%, at least 95%, at least 96%, at least 97%, at least 98%, at least 99%, or 100% identical to SEQ ID NO: 54, and a variable heavy chain region that is at least 85%, at least 90%, at least 95%, at least 96%, at least 97%, at least 98%, at least 99%, or 100% identical to SEQ ID NO: 50; (f) a variable light chain region that is at least 85%, at least 90%, at least 95%, at least 96%, at least 97%, at least 98%, at least 99%, or 100% identical to SEQ ID NO: 62, and a variable heavy chain region that is at least 85%, at least 90%, at least 95%, at least 96%, at least 97%, at least 98%, at least 99%, or 100% identical to SEQ ID NO: 58; (g) a variable light chain region that is at least 85%, at least 90%, at least 95%, at least 96%, at least 97%, at least 98%, at least 99%, or 100% identical to SEQ ID NO: 70, and a variable heavy chain region that is at least 85%, at least 90%, at least 95%, at least 96%, at least 97%, at least 98%, at least 99%, or 100% identical to SEQ ID NO: 66; (h) a variable light chain region that is at least 85%, at least 90%, at least 95%, at least 96%, at least 97%, at least 98%, at least 99%, or 100% identical to SEQ ID NO: 78, and a variable heavy chain region that is at least 85%, at least 90%, at least 95%, at least 96%, at least 97%, at least 98%, at least 99%, or 100% identical to SEQ ID NO: 74; (i) a variable light chain region that is at least 85%, at least 90%, at least 95%, at least 96%, at least 97%, at least 98%, at least 99%, or 100% identical to SEQ ID NO: 86, and a variable heavy chain region that is at least 85%, at least 90%, at least 95%, at least 96%, at least 97%, at least 98%, at least 99%, or 100% identical to SEQ ID NO: 82; (j) a variable light chain region that is at least 85% identical to SEQ ID NO: 94, and a variable heavy chain region that is at least 85%, at least 90%, at least 95%, at least 96%, at least 97%, at least 98%, at least 99%, or 100% identical to SEQ ID NO: 90; or (k) a variable light chain region that is at least 85%, at least 90%, at least 95%, at least 96%, at least 97%, at least 98%, at least 99%, or 100% identical to SEQ ID NO: 102, and a variable heavy chain region that is at least 85%, at least 90%, at least 95%, at least 96%, at least 97%, at least 98%, at least 99%, or 100% identical to SEQ ID NO: 98. The antibody or antigen-binding fragment thereof specifically binds plazomicin or a pharmaceutically acceptable salt thereof.

In some embodiments, the antibody or antigen-binding fragment thereof comprises (a) a variable light chain region according to SEQ ID NO: 26, and a variable heavy chain region according to SEQ ID NO: 22; (b) a variable light chain region according to SEQ ID NO: 30, and a variable heavy chain region according to SEQ ID NO: 22; (c) a variable light chain region according to SEQ ID NO: 38, and a variable heavy chain region according 1 to SEQ ID NO: 34; (d) a variable light chain region according to SEQ ID NO: 46, and a variable heavy chain region according to SEQ ID NO: 42; (e) a variable light chain region according to SEQ ID NO: 54, and a variable heavy chain region according to SEQ ID NO: 50; (f) a variable light chain region according to SEQ ID NO: 62, and a variable heavy chain region according to SEQ ID NO: 58; (g) a variable light chain region according to SEQ ID NO: 70, and a variable heavy chain region according to SEQ ID NO: 66; (h) a variable light chain region according to SEQ ID NO: 78, and a variable heavy chain region according to SEQ ID NO: 74; (i) a variable light chain region according to SEQ ID NO: 86, and a variable heavy chain region according to SEQ ID NO: 82; (j) a variable light chain region according to SEQ ID NO: 94, and a variable heavy chain region according to SEQ ID NO: 90; or (k) a variable light chain region according to SEQ ID NO: 102, and a variable heavy chain region according to SEQ ID NO: 98. The antibody or antigen-binding fragment thereof specifically binds plazomicin or a pharmaceutically acceptable salt thereof.

In particular embodiments the antibody may comprise a heavy chain variable region comprising any of the following: a) SEQ ID NOs: 23, 24, and 22; b) SEQ ID NOs:35, 36, and 37; c) SEQ ID NOs:43, 44, and 45; d) SEQ ID NOs:51, 52, and 53; e) SEQ ID NOs:59, 60, and 61; f) SEQ ID NOs: 67, 68, 69; g) SEQ ID NOs: 75, 76, and 77; h) SEQ ID NOs:83, 84, and 85; i) SEQ ID NOs:91, 92, and 93; j) SEQ ID NOs:99, 100, and 101; k) SEQ ID NO:22; 1) SEQ ID NO:34; m) SEQ ID NO:42; n) SEQ ID NO:50; o) SEQ ID NO:58; p) SEQ ID NO:66; q) SEQ ID NO:74; r) SEQ ID NO:82; s) SEQ ID NO:90; or t) SEQ ID NO:98; and a light chain variable region comprising any of the following: a) SEQ ID NOs: 27, 28, and 29; b) SEQ ID NOs:31, 32, and 33; c) SEQ ID NOs:39, 40, and 41; d) SEQ ID NOs:47, 48, and 49; e) SEQ ID NOs:55, 56, and 57; f) SEQ ID NOs:63, 64, and 65; g) SEQ ID NOs:71, 72, and 73; h) SEQ ID NOs:79, 80, and 81; i) SEQ ID NOs:87, 88, and 89; j) SEQ ID NOs:95, 96, and 97; k) SEQ ID NOs:103, 104, and 105; 1) SEQ ID NO:26; m) SEQ ID NO:30; n) SEQ ID NO:38; o) SEQ ID NO:46; p) SEQ ID NO:54; q) SEQ ID NO:62; r) SEQ ID NO:70; s) SEQ ID NO:78; t) SEQ ID NO:86; u) SEQ ID NO:94; or v) SEQ ID NO:102.

A further alternative is to generate novel VH or VL regions carrying one or more CDR-derived sequences of the herein described invention embodiments using random mutagenesis of one or more selected VH and/or VL genes to generate mutations within the entire variable domain. Such a technique is described by Gram et al (1992, Proc. Natl. Acad. Sci., USA, 89:3576-3580), who used error-prone PCR. Another method which may be used is to direct mutagenesis to CDR regions of VH or VL genes. Such techniques are disclosed by Barbas et al., (1994, Proc. Natl. Acad. Sci., USA, 91:3809-3813) and Schier et al (1996, J. Mol. Biol. 263:551-567).

In certain embodiments, a specific VH and/or VL of the antibodies described herein may be used to screen a library of the complementary variable domain to identify antibodies with desirable properties, such as increased affinity for plazomicin. Such methods are described, for example, in Portolano et al., J. Immunol. (1993) 150:880-887; Clarkson et al., Nature (1991) 352:624-628.

Exemplary variable light and heavy chain regions, along with VH-CDR1, VH-CDR2, VH-CDR3, VL-CDR1, VL-CDR2, and VL-CDR3 sequences according to various delineations and consensus sequences is provided in Table 3, below, and in the listing of sequences.

**Table 3: Exemplary anti-plazomicin antibody sequences, CDR sequences, and consensus sequences**

| **Antibody or Fragment** | **SEQ ID NO:** | **Chain** | **Amino acid sequence** |
|---|---|---|---|
| MC329-A/B variable region | 22 | Heavy | |
| MC329-A variable region | 26 | Light | |
| MC329-B variable region | 30 | Light | |
| MC330 variable region | 34 | Heavy | |
| MC330 variable region | 38 | Light | |
| MC331 variable region | 42 | Heavy | |
| MC331 variable region | 46 | Light | |
| MC332 variable region | 50 | Heavy | |
| MC332 variable region | 54 | Light | |
| MC334 variable region | 58 | Heavy | |
| MC334 variable region | 62 | Light | |
| MC335 variable region | 66 | Heavy | |
| MC335 variable region | 70 | Light | |
| MC336 variable region | 74 | Heavy | |
| MC336 variable region | 78 | Light | |
| MC337 variable region | 82 | Heavy | |
| MC337 variable region | 86 | Light | |
| MC339 variable region | 90 | Heavy | |
| MC339 variable region | 94 | Light | |
| MC340 variable region | 98 | Heavy | |
| MC340 variable region | 102 | Light | |
| MC329A/ MC329B VH-CDR1 | 23 | Heavy | GYSITTNY |
| MC329A/ MC329B VH-CDR1 | 106 | Heavy | TNYAWN |
| MC329A/ MC329B VH-CDR1 | 107 | Heavy | GYSITTN |
| MC329A/ MC329B VH-CDR1 | 108 | Heavy | TTNYAWN |
| MC329A/ MC329B VH-CDR2 | 24 | Heavy | ITSSGRT |
| MC329A/ MC329B VH-CDR2 | 109 | Heavy | YITSSGRTSYNPPLKS |
| MC329A/ MC329B VH-CDR2 | 110 | Heavy | SSG |
| MC329A/ MC329B VH-CDR2 | 111 | Heavy | WMGYITSSGRTS |
| MC329A/ MC329B VH-CDR3 | 25 | Heavy | THTTAYLDY |
| MC329A/ MC329B VH-CDR3 | 112 | Heavy | TTAYLDY |
| MC329A/ MC329B VH-CDR3 | 113 | Heavy | TAYLD |
| MC329A/ MC329B VH-CDR3 | 114 | Heavy | THTTAYLD |
| MC330 VH-CDR1 | 35 | Heavy | GYSITSAYA |
| MC330 VH-CDR1 | 115 | Heavy | SAYAWN |
| MC330 VH-CDR1 | 116 | Heavy | GYSITSA |
| MC330 VH-CDR1 | 117 | Heavy | TSAYAWN |
| MC330 VH-CDR2 | 36 | Heavy | INSSGST |
| MC330 VH-CDR2 | 118 | Heavy | YINSSGSTSYHPSLKG |
| MC330 VH-CDR2 | 119 | Heavy | SSG |
| MC330 VH-CDR2 | 120 | Heavy | WLGYINSSGSTS |
| MC330 VH-CDR3 | 37 | Heavy | THTTSYFDY |
| MC330 VH-CDR3 | 121 | Heavy | TTSYFDY |
| MC330 VH-CDR3 | 122 | Heavy | TSYFD |
| MC330 VH-CDR3 | 123 | Heavy | THTTSYFD |
| MC331 VH-CDR1 | 43 | Heavy | GNTFTSYW |
| MC331 VH-CDR1 | 124 | Heavy | SYWMH |
| MC331 VH-CDR1 | 125 | Heavy | GNTFTSY |
| MC331 VH-CDR1 | 126 | Heavy | TSYWMH |
| MC331 VH-CDR2 | 44 | Heavy | IDPSTGYT |
| MC331 VH-CDR2 | 127 | Heavy | YIDPSTGYTEYNQKFKD |
| MC331 VH-CDR2 | 128 | Heavy | STG |
| MC331 VH-CDR2 | 129 | Heavy | WIGYIDPSTGYTE |
| MC331 VH-CDR3 | 45 | Heavy | TKSGKWDDY |
| MC331 VH-CDR3 | 130 | Heavy | SGKWDDY |
| MC331 VH-CDR3 | 131 | Heavy | GKWDD |
| MC331 VH-CDR3 | 132 | Heavy | TKSGKWDD |
| MC332 VH-CDR1 | 51 | Heavy | GYKFSRYT |
| MC332 VH-CDR1 | 133 | Heavy | RYWIE |
| MC332 VH-CDR1 | 134 | Heavy | GYKFSRY |
| MC332 VH-CDR1 | 135 | Heavy | GYKFS |
| MC332 VH-CDR2 | 136 | Heavy | EIFPGSDSTDYNEKFKG |
| MC332 VH-CDR2 | 52 | Heavy | IFPGSDST |
| MC332 VH-CDR2 | 137 | Heavy | GSD |
| MC332 VH-CDR2 | 138 | Heavy | WIGEIFPGSDSTD |
| MC332 VH-CDR3 | 53 | Heavy | ARDGGGLDEFAY |
| MC332 VH-CDR3 | 139 | Heavy | DGGGLDEF AY |
| MC332 VH-CDR3 | 140 | Heavy | GGGLDEFA |
| MC332 VH-CDR3 | 141 | Heavy | ARDGGGLDEFA |
| MC334 VH-CDR1 | 59 | Heavy | GYKFSRYW |
| MC334 VH-CDR1 | 142 | Heavy | RYWIE |
| MC334 VH-CDR1 | 143 | Heavy | GYKFSRY |
| MC334 VH-CDR1 | 144 | Heavy | GYKFS |
| MC334 VH-CDR2 | 60 | Heavy | IFPGSDST |
| MC334 VH-CDR2 | 145 | Heavy | EIFPGSDSTDYNEKFKG |
| MC334 VH-CDR2 | 146 | Heavy | GSD |
| MC334 VH-CDR2 | 147 | Heavy | WIGEIFPGSDSTD |
| MC334 VH-CDR3 | 61 | Heavy | ARDGGGLDEFAY |
| MC334 VH-CDR3 | 148 | Heavy | DGGGLDEF AY |
| MC334 VH-CDR3 | 149 | Heavy | GGGLDEFA |
| MC334 VH-CDR3 | 150 | Heavy | ARDGGGLDEFA |
| MC335 VH-CDR1 | 67 | Heavy | GSSITSDYA |
| MC335 VH-CDR1 | 151 | Heavy | SDYAWN |
| MC335 VH-CDR1 | 152 | Heavy | GSSITSD |
| MC335 VH-CDR1 | 153 | Heavy | TSDYAWN |
| MC335 VH-CDR2 | 68 | Heavy | ISQSGST |
| MC335 VH-CDR2 | 154 | Heavy | YISQSGSTSYNPSLKS |
| MC335 VH-CDR2 | 155 | Heavy | QSG |
| MC335 VH-CDR2 | 156 | Heavy | WMGYISQSGSTS |
| MC335 VH-CDR3 | 69 | Heavy | TGNSYTMDY |
| MC335 VH-CDR3 | 157 | Heavy | NSYTMDY |
| MC335 VH-CDR3 | 158 | Heavy | SYTMD |
| MC335 VH-CDR3 | 159 | Heavy | TGNSYTMD |
| MC336 VH-CDR1 | 75 | Heavy | GDSITSDYA |
| MC336 VH-CDR1 | 160 | Heavy | SDYAWN |
| MC336 VH-CDR1 | 161 | Heavy | GDSITSD |
| MC336 VH-CDR1 | 162 | Heavy | TSDYAWN |
| MC336 VH-CDR2 | 76 | Heavy | ISYSGST |
| MC336 VH-CDR2 | 163 | Heavy | YISYSGSTSYNPSLKS |
| MC336 VH-CDR2 | 164 | Heavy | YSG |
| MC336 VH-CDR2 | 165 | Heavy | WMGYISYSGSTS |
| MC336 VH-CDR3 | 77 | Heavy | TGNSYTMDY |
| MC336 VH-CDR3 | 166 | Heavy | NSYTMDY |
| MC336 VH-CDR3 | 167 | Heavy | SYTMD |
| MC336 VH-CDR3 | 168 | Heavy | TGNSYTMD |
| MC337 VH-CDR1 | 83 | Heavy | GYTFTSYV |
| MC337 VH-CDR1 | 169 | Heavy | SYVIH |
| MC337 VH-CDR1 | 170 | Heavy | GYTFTSY |
| MC337 VH-CDR1 | 171 | Heavy | TSYVIH |
| MC337 VH-CDR2 | 84 | Heavy | INPFNDGT |
| MC337 VH-CDR2 | 172 | Heavy | YINPFNDGTKYNEKFKG |
| MC337 VH-CDR2 | 173 | Heavy | FND |
| MC337 VH-CDR2 | 174 | Heavy | WIGYINPFNDGTK |
| MC337 VH-CDR3 | 85 | Heavy | QGVGSSGSDY |
| MC337 VH-CDR3 | 175 | Heavy | VGSSGSDY |
| MC337 VH-CDR3 | 176 | Heavy | GSSGSD |
| MC337 VH-CDR3 | 177 | Heavy | EGVGSSGSD |
| MC339 VH-CDR1 | 91 | Heavy | GFSFTDYN |
| MC339 VH-CDR1 | 178 | Heavy | DYNMY |
| MC339 VH-CDR1 | 179 | Heavy | GFSFTDY |
| MC339 VH-CDR1 | 180 | Heavy | TDYNMY |
| MC339 VH-CDR2 | 181 | Heavy | YIDPYNGGTSYNQKFKG |
| MC339 VH-CDR2 | 92 | Heavy | IDPYNGGT |
| MC339 VH-CDR2 | 182 | Heavy | YNG |
| MC339 VH-CDR2 | 183 | Heavy | WIGYIDPYNGGTS |
| MC339 VH-CDR3 | 93 | Heavy | ASAGNYDDY |
| MC339 VH-CDR3 | 184 | Heavy | AGNYDDY |
| MC339 VH-CDR3 | 185 | Heavy | GNYDD |
| MC339 VH-CDR3 | 186 | Heavy | ASAGNYDD |
| MC340 VH-CDR1 | 99 | Heavy | GL TFRNYW |
| MC340 VH-CDR1 | 187 | Heavy | NYWMS |
| MC340 VH-CDR1 | 188 | Heavy | GLTFRNY |
| MC340 VH-CDR1 | 189 | Heavy | GLTFR |
| MC340 VH-CDR2 | 100 | Heavy | IRLKSDNYAT |
| MC340 VH-CDR2 | 190 | Heavy | EIRLKSDNYATHYAESVKG |
| MC340 VH-CDR2 | 191 | Heavy | DNY |
| MC340 VH-CDR2 | 192 | Heavy | WVAEIRLKSDNYATH |
| MC340 VH-CDR3 | 101 | Heavy | TVYGKDFDY |
| MC340 VH-CDR3 | 193 | Heavy | YGKDFDY |
| MC340 VH-CDR3 | 194 | Heavy | GKDFD |
| MC340 VH-CDR3 | 195 | Heavy | TVYGKDFD |
| MC329A VL-CDR1 | 27 | Light | QSVSND |
| MC329A VL-CDR1 | 196 | Light | KASQSVSNDV A |
| MC329A VL-CDR1 | 197 | Light | SQSVSND |
| MC329A VL-CDR1 | 198 | Light | SNDVAWY |
| MC329A VL-CDR2 | 199 | Light | YASNRYT |
| MC329A VL-CDR2 | 28 | Light | YAS |
| MC329A VL-CDR2 | 200 | Light | LLISY ASNRY |
| MC329A VL-CDR3 | 29 | Light | QQDYSSPYT |
| MC329A VL-CDR3 | 201 | Light | DYSSPY |
| MC329A VL-CDR3 | 202 | Light | QQDYSSPY |
| MC329B VL-CDR1 | 31 | Light | QDIKSY |
| MC329B VL-CDR1 | 203 | Light | KASQDIKSYLS |
| MC329B VL-CDR1 | 204 | Light | SQDIKSY |
| MC329B VL-CDR1 | 205 | Light | KSYLSWY |
| MC329B VL-CDR2 | 206 | Light | YATSLAD |
| MC329B VL-CDR2 | 32 | Light | YAT |
| MC329B VL-CDR2 | 207 | Light | TLIYYAT SLA |
| MC329B VL-CDR3 | 33 | Light | LQHGESPLTFGAGTKLELK |
| MC329B VL-CDR3 | 208 | Light | LQHGESPL T |
| MC329B VL-CDR3 | 209 | Light | HGESPL |
| MC329B VL-CDR3 | 210 | Light | LQHGESPL |
| MC330 VL-CDR1 | 39 | Light | QSVSND |
| MC330 VL-CDR1 | 211 | Light | KASQSVSNDV A |
| MC330 VL-CDR1 | 212 | Light | SQSVSND |
| MC330 VL-CDR1 | 213 | Light | SNDVAWY |
| MC330 VL-CDR2 | 214 | Light | YASNRYT |
| MC330 VL-CDR2 | 40 | Light | YAS |
| MC330 VL-CDR2 | 215 | Light | LLISY ASNRY |
| MC330 VL-CDR3 | 41 | Light | QQDYSSPYT |
| MC330 VL-CDR3 | 216 | Light | YSSPY |
| MC330 VL-CDR3 | 217 | Light | QQDYSSPY |
| MC331 VL-CDR1 | 47 | Light | QSVTND |
| MC331 VL-CDR1 | 218 | Light | KASQSVTNDVA |
| MC331 VL-CDR1 | 219 | Light | QSVTND |
| MC331 VL-CDR1 | 220 | Light | TNDVAWY |
| MC331 VL-CDR2 | 221 | Light | YASNRYT |
| MC331 VL-CDR2 | 48 | Light | YAS |
| MC331 VL-CDR2 | 222 | Light | LLICYASNRY |
| MC331 VL-CDR3 | 49 | Light | QQDHTSPFTF |
| MC331 VL-CDR3 | 223 | Light | QQDHTSPFT |
| MC331 VL-CDR3 | 224 | Light | DHTSPF |
| MC331 VL-CDR3 | 225 | Light | QQDHTSPF |
| MC332 VL-CDR1 | 55 | Light | QSLTNSYGY |
| MC332 VL-CDR1 | 226 | Light | RSSQSLTNSYGYTYLS |
| MC332 VL-CDR1 | 227 | Light | SQSLTNSYGYTY |
| MC332 VL-CDR1 | 228 | Light | YTYLSWY |
| MC332 VL-CDR2 | 229 | Light | GISNRFS |
| MC332 VL-CDR2 | 56 | Light | GIS |
| MC332 VL-CDR2 | 230 | Light | LLIYGISNRF |
| MC332 VL-CDR3 | 57 | Light | LQGTHQPYT |
| MC332 VL-CDR3 | 231 | Light | GTHQPY |
| MC332 VL-CDR3 | 232 | Light | LQGTHQPY |
| MC334 VL-CDR1 | 63 | Light | QSLTNSYGYTY |
| MC334 VL-CDR1 | 233 | Light | RSSQSLTNSYGYTYLS |
| MC334 VL-CDR1 | 234 | Light | SQSLTNSYGYTY |
| MC334 VL-CDR1 | 235 | Light | YTYLSWY |
| MC334 VL-CDR2 | 236 | Light | GISNRFS |
| MC334 VL-CDR2 | 64 | Light | GIS |
| MC334 VL-CDR2 | 237 | Light | LLIYGISNRF |
| MC334 VL-CDR3 | 65 | Light | LQGTHQPYT |
| MC334 VL-CDR3 | 238 | Light | GTHQPY |
| MC334 VL-CDR3 | 239 | Light | LQGTHQPY |
| MC335 VL-CDR1 | 71 | Light | QSVSND |
| MC335 VL-CDR1 | 240 | Light | KASQSVSNDV A |
| MC335 VL-CDR1 | 241 | Light | SQSVSND |
| MC335 VL-CDR1 | 242 | Light | SNDVAWY |
| MC335 VL-CDR2 | 243 | Light | YASNRYT |
| MC335 VL-CDR2 | 72 | Light | YAS |
| MC335 VL-CDR2 | 244 | Light | LLISY ASNRY |
| MC335 VL-CDR3 | 73 | Light | QQDYSSPWT |
| MC335 VL-CDR3 | 245 | Light | DYSSPW |
| MC335 VL-CDR3 | 246 | Light | QQDYSSPW |
| MC336 VL-CDR1 | 79 | Light | QSVSND |
| MC336 VL-CDR1 | 247 | Light | KASQSVSNDV A |
| MC336 VL-CDR1 | 248 | Light | SQSVSND |
| MC336 VL-CDR1 | 249 | Light | SNDVAWY |
| MC336 VL-CDR2 | 250 | Light | YASNRYT |
| MC336 VL-CDR2 | 80 | Light | YAS |
| MC336 VL-CDR2 | 251 | Light | YASNRY |
| MC336 VL-CDR3 | 81 | Light | QQDYISPWT |
| MC336 VL-CDR3 | 252 | Light | DYISPW |
| MC336 VL-CDR3 | 253 | Light | QQDYISPW |
| MC337 VL-CDR1 | 87 | Light | QSVRND |
| MC337 VL-CDR1 | 254 | Light | KASQSVRNDVA |
| MC337 VL-CDR1 | 255 | Light | SQSVRND |
| MC337 VL-CDR1 | 256 | Light | RNDVAWY |
| MC337 VL-CDR2 | 257 | Light | YASNRYT |
| MC337 VL-CDR2 | 88 | Light | YAS |
| MC337 VL-CDR2 | 258 | Light | LLISY ASNRY |
| MC337 VL-CDR3 | 89 | Light | QQDYSSPYT |
| MC337 VL-CDR3 | 259 | Light | DYSSPY |
| MC337 VL-CDR3 | 260 | Light | QQDYSSPY |
| MC339 VL-CDR1 | 95 | Light | QSVSND |
| MC339 VL-CDR1 | 261 | Light | KASQSVSNDV A |
| MC339 VL-CDR1 | 262 | Light | SQSVSND |
| MC339 VL-CDR1 | 263 | Light | SNDVAWY |
| MC339 VL-CDR2 | 264 | Light | YASNRYT |
| MC339 VL-CDR2 | 96 | Light | YAS |
| MC339 VL-CDR2 | 265 | Light | LLIYYASNRY |
| MC339 VL-CDR3 | 97 | Light | QQDYTSPYT |
| MC339 VL-CDR3 | 266 | Light | DYTSPY |
| MC339 VL-CDR3 | 267 | Light | QQDYTSPY |
| MC340 VL-CDR1 | 103 | Light | QSVSND |
| MC340 VL-CDR1 | 268 | Light | KASQSVSNDV A |
| MC340 VL-CDR1 | 269 | Light | SQSVSND |
| MC340 VL-CDR1 | 270 | Light | SNDVAWY |
| MC340 VL-CDR2 | 271 | Light | YASNRYT |
| MC340 VL-CDR2 | 104 | Light | YAS |
| MC340 VL-CDR2 | 272 | Light | LLISY ASNRY |
| MC340 VL-CDR3 | 105 | Light | QQDYSSPWT |
| MC340 VL-CDR3 | 273 | Light | DYSSPW |
| MC340 VL-CDR3 | 274 | Light | QQDYSSPW |
| VL-CDR1 Consensus | 275 | Light | KASQSVX₁NDVA, wherein X₁ is S, T, or R |
| VL-CDR1 Consensus | 276 | Light | SQSVX₁ND, wherein X₁ is S, T, or R |
| VL-CDR1 Consensus | 277 | Light | X₁NDVAWY, wherein X₁ is S, T, or R |
| VL-CDR1 Consensus | 278 | Light | KASQSVX₁NDVAWY, wherein X₁ is S, T, or R |
| VL-CDR2 Consensus | 279 | Light | YASNRYT |
| VL-CDR2 Consensus | 280 | Light | YAS |
| VL-CDR2 Consensus | 281 | Light | LLIX₁YASNRY, wherein X₁ is Y, S or C |
| VL-CDR2 Consensus | 282 | Light | LLIX₁YASNRYT, wherein X₁ is Y, S or C |
| VL-CDR3 Consensus | 283 | Light | QQDX₂X₃SPX₄T, wherein X₂ is Y or H; X₃ is S, T, or I; and X₄ is Y, F, or W |
| VL-CDR3 Consensus | 284 | Light | QQDX₂X₃SPX₄, wherein X₂ is Y or H; X₃ is S, T, or I; and X₄ is Y, F, or W |
| VL-CDR3 Consensus | 285 | Light | DX₂X₃SPX₄, wherein X₂ is Y or H; X₃ is S, T, or I; and X₄ is Y, F, or W |

In some of any of the embodiments herein, the antibody further comprises a heavy chain constant domain and/or a light chain constant domain. In some embodiments, the heavy chain and/or light constant domain is murine or human. In some embodiments, the heavy chain constant domain is IgG1, IgG2a, IgG2b or IgM.

A "constant region" or "constant domain" is a domain in an antibody heavy or light chain that contains a sequence of amino acids that is comparatively more conserved among antibodies than the variable region domain. The constant regions of immunoglobulins show less sequence diversity than the variable regions, and are responsible for binding a number of natural proteins to elicit important biochemical events. Each light chain has a single light chain constant region (CL) domain, which is either of the kappa or lambda type. Each heavy chain contains one or more heavy chain constant region (CH) domains that can be assigned to different classes or isotypes. There are five classes of immunoglobulins: IgA, IgD, IgE, IgG and IgM, having heavy chains designated α, δ, ε, γ and µ, respectively. Full-length IgA, IgD and IgG isotypes contain CH1, CH2, CH3 and a hinge region, while IgE and IgM contain CH1, CH2, CH3 and CH4. The γ and a classes are further divided into subclasses on the basis of relatively minor differences in the CH sequence and function, *e.g*., humans express the following subclasses: IgG1, IgG2, IgG3, IgG4, IgAl and IgA2. IgG1 antibodies can exist in multiple polymorphic variants termed allotypes (reviewed in Jefferis and Lefranc 2009. mAbs Vol 1 Issue 41-7) any of which are suitable. In humans there are five different classes of antibodies including IgA (which includes subclasses IgAl and IgA2), IgD, IgE, IgG (which includes subclasses IgG1, IgG2, IgG3, and IgG4), and IgM. The distinguishing features between these antibody classes are their constant, Fc regions, although subtler differences may exist in the V region. An antibody constant region can include an Fc portion. Although the boundaries of the Fc region of an immunoglobulin heavy chain might vary, the human IgG heavy-chain Fc region usually includes the region containing the CH2 and CH3 domains and the hinge region, such as an amino acid residue at position Cys226, or from Pro230, to the carboxyl-terminus thereof.

In certain embodiments, antibody variable domains with the desired binding specificities are fused to immunoglobulin constant domain sequences. In certain embodiments, the fusion is with an Ig heavy chain constant domain, comprising at least part of the hinge, CH2, and CH3 regions. Certain embodiments have the first heavy-chain constant region (CH1) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments in embodiments when unequal ratios of the three polypeptide chains used in the construction provide the optimum yield of the desired fusion antibody. It is, however, possible to insert the coding sequences for two or all three polypeptide chains into a single expression vector when the expression of at least two polypeptide chains in equal ratios results in high yields or when the ratios have no significant effect on the yield of the desired chain combination.

Antibodies, and antigen-binding fragments and variants thereof, of the present invention may also be modified to include a detectable label, e.g., an epitope tag or label, *e.g.,* for use in purification or diagnostic applications. These may be conjugated to the antibody as a fusion protein or conjugate, *e.g*., using a linker or linking group. There are many linking groups known in the art for making antibody conjugates, including, for example, those disclosed in U.S. Pat. No. 5,208,020 or EP Patent 0 425 235 B1, and Chari et al., Cancer Research 52: 127-131 (1992). Linking groups include disulfide groups, thioester groups, acid labile groups, photolabile groups, peptidase labile groups, or esterase labile groups, as disclosed in the above-identified patents. Examples of tags and/or labels can include, but are not limited to, FLAG tags, poly-histidine tags (*e.g*. 6xHis), cMyc tags, glutathione-S-transferase tags, avidin, fluorescent labels, polymer particles, metal particles, haptens, enzyme labels, luminescent labels, electrochemiluminescent labels, bioluminescent labels, radioisotopes, or oligonucleotides.

The present invention also includes variants and fragments of the antibodies, and antigen-binding fragments thereof, described herein, wherein the variants and fragments bind to plazomicin. In particular embodiments, the variants comprise one or more amino acid modification as compared to any of the antibodies, or antigen-binding fragments thereof, described herein, *e.g.*, an amino acid substitution, deletion, or addition.

As used herein, the term "amino acid" refers to both naturally occurring and non-naturally occurring amino acids as well as amino acid analogs and mimetics. Naturally occurring amino acids include the 20 (L)-amino acids utilized during protein biosynthesis as well as others such as 4-hydroxyproline, hydroxylysine, desmosine, isodesmosine, homocysteine, citrulline and ornithine, for example. Non-naturally occurring amino acids include, for example, (D)-amino acids, norleucine, norvaline, p-fluorophenylalanine, ethionine and the like, which are known to a person skilled in the art. Amino acid analogs include modified forms of naturally and non-naturally occurring amino acids. Such modifications can include, for example, substitution or replacement of chemical groups and moieties on the amino acid or by derivitization of the amino acid. Amino acid mimetics include, for example, organic structures which exhibit functionally similar properties such as charge and charge spacing characteristic of the reference amino acid. For example, an organic structure which mimics Arginine (Arg or R) would have a positive charge moiety located in similar molecular space and having the same degree of mobility as the e-amino group of the side chain of the naturally occurring Arg amino acid. Mimetics also include constrained structures so as to maintain optimal spacing and charge interactions of the amino acid or of the amino acid functional groups. Those skilled in the art know or can determine what structures constitute functionally equivalent amino acid analogs and amino acid mimetics.

The terms "polypeptide" "protein" and "peptide" and "glycoprotein" are used interchangeably and mean a polymer of amino acids (naturally occurring, non-naturally occurring, or both) not limited to any particular length. The term does not exclude modifications such as myristylation, sulfation, glycosylation, phosphorylation and addition or deletion of signal sequences. The terms "polypeptide" or "protein" means one or more chains of amino acids, wherein each chain comprises amino acids covalently linked by peptide bonds, and wherein said polypeptide or protein can comprise a plurality of chains non-covalently and/or covalently linked together by peptide bonds, having the sequence of native proteins, that is, proteins produced by naturally-occurring and specifically non-recombinant cells, or genetically-engineered or recombinant cells, and comprise molecules having the amino acid sequence of the native protein, or molecules having deletions from, additions to, and/or substitutions of one or more amino acids of the native sequence. The terms "polypeptide" and "protein" specifically encompass the antibodies that bind to plazomicin of the present disclosure, or sequences that have deletions from, additions to, and/or substitutions of one or more amino acid of an anti-plazomicin antibody. Thus, a "polypeptide" or a "protein" can comprise one (termed "a monomer") or a plurality (termed "a multimer") of amino acid chains.

The term "isolated protein" referred to herein means that a subject protein (1) is free of at least some other proteins with which it would typically be found in nature, (2) is essentially free of other proteins from the same source, *e.g*., from the same species, (3) is expressed by a cell from a different species, (4) has been separated from at least about 50 percent of polynucleotides, lipids, carbohydrates, or other materials with which it is associated in nature, (5) is not associated (by covalent or noncovalent interaction) with portions of a protein with which the "isolated protein" is associated in nature, (6) is operably associated (by covalent or noncovalent interaction) with a polypeptide with which it is not associated in nature, or (7) does not occur in nature. Such an isolated protein can be encoded by genomic DNA, cDNA, mRNA or other RNA, of may be of synthetic origin, or any combination thereof. In certain embodiments, the isolated protein is substantially free from proteins or polypeptides or other contaminants that are found in its natural environment that would interfere with its use (therapeutic, diagnostic, prophylactic, research or otherwise).

The term "polypeptide fragment" refers to a polypeptide, which can be monomeric or multimeric, with an amino-terminal deletion, a carboxyl-terminal deletion, and/or an internal deletion or substitution of a naturally-occurring or recombinantly-produced polypeptide. In certain embodiments, a polypeptide fragment can comprise an amino acid chain at least 5 to about 500 amino acids long. It will be appreciated that in certain embodiments, fragments are at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 150, 200, 250, 300, 350, 400, or 450 amino acids long. Particularly useful polypeptide fragments include functional domains, including antigen-binding domains or fragments of antibodies. In the case of an anti-plazomicin antibody, useful fragments include, but are not limited to: a CDR region, especially a CDR3 region of the heavy or light chain; a variable region of a heavy or light chain; a portion of an antibody chain or just its variable region including two CDRs; and the like.

A peptide linker/spacer sequence may also be employed to separate multiple polypeptide components by a distance sufficient to ensure that each polypeptide folds into its secondary and/or tertiary structures, if desired. Such a peptide linker sequence can be incorporated into a fusion polypeptide using standard techniques well known in the art.

Certain peptide spacer sequences may be chosen, for example, based on: (1) their ability to adopt a flexible extended conformation; (2) their inability to adopt a secondary structure that could interact with functional epitopes on the first and second polypeptides; and/or (3) the lack of hydrophobic or charged residues that might react with the polypeptide functional epitopes.

In one illustrative embodiment, peptide spacer sequences contain, for example, Gly, Asn and Ser residues. Additional near neutral amino acids, such as Thr and Ala, may also be included in the spacer sequence.

Further amino acid sequences which may be usefully employed as spacers include those disclosed in Maratea et al., Gene 40:39 46 (1985); Murphy et al., Proc. Natl. Acad. Sci. USA 83:8258 8262 (1986); U.S. Pat. No. 4,935,233 and U.S. Pat. No. 4,751,180.

Further illustrative spacers may include, for example, Glu-Gly-Lys-Ser-Ser-Gly-Ser-Gly-Ser-Glu-Ser-Lys-Val-Asp (Chaudhary et al., 1990, Proc. Natl. Acad. Sci. U.S.A. 87:1066-1070) and Lys-Glu-Ser-Gly-Ser-Val-Ser-Ser-Glu-Gln-Leu-Ala-Gln-Phe-Arg-Ser-Leu-Asp (Bird et al., 1988, Science 242:423-426).

In some embodiments, spacer sequences are not required when the first and second polypeptides have non-essential N-terminal amino acid regions that can be used to separate the functional domains and prevent steric interference. Two coding sequences can be fused directly without any spacer or by using a flexible polylinker composed, for example, of the pentamer Gly-Gly-Gly-Gly-Ser repeated 1 to 3 times. Such a spacer has been used in constructing single chain antibodies (scFv) by being inserted between VH and VL (Bird et al., 1988, Science 242:423-426; Huston et al., 1988, Proc. Natl. Acad. Sci. U.S.A. 85:5979-5883).

A peptide spacer, in certain embodiments, is designed to enable the correct interaction between two beta-sheets forming the variable region of the single chain antibody.

In certain embodiments, a peptide spacer is between 1 to 5 amino acids, between 5 to 10 amino acids, between 5 to 25 amino acids, between 5 to 50 amino acids, between 10 to 25 amino acids, between 10 to 50 amino acids, between 10 to 100 amino acids, or any intervening range of amino acids. In some embodiments, a peptide spacer comprises about 1, 5, 10, 15, 20, 25, 30, 35, 40, 45, 50 or more amino acids in length.

Amino acid sequence modification(s) of the antibodies described herein are contemplated. For example, it may be desirable to improve the binding affinity and/or other biological properties of the antibody. For example, amino acid sequence variants of an antibody may be prepared by introducing appropriate nucleotide changes into a polynucleotide that encodes the antibody, or a chain thereof, or by peptide synthesis. Such modifications include, for example, deletions from, and/or insertions into and/or substitutions of, residues within the amino acid sequences of the antibody. Any combination of deletion, insertion, and substitution may be made to arrive at the final antibody, provided that the final construct possesses the desired characteristics (*e.g*., high affinity binding to plazomicin). The amino acid changes may alter post-translational processes of the antibody, such as changing the number or position of glycosylation sites. Any of the variations and modifications described above for polypeptides of the present invention may be included in antibodies of the present invention.

Terms used to describe sequence relationships between two or more protein sequences (or nucleotide sequences) include "reference sequence," "comparison window," "sequence identity," "percentage of sequence identity" and "substantial identity." A "reference sequence" is at least 12 but frequently 15 to 18 and often at least 25 monomer units, inclusive of nucleotides and amino acid residues, in length. Because two protein sequences may each comprise (1) a sequence (*i.e.*, only a portion of the complete sequence) that is similar between the two proteins, and (2) a sequence that is divergent between the two proteins, sequence comparisons between two (or more) protein sequences are typically performed by comparing sequences of the two protein sequences over a "comparison window" to identify and compare local regions of sequence similarity. A "comparison window" refers to a conceptual segment of at least 6 contiguous positions, usually about 50 to about 100, more usually about 100 to about 150 in which a sequence is compared to a reference sequence of the same number of contiguous positions after the two sequences are optimally aligned. The comparison window may comprise additions or deletions (*i.e.*, gaps) of about 20% or less as compared to the reference sequence (which does not comprise additions or deletions) for optimal alignment of the two sequences. Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (*i.e.*, resulting in the highest percentage homology over the comparison window) generated by any of the various methods selected. Reference also may be made to the BLAST family of programs as for example disclosed by Altschul et al., 1997, Nucl. Acids Res. 25:3389. A detailed discussion of sequence analysis can be found in Unit 19.3 of Ausubel et al., "Current Protocols in Molecular Biology," John Wiley & Sons Inc, 1994-1998, Chapter 15.

Optimal alignment of polypeptide or polynucleotide sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J., Unified Approach to Alignment and Phylogenes, pp. 626-645 (1990); Methods in Enzymology vol. 183, Academic Press, Inc., San Diego, CA; Higgins, D.G. and Sharp, P.M., CABIOS 5:151-153 (1989); Myers, E.W. and Muller W., CABIOS 4:11-17 (1988); Robinson, E.D., Comb. Theor 11:105 (1971); Santou, N. Nes, M., Mol. Biol. Evol. 4:406-425 (1987); Sneath, P.H.A. and Sokal, R.R., Numerical Taxonomy - the Principles and Practice of Numerical Taxonomy, Freeman Press, San Francisco, CA (1973); Wilbur, W.J. and Lipman, D.J., Proc. Natl. Acad., Sci. USA 80:726-730 (1983).

Calculations of sequence similarity or sequence identity between sequences (the terms are used interchangeably herein) are performed as follows. To determine the percent identity of two amino acid sequences, or of two nucleic acid sequences, the sequences are aligned for optimal comparison purposes (*e*.*g*., gaps can be introduced in one or both of a first and a second amino acid or nucleic acid sequence for optimal alignment and non-homologous sequences can be disregarded for comparison purposes). In certain embodiments, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position.

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In one embodiment, the percent identity between two amino acid sequences is determined using the Needleman and Wunsch, (1970, J. Mol. Biol. 48: 444-453) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. An additional set of parameters (and the one that should be used unless otherwise specified) are a Blossum 62 scoring matrix with a gap penalty of 12, a gap extend penalty of 4, and a frame shift gap penalty of 5.

The percent identity between two amino acid or nucleotide sequences can be determined using the algorithm of E. Meyers and W. Miller (1989, Cabios, 4: 11-17) which has been incorporated into the ALIGN program (version 2.0), using a PAM120 weight residue table, a gap length penalty of 12 and a gap penalty of 4.

Determination of the three-dimensional structures of representative polypeptides (e.g., variant plazomicin-specific antibodies as provided herein, for instance, an antibody protein having an antigen-binding fragment as provided herein) may be made through routine methodologies such that substitution, addition, deletion or insertion of one or more amino acids with selected natural or non-natural amino acids can be virtually modeled for purposes of determining whether a so derived structural variant retains the space-filling properties of presently disclosed species. *See,* for instance, Donate et al., 1994 Prot. Sci. 3:2378; Bradley et al., Science 309: 1868-1871 (2005); Schueler-Furman et al., Science 310:638 (2005); Dietz et al., Proc. Nat. Acad. Sci. USA 103:1244 (2006); Dodson et al., Nature 450:176 (2007); Qian et al., Nature 450:259 (2007); Raman et al. Science 327:1014-1018 (2010). Some additional non-limiting examples of computer algorithms that may be used for these and related embodiments, such as for rational design of plazomicin-specific antibodies antigen-binding domains thereof as provided herein, include VMD which is a molecular visualization program for displaying, animating, and analyzing large biomolecular systems using 3-D graphics and built-in scripting (see the website for the Theoretical and Computational Biophysics Group, University of Illinois at Urbana-Champagne, at ks.uiuc.edu/Research/vmd/. Many other computer programs are known in the art and available to the skilled person and which allow for determining atomic dimensions from space-filling models (van der Waals radii) of energy-minimized conformations; GRID, which seeks to determine regions of high affinity for different chemical groups, thereby enhancing binding, Monte Carlo searches, which calculate mathematical alignment, and CHARMM (Brooks et al. (1983) J. Comput. Chem. 4:187-217) and AMBER (Weiner et al (1981) J. Comput. Chem. 106: 765), which assess force field calculations, and analysis (see also, Eisenfield et al. (1991) Am. J. Physiol. 261:C376-386; Lybrand (1991) J. Pharm. Belg. 46:49-54; Froimowitz (1990) Biotechniques 8:640-644; Burbam et al. (1990) Proteins 7:99-111; Pedersen (1985) Environ. Health Perspect. 61:185-190; and Kini et al. (1991) J. Biomol. Struct. Dyn. 9:475-488). A variety of appropriate computational computer programs are also commercially available, such as from Schrödinger (Munich, Germany).

The isolated antibodies and antigen-binding fragments described herein can be included in a composition, which may be a liquid or a solid composition. For example, in some embodiments, the isolated antibodies or antigen-binding fragments are included in an aqueous liquid. In some embodiments, the isolated antibodies and antigen-binding fragments are included in a lyophilized solid. In some embodiments, the composition is sterile. The composition can include one or more carriers, excipients, or stabilizers. In some embodiments, the one or more carriers, excipients, or stabilizers are pharmaceutically acceptable such that they are nontoxic to the cell or mammal being exposed to the composition at the dosages and concentrations employed. Examples of pharmaceutically acceptable carriers include a saline solution; buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, polyethylene glycol (PEG), and PLURONICS.

### Articles of Manufacture, Kits, and Devices

In another teaching of the disclosure, an article of manufacture or a kit containing materials useful for determining a level or amount of plazomicin or a pharmaceutically acceptable salt thereof in a sample (such as a biological sample) is provided. In some teachings, the article of manufacture or kit includes a container suitable for containing a composition comprising an antibody or antibody fragment thereof that specifically binds to plazomicin or a pharmaceutically acceptable salt thereof. Suitable containers include, for example, bottles, vials, syringes, IV solution bags, etc. The containers may be formed from a variety of materials such as glass or plastic. The article of manufacture or kit can include a label on the container or a package inserts. A "package insert" refers to instructions that are included in commercial packages that contain instructions for use of the antibody or antigen-binding fragment thereof. For example, in some teachings, the instructions provide instruction for performing an assay for detecting plazomicin or a pharmaceutically acceptable salt thereof in a biological sample and/or for determining an amount of plazomicin or a pharmaceutically acceptable salt thereof in a sample. In some teachings, the article of manufacture or kit includes reagents for determining a level or amount of plazomicin or a pharmaceutically acceptable salt thereof in a sample. Such reagents can include, for example, secondary antibodies, fluorophores, buffers, or any other materials for performing an assay, such as an ELISA assay. In some teachings, the article or manufacture or kit includes plazomicin for use in connection with performing or carrying out the immunoassay. The reagents can be provided in the same container as the antibody or antigen binding fragment, or in a different container.

Also taught herein is a solid support comprising an antibody or antigen-binding fragment described herein attached to the support. The antibody or antigen-binding fragment can be attached to the support by any suitable method. The support comprising the attached antibody or antigen-binding fragment can be used, for example, to perform an assay for determining an amount or level of plazomicin or a pharmaceutically acceptable salt thereof in a sample (such as a biological sample). The solid support can be, for example a bead, a column, an array, an assay plate, a microwell (e.g. well of an ELISA plate), a stick, a filter, or a strip. Exemplary materials for the surface include polymers, latex, polystyrene, PMMA or silica. Also provided herein is a device comprising the solid support, which can be used for the assay.

Also taught herein is a solid support comprising plazomicin or a pharmaceutically acceptable salt thereof bound or attached to the support. The plazomicin or pharmaceutically acceptable salt thereof can be attached to the support by any suitable method. The support comprising the attached plazomicin or pharmaceutically acceptable salt thereof can be used, for example, in conjunction with an antibody or antigen-binding fragment specific for the plazomicin or pharmaceutically acceptable salt thereof, such as to perform an assay for determining an amount or level of plazomicin or a pharmaceutically acceptable salt thereof in a sample (such as a biological sample). The solid support can be, for example a bead, a column, an array, an assay plate, a microwell (e.g. well of an ELISA plate), a stick, a filter, or a strip. Exemplary materials for the surface include polymers, latex, polystyrene, PMMA or silica. Also provided herein is a device comprising the solid support, which can be used for the assay.

### Nucleic Acids and Polynucleotides

The present invention further provides in certain embodiments an isolated nucleic acid encoding an antibody, or antigen-binding fragment thereof, as described herein (*e.g.*, a nucleic acid which codes for a CDR or VH or VL domain). In some embodiments, the nucleic acid encodes a variable heavy chain region of an antibody or antigen-binding fragment thereof described herein. In some embodiments, the nucleic acid encodes a variable light chain region of an antibody or antigen-binding fragment thereof described herein. Nucleic acids include deoxyribonucleic acids (DNA) and ribonucleic acids (RNA). These and related embodiments may include polynucleotides encoding antibodies that bind plazomicin as described herein. The term "isolated polynucleotide" as used herein refers to a polynucleotide of genomic, cDNA, or synthetic origin or some combination thereof, which by virtue of its origin the isolated polynucleotide (1) is not associated with all or a portion of a polynucleotide in which the isolated polynucleotide is found in nature, (2) is linked to a polynucleotide to which it is not linked in nature, or (3) does not occur in nature as part of a larger sequence.

The term "operably linked" means that the components to which the term is applied are in a relationship that allows them to carry out their inherent functions under suitable conditions. For example, a transcription control sequence "operably linked" to a protein coding sequence is ligated thereto so that expression of the protein coding sequence is achieved under conditions compatible with the transcriptional activity of the control sequences.

The term "control sequence" as used herein refers to polynucleotide sequences that can affect expression, processing or intracellular localization of coding sequences to which they are ligated or operably linked. The nature of such control sequences may depend upon the host organism. In particular embodiments, transcription control sequences for prokaryotes may include a promoter, ribosomal binding site, and transcription termination sequence. In further particular embodiments, transcription control sequences for eukaryotes may include promoters comprising one or a plurality of recognition sites for transcription factors, transcription enhancer sequences, transcription termination sequences and polyadenylation sequences. In certain embodiments, "control sequences" can include leader sequences and/or fusion partner sequences.

The term "polynucleotide" as referred to herein means single-stranded or double-stranded nucleic acid polymers. In certain embodiments, the nucleotides comprising the polynucleotide can be RNA or DNA or a modified form of either type of nucleotide, such as a modified messenger RNA. Said modifications may include, but are not limited to, base modifications such as bromouridine, ribose modifications such as arabinoside and 2',3'-dideoxyribose and internucleotide linkage modifications such as phosphorothioate, phosphorodithioate, phosphoroselenoate, phosphorodiselenoate, phosphoroanilothioate, phoshoraniladate and phosphoroamidate. The term "polynucleotide" specifically includes single and double stranded forms of DNA.

The term "naturally occurring nucleotides" includes DNA (A, T, C, G) and RNA (A, U, C, G) nucleotides. The term "modified nucleotides" includes nucleotides with modified or substituted sugar groups and the like, base modifications, ribose modifications, and internucleotide linkage modifications. One skilled in the art will understand that a modified nucleotide may comprise alterations that are not naturally occurring, such that a polynucleotide sequence comprising one or more modified nucleotides is also non-naturally occurring. The term "oligonucleotide linkages" includes oligonucleotide linkages such as phosphorothioate, phosphorodithioate, phosphoroselenoate, phosphorodiselenoate, phosphoroanilothioate, phoshoraniladate, phosphoroamidate, and the like (see, *e.g.*, LaPlanche et al., 1986, Nucl. Acids Res., 14:9081; Stec et al., 1984, J. Am. Chem. Soc., 106:6077; Stein et al., 1988, Nucl. Acids Res., 16:3209; Zon et al., 1991, Anti-Cancer Drug Design, 6:539; Zon et al., 1991, OLIGONUCLEOTIDES AND ANALOGUES: A PRACTICAL APPROACH, pp. 87-108 (F. Eckstein, Ed.), Oxford University Press, Oxford England; Stec et al., U.S. Pat. No. 5,151,510; Uhlmann and Peyman, 1990, Chemical Reviews, 90:543). An oligonucleotide can include a detectable label to enable detection of the oligonucleotide or hybridization thereof.

Embodiments of the present invention also include oligonucleotides, whether for detection, amplification, antisense therapies, or other purpose. For these and related purposes, the term "oligonucleotide" or "oligo" or "oligomer" is intended to encompass a singular "oligonucleotide" as well as plural "oligonucleotides," and refers to any polymer of two or more of nucleotides, nucleosides, nucleobases or related compounds used as a reagent in the amplification methods of the present invention, as well as subsequent detection methods. The oligonucleotide may be DNA and/or RNA and/or analogs thereof.

The term oligonucleotide does not necessarily denote any particular function to the reagent, rather, it is used generically to cover all such reagents described herein. An oligonucleotide may serve various different functions, *e.g*., it may function as a primer if it is capable of hybridizing to a complementary strand and can further be extended in the presence of a nucleic acid polymerase, it may provide a promoter if it contains a sequence recognized by an RNA polymerase and allows for transcription, and it may function to prevent hybridization or impede primer extension if appropriately situated and/or modified. An oligonucleotide may also function as a probe. An oligonucleotide can be virtually any length, limited only by its specific function, *e.g.,* in an amplification reaction, in detecting an amplification product of the amplification reaction, or in an antisense or RNA interference application. Any of the oligonucleotides described herein can be used as a primer or a probe.

The term "primer" as used herein refers to a single-stranded oligonucleotide capable of acting as a point of initiation for template-directed DNA synthesis under suitable conditions defined, for example, by buffer and temperature, in the presence of four different nucleoside triphosphates and an agent for polymerization, such as a DNA or RNA polymerase or reverse transcriptase. The length of the primer, in any given case, depends on, for example, the intended use of the primer, and generally ranges from about 15 to 30 nucleotides, although shorter and longer primers may be used. Short primer molecules generally require cooler temperatures to form sufficiently stable hybrid complexes with the template. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified.

The term "probe" as used herein includes a surface-immobilized or soluble but capable of being immobilized molecule that can be recognized by a particular target. Probes and primers as used herein typically comprise at least 10-15 contiguous nucleotides of a known sequence. In order to enhance specificity, longer probes and primers may also be employed, such as probes and primers that comprise at least 20, 25, 30, 40, 50, 60, 70, 80, 90, 100, or at least 150 nucleotides of a reference sequence or its complement, *e.g*., nucleotide sequences encoding anti-plazomicin antibodies, antigen-binding fragments thereof. Probes and primers may be considerably longer than these examples, and it is understood that any length supported by the knowledge in the art and the specification, including the tables, figures, and Sequence Listing, may be used.

As will be understood by those skilled in the art, polynucleotides may include genomic sequences, extra-genomic and plasmid-encoded sequences and smaller engineered gene segments that express, or may be adapted to express, proteins, polypeptides, peptides and the like. Such segments may be naturally isolated, or modified synthetically by the skilled person.

As will be also recognized by the skilled artisan, polynucleotides may be single-stranded (coding or antisense) or double-stranded, and may be DNA (genomic, cDNA or synthetic) or RNA molecules. RNA molecules may include heteronuclear RNA (hnRNA) molecules, which contain introns and correspond to a DNA molecule in a one-to-one manner, and messenger RNA (mRNA) molecules, which do not contain introns. Additional coding or non-coding sequences may, but need not, be present within a polynucleotide according to the present disclosure, and a polynucleotide may, but need not, be linked to other molecules and/or support materials. Polynucleotides may comprise a native sequence or may comprise a sequence that encodes a variant or derivative of such a sequence.

Therefore, according to these and related embodiments, the present disclosure also provides polynucleotides encoding the anti-plazomicin antibodies described herein. In certain embodiments, polynucleotides are provided that comprise some or all of a polynucleotide sequence encoding an antibody as described herein and complements of such polynucleotides. For example, SEQ ID NOs:1-21 comprise some or all of a polynucleotide sequence encoding an antibody, or antibody fragment thereof, as described herein. Due to the degeneracy of the genetic code, it is understood that a variety of different polynucleotide sequences can encode the same polypeptide, and the present invention contemplates all polynucleotides that encode any of the polypeptides described herein. In certain embodiments, any of the polynucleotides described herein may be codon-optimized, e.g., to enhance expression or stability during recombinant production.

In related embodiments, the present disclosure also provides for "polynucleotide variants" that may have substantial identity to a polynucleotide sequence encoding an anti-plazomicin antibody described herein. For example, a polynucleotide variant may be a polynucleotide comprising at least 70%, 75%, 80%, 85%, 90%, 95%, 96%, 97%, 98%, or 99% or higher sequence identity compared to a reference polynucleotide sequence such as a sequence encoding an antibody described herein, using the methods described herein (*e.g.*, BLAST analysis using standard parameters, as described below). One skilled in this art will recognize that these values can be appropriately adjusted to determine corresponding identity of proteins encoded by two nucleotide sequences by taking into account codon degeneracy, amino acid similarity, reading frame positioning and the like.

Typically, polynucleotide variants will contain one or more substitutions, additions, deletions and/or insertions, such that the binding affinity of the antibody encoded by the variant polynucleotide is not substantially diminished relative to an antibody encoded by a polynucleotide sequence specifically set forth herein. For example, a polynucleotide variant may comprise at least about 1, 2, 3, 4, 5, 6, 7, 8, 9, 10, 11, 12, 13, 14, 15, or more substitutions, additions, deletions, or insertions compared to a reference polynucleotide sequence, such as a sequence encoding and antibody, or antigen-binding fragment thereof, described herein.

In some embodiments, a polynucleotide encoding a variable light chain of an anti-plazomicin antibody comprises a nucleotide sequence having at least 80%, at least 85%, at least 90%, at least 92%, at least 95%, at least 96%, at least 97%, at least 98%, at least 99%, or 100% identity to the nucleotide sequence selected from any one of SEQ ID Nos: 2, 3, 5, 7, 9, 11, 13, 15, 17, 19 or 21 or a complement of any of the foregoing. In some embodiments, a polynucleotide encoding a variable heavy chain of an anti-plazomicin antibody comprises a nucleotide sequence having at least 80%, at least 85%, at least 90%, at least 92%, at least 95%, at least 96%, at least 97%, at least 98%, at least 99%, or 100% identity to the nucleotide sequence selected from any one of SEQ ID Nos: 1, 4, 6, 8, 10, 12, 14, 16, 18, or 20 or a complement of any of the foregoing.

In related embodiments, the present disclosure provides for "polynucleotide fragments" that may comprise or consist essentially of various lengths of contiguous stretches of sequence identical to or complementary to a sequence encoding an antibody as described herein. For example, polynucleotides are provided that comprise or consist essentially of at least about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95, 100, 110, 120, 130, 140, 150, 200, 300, 400, 500 or 1000 or more contiguous nucleotides of a sequences the encodes an antibody, or antigen-binding fragment thereof, disclosed herein as well as all intermediate lengths there between. It will be readily understood that "intermediate lengths", in this context, means any length between the quoted values, such as 50, 51, 52, 53, *etc*.; 100, 101, 102, 103, *etc*.; 150, 151, 152, 153, *etc*.; including all integers through 200-500; 500-1,000, and the like. A polynucleotide sequence as described here may be extended at one or both ends by additional nucleotides not found in the native sequence. This additional sequence may consist of 1, 2, 3, 4, 5, 6, 7, 8, 9, 10, 11, 12, 13, 14, 15, 16, 17, 18, 19, or 20 nucleotides at either end of a polynucleotide encoding an antibody described herein or at both ends of a polynucleotide encoding an antibody described herein.

In some embodiments, polynucleotides are provided that are capable of hybridizing under moderate to high stringency conditions to a polynucleotide sequence encoding an antibody, or antigen-binding fragment thereof, provided herein, or a fragment thereof, or a complementary sequence thereof, *e.g*., oligonucleotides, primers, or probes. Hybridization techniques are well known in the art of molecular biology. For purposes of illustration, suitable moderately stringent conditions for testing the hybridization of a polynucleotide as provided herein with other polynucleotides include prewashing in a solution of 5 X SSC, 0.5% SDS, 1.0 mM EDTA (pH 8.0); hybridizing at 50°C-60°C, 5 X SSC, overnight; followed by washing twice at 65°C for 20 minutes with each of 2X, 0.5X and 0.2X SSC containing 0.1% SDS. One skilled in the art will understand that the stringency of hybridization can be readily manipulated, such as by altering the salt content of the hybridization solution and/or the temperature at which the hybridization is performed. For example, suitable highly stringent hybridization conditions can include those described above, with the exception that the temperature of hybridization is increased, *e.g*., to 60°C-65°C or 65°C-70°C.

In certain embodiments, the polynucleotides described above, *e.g*., polynucleotide variants, fragments and hybridizing sequences, encode antibodies that bind plazomicin, or antigen-binding fragments thereof. In certain embodiments, such polynucleotides encode antibodies or antigen-binding fragments, or CDRs thereof that bind to plazomicin at least about 50%, at least about 70%, and in certain embodiments, at least about 90% as well as an antibody sequence specifically set forth herein. In further embodiments, such polynucleotides encode antibodies or antigen-binding fragments, or CDRs thereof, that bind to plazomicin with greater affinity than the antibodies set forth herein, for example, that bind quantitatively at least about 105%, 106%, 107%, 108%, 109%, or 110% as well as an antibody sequence specifically set forth herein.

As described elsewhere herein, determination of the three-dimensional structures of representative polypeptides (*e*.*g*., variant plazomicin-specific antibodies as provided herein, for instance, an antibody protein having an antigen-binding fragment as provided herein) may be made through routine methodologies such that substitution, addition, deletion or insertion of one or more amino acids with selected natural or non-natural amino acids can be virtually modeled for purposes of determining whether a so derived structural variant retains the space-filling properties of presently disclosed species. A variety of computer programs are known to the skilled artisan for determining appropriate amino acid substitutions (or appropriate polynucleotides encoding the amino acid sequence) within an antibody such that, for example, affinity is maintained or better affinity is achieved.

The polynucleotides described herein, or fragments thereof, regardless of the length of the coding sequence itself, may be combined with other DNA sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed, with the total length being limited by the ease of preparation and use in the intended recombinant DNA protocol. For example, illustrative polynucleotide segments with total lengths of about 10,000, about 5000, about 3000, about 2,000, about 1,000, about 500, about 200, about 100, about 50 base pairs in length, and the like, (including all intermediate lengths) are contemplated to be useful.

When comparing polynucleotide sequences, two sequences are said to be "identical" if the sequence of nucleotides in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J., Unified Approach to Alignment and Phylogenes, pp. 626-645 (1990); Methods in Enzymology vol. 183, Academic Press, Inc., San Diego, CA; Higgins, D.G. and Sharp, P.M., CABIOS 5:151-153 (1989); Myers, E.W. and Muller W., CABIOS 4:11-17 (1988); Robinson, E.D., Comb. Theor 11:105 (1971); Santou, N. Nes, M., Mol. Biol. Evol. 4:406-425 (1987); Sneath, P.H.A. and Sokal, R.R., Numerical Taxonomy - the Principles and Practice of Numerical Taxonomy, Freeman Press, San Francisco, CA (1973); Wilbur, W.J. and Lipman, D.J., Proc. Natl. Acad., Sci. USA 80:726-730 (1983).

Alternatively, optimal alignment of sequences for comparison may be conducted by the local identity algorithm of Smith and Waterman, Add. APL. Math 2:482 (1981), by the identity alignment algorithm of Needleman and Wunsch, J. Mol. Biol. 48:443 (1970), by the search for similarity methods of Pearson and Lipman, Proc. Natl. Acad. Sci. USA 85: 2444 (1988), by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One example of algorithms that are suitable for determining percent sequence identity and sequence similarity are the BLAST and BLAST 2.0 algorithms, which are described in Altschul et al., Nucl. Acids Res. 25:3389-3402 (1977), and Altschul et al., J. Mol. Biol. 215:403-410 (1990), respectively. BLAST and BLAST 2.0 can be used, for example with the parameters described herein, to determine percent sequence identity among two or more the polynucleotides. Software for performing BLAST analyses is publicly available through the National Center for Biotechnology Information. In one illustrative example, cumulative scores can be calculated using, for nucleotide sequences, the parameters M (reward score for a pair of matching residues; always >0) and N (penalty score for mismatching residues; always <0). Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a word length (W) of 11, and expectation (E) of 10, and the BLOSUM62 scoring matrix (see Henikoff and Henikoff, Proc. Natl. Acad. Sci. USA 89:10915 (1989)) alignments, (B) of 50, expectation (E) of 10, M=5, N=-4 and a comparison of both strands.

In certain embodiments, the "percentage of sequence identity" is determined by comparing two optimally aligned sequences over a window of comparison of at least 20 positions, wherein the portion of the polynucleotide sequence in the comparison window may comprise additions or deletions (*i.e.,* gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases occurs in both sequences to yield the number of matched positions, dividing the number of matched positions by the total number of positions in the reference sequence (*i.e.,* the window size) and multiplying the results by 100 to yield the percentage of sequence identity.

It will be appreciated by those of ordinary skill in the art that, as a result of the degeneracy of the genetic code, there are many nucleotide sequences that encode an antibody as described herein. Some of these polynucleotides bear minimal sequence identity to the nucleotide sequence of the native or original polynucleotide sequences that encode antibodies that bind to plazomicin. Nonetheless, polynucleotides that vary due to differences in codon usage are expressly contemplated by the present disclosure. In certain embodiments, sequences that have been codon-optimized for mammalian expression are specifically contemplated.

Therefore, in a further embodiment of the invention, a mutagenesis approach, such as site-specific mutagenesis, may be employed for the preparation of variants and/or derivatives of the antibodies described herein. By this approach, specific modifications in a polypeptide sequence can be made through mutagenesis of the underlying polynucleotides that encode them. These techniques provides a straightforward approach to prepare and test sequence variants, for example, incorporating one or more of the foregoing considerations, by introducing one or more nucleotide sequence changes into the polynucleotide.

Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Mutations may be employed in a selected polynucleotide sequence to improve, alter, decrease, modify, or otherwise change the properties of the polynucleotide itself, and/or alter the properties, activity, composition, stability, or primary sequence of the encoded polypeptide.

In certain embodiments, the inventors contemplate the mutagenesis of the polynucleotide sequences that encode an antibody disclosed herein, or an antigen-binding fragment thereof, to alter one or more properties of the encoded polypeptide, such as the binding affinity of the antibody or the antigen-binding fragment thereof. The techniques of site-specific mutagenesis are well-known in the art, and are widely used to create variants of both polypeptides and polynucleotides. For example, site-specific mutagenesis is often used to alter a specific portion of a DNA molecule. In such embodiments, a primer comprising typically about 14 to about 25 nucleotides or so in length is employed, with about 5 to about 10 residues on both sides of the junction of the sequence being altered.

The term "vector" is used to refer to any molecule (*e*.*g*., nucleic acid, plasmid, or virus) used to transfer coding information to a host cell. The term "expression vector" refers to a vector that is suitable for transformation of a host cell and contains nucleic acid sequences that direct and/or control expression of inserted heterologous nucleic acid sequences. Expression includes, but is not limited to, processes such as transcription, translation, and RNA splicing, if introns are present.

A variety of expression vector/host systems are known and may be utilized to contain and express polynucleotide sequences. These include, but are not limited to, microorganisms such as bacteria transformed with recombinant bacteriophage, plasmid, or cosmid DNA expression vectors; yeast transformed with yeast expression vectors; insect cell systems infected with virus expression vectors (*e*.*g*., baculovirus); plant cell systems transformed with virus expression vectors (*e*.*g*., cauliflower mosaic virus, CaMV; tobacco mosaic virus, TMV) or with bacterial expression vectors (*e.g*., Ti or pBR322 plasmids); or animal cell systems, including mammalian cell and more specifically human cell systems.

As will be appreciated by those of skill in the art, site-specific mutagenesis techniques have often employed a phage vector that exists in both a single stranded and double stranded form. Typical vectors useful in site-directed mutagenesis include vectors such as the M13 phage. These phages are readily commercially-available and their use is generally well-known to those skilled in the art. Double-stranded plasmids are also routinely employed in site directed mutagenesis that eliminates the step of transferring the gene of interest from a plasmid to a phage.

In general, site-directed mutagenesis in accordance herewith is performed by first obtaining a single-stranded vector or melting apart of two strands of a double-stranded vector that includes within its sequence a DNA sequence that encodes the desired peptide. An oligonucleotide primer bearing the desired mutated sequence is prepared, generally synthetically. This primer is then annealed with the single-stranded vector, and subjected to DNA polymerizing enzymes such as *E. coli* polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform appropriate cells, such as *E. coli* cells, and clones are selected which include recombinant vectors bearing the mutated sequence arrangement.

The preparation of sequence variants of the selected peptide-encoding DNA segments using site-directed mutagenesis provides a means of producing potentially useful species and is not meant to be limiting as there are other ways in which sequence variants of peptides and the DNA sequences encoding them may be obtained. For example, recombinant vectors encoding the desired peptide sequence may be treated with mutagenic agents, such as hydroxylamine, to obtain sequence variants. Specific details regarding these methods and protocols are found in the teachings of Maloy *et al.,* 1994; Segal, 1976; Prokop and Bajpai, 1991; Kuby, 1994; and Maniatis *et al.,* 1982.

As used herein, the term "oligonucleotide directed mutagenesis procedure" refers to template-dependent processes and vector-mediated propagation which result in an increase in the concentration of a specific nucleic acid molecule relative to its initial concentration, or in an increase in the concentration of a detectable signal, such as amplification. As used herein, the term "oligonucleotide directed mutagenesis procedure" is intended to refer to a process that involves the template-dependent extension of a primer molecule. The term template dependent process refers to nucleic acid synthesis of a RNA or a DNA molecule wherein the sequence of the newly synthesized strand of nucleic acid is dictated by the well-known rules of complementary base pairing (see, for example, Watson, 1987). Typically, vector mediated methodologies involve the introduction of the nucleic acid fragment into a DNA or RNA vector, the clonal amplification of the vector, and the recovery of the amplified nucleic acid fragment. Examples of such methodologies are provided by U. S. Patent No. 4,237,224.

Another approach for the production of polypeptide variants, recursive sequence recombination, as described in U.S. Patent No. 5,837,458, may be employed. In this approach, iterative cycles of recombination and screening or selection are performed to "evolve" individual polynucleotide variants having, for example, increased binding affinity. Certain embodiments also provide constructs in the form of plasmids, vectors, transcription or expression cassettes which comprise at least one polynucleotide as described herein.

In some embodiments, the nucleic acids encoding a subject monoclonal antibody are introduced directly into a host cell, and the cell incubated under conditions sufficient to induce expression of the encoded antibody. The antibodies of this disclosure are prepared using standard techniques well known to those of skill in the art in combination with the polypeptide and nucleic acid sequences provided herein. The polypeptide sequences may be used to determine appropriate nucleic acid sequences encoding the particular antibody disclosed thereby. The nucleic acid sequence may be optimized to reflect particular codon "preferences" for various expression systems according to standard methods well known to those of skill in the art.

According to certain related embodiments there is provided a recombinant host cell which comprises one or more constructs as described herein; a nucleic acid encoding any antibody, CDR, VH or VL domain, or antigen-binding fragment thereof; and a method of production of the encoded product, which method comprises expression from encoding nucleic acid therefor. Expression may conveniently be achieved by culturing under appropriate conditions recombinant host cells containing the nucleic acid. Following production by expression, an antibody or antigen-binding fragment thereof, may be isolated and/or purified using any suitable technique, and then used as desired.

Antibodies or antigen-binding fragments thereof as provided herein, and encoding nucleic acid molecules and vectors, may be isolated and/or purified, *e.g.* from their natural environment, in substantially pure or homogeneous form, or, in the case of nucleic acid, free or substantially free of nucleic acid or genes of origin other than the sequence encoding a polypeptide with the desired function. Nucleic acid may comprise DNA or RNA and may be wholly or partially synthetic. Reference to a nucleotide sequence as set out herein encompasses a DNA molecule with the specified sequence, and encompasses a RNA molecule with the specified sequence in which U is substituted for T, unless context requires otherwise.

### Methods of Antibody Expression and Generation

This disclosure is not intended to be limited with regard to the source of the antibody or the manner in which it is made (*e.g*., by hybridoma, phage selection, recombinant expression, transgenic animals, etc.). Various procedures known within the art may be used for the production of polyclonal or monoclonal antibodies directed against plazomicin (*See*, *for example,* Antibodies: A Laboratory Manual, Harlow E, and Lane D, 1988, Cold Spring Harbor Laboratory Press, Cold Spring Harbor, NY). In some embodiments, the anti-plazomicin antibodies, and antigen binding fragments thereof, are recombinantly expressed and produced.

Systems for cloning and expression of a polypeptide in a variety of different host cells are well known. Suitable host cells include bacteria, mammalian cells, yeast and baculovirus systems. Mammalian cell lines available in the art for expression of a heterologous polypeptide include Chinese hamster ovary cells, HeLa cells, baby hamster kidney cells, NSO mouse melanoma cells and many others.

Certain embodiments may employ *E*. *coli*-based expression systems (*see, e.g.,* Structural Genomics Consortium et al., Nature Methods. 5:135-146, 2008). These and related embodiments may rely partially or totally on ligation-independent cloning (LIC) to produce a suitable expression vector. In specific embodiments, protein expression may be controlled by a T7 RNA polymerase (*e.g.,* pET vector series). These and related embodiments may utilize the expression host strain BL21(DE3), a λDE3 lysogen of BL21 that supports T7-mediated expression and is deficient in Ion and ompT proteases for improved target protein stability. Also included are expression host strains carrying plasmids encoding tRNAs rarely used in *E. coli,* such as ROSETTA^{™} (DE3) and Rosetta 2 (DE3) strains. Cell lysis and sample handling may also be improved using reagents sold under the trademarks BENZONASE^{®} nuclease and BUGBUSTER^{®} Protein Extraction Reagent. For cell culture, auto-inducing media can improve the efficiency of many expression systems, including high-throughput expression systems. Media of this type (*e.g.,* OVERNIGHT EXPRESS^{™} Autoinduction System) gradually elicit protein expression through metabolic shift without the addition of artificial inducing agents such as IPTG. Particular embodiments employ hexahistidine tags (such as those sold under the trademark HIS•TAG^{®} fusions), followed by immobilized metal affinity chromatography (IMAC) purification, or related techniques. In certain aspects, however, clinical grade proteins can be isolated from *E*. *coli* inclusion bodies, without or without the use of affinity tags *(see, e.g.,* Shimp et al., Protein Expr Purif. 50:58-67, 2006). As a further example, certain embodiments may employ a cold-shock induced *E. coli* high-yield production system, because over-expression of proteins in *Escherichia coli* at low temperature improves their solubility and stability (*see, e.g.,* Qing et al., Nature Biotechnology. 22:877-882, 2004).

In the yeast *Saccharomyces cerevisiae*, a number of vectors containing constitutive or inducible promoters such as alpha factor, alcohol oxidase, and PGH may be used. For reviews, *see* Ausubel *et al.* (supra) and Grant et al., Methods Enzymol. 153:516-544 (1987). Also included are *Pichia pandoris* expression systems (*see, e.g.,* Li et al., Nature Biotechnology. 24, 210 - 215, 2006; and Hamilton et al., Science, 301:1244, 2003). Certain embodiments include yeast systems that are engineered to selectively glycosylate proteins, including yeast that have humanized N-glycosylation pathways, among others (*see, e.g.,* Hamilton et al., Science. 313:1441-1443, 2006; Wildt et al., Nature Reviews Microbiol. 3:119-28, 2005; and Gerngross et al., Nature-Biotechnology. 22:1409 -1414, 2004; U.S. Patent Nos. 7,629,163; 7,326,681; and 7,029,872). Merely by way of example, recombinant yeast cultures can be grown in Fernbach Flasks or 15L, 50L, 100L, and 200L fermentors, among others.

In cases where plant expression vectors are used, the expression of sequences encoding polypeptides may be driven by any of a number of promoters. For example, viral promoters such as the 35S and 19S promoters of CaMV may be used alone or in combination with the omega leader sequence from TMV (Takamatsu, EMBO J. 6:307-311 (1987)). Alternatively, plant promoters such as the small subunit of RUBISCO or heat shock promoters may be used (Coruzzi et al., EMBO J. 3:1671-1680 (1984); Broglie et al., Science 224:838-843 (1984); and Winter et al., Results Probl. Cell Differ. 17:85-105 (1991)). These constructs can be introduced into plant cells by direct DNA transformation or pathogen-mediated transfection. Such techniques are described in a number of generally available reviews (see, *e.g.*, Hobbs in McGraw Hill, Yearbook of Science and Technology, pp. 191-196 (1992)).

An insect system may also be used to express a polypeptide of interest. For example, in one such system, *Autographa californica* nuclear polyhedrosis virus (AcNPV) is used as a vector to express foreign genes in *Spodoptera frugiperda* cells or in *Trichoplusia* larvae. The sequences encoding the polypeptide may be cloned into a non-essential region of the virus, such as the polyhedrin gene, and placed under control of the polyhedrin promoter. Successful insertion of the polypeptide-encoding sequence will render the polyhedrin gene inactive and produce recombinant virus lacking coat protein. The recombinant viruses may then be used to infect, for example, *S. frugiperda* cells or *Trichoplusia larvae* in which the polypeptide of interest may be expressed (Engelhard et al., Proc. Natl. Acad. Sci. U.S.A. 91:3224-3227 (1994)). Also included are baculovirus expression systems, including those that utilize SF9, SF21, and Tni cells (*see, e.g.,* Murphy and Piwnica-Worms, Curr Protoc Protein Sci. Chapter 5:Unit5.4, 2001). Insect systems can provide post-translation modifications that are similar to mammalian systems.

In mammalian host cells, a number of viral-based expression systems are generally available. For example, in cases where an adenovirus is used as an expression vector, sequences encoding a polypeptide of interest may be ligated into an adenovirus transcription/translation complex consisting of the late promoter and tripartite leader sequence. Insertion in a non-essential E1 or E3 region of the viral genome may be used to obtain a viable virus which is capable of expressing the polypeptide in infected host cells (Logan & Shenk, Proc. Natl. Acad. Sci. U.S.A. 81:3655-3659 (1984)). In addition, transcription enhancers, such as the Rous sarcoma virus (RSV) enhancer, may be used to increase expression in mammalian host cells.

Examples of useful mammalian host cell lines include monkey kidney CV1 line transformed by SV40 (COS-7, ATCC CRL 1651); human embryonic kidney line (293 or 293 cells sub-cloned for growth in suspension culture, Graham et al., J. Gen Virol. 36:59 (1977)); baby hamster kidney cells (BHK, ATCC CCL 10); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TR1 cells (Mather et al., Annals N.Y. Acad. Sci. 383:44-68 (1982)); MRC 5 cells; FS4 cells; and a human hepatoma line (Hep G2). Useful mammalian host cell lines also include Chinese hamster ovary (CHO) cells, including DHFR-CHO cells (Urlaub et al., PNAS USA 77:4216 (1980)); and myeloma cell lines such as NSO and Sp2/0. For a review of certain mammalian host cell lines suitable for antibody production, *see, e.g*., Yazaki and Wu, Methods in Molecular Biology, Vol. 248 (B. K.C Lo, ed., Humana Press, Totowa, N.J., 2003), pp. 255-268. Certain mammalian cell expression systems include CHO and HEK293-cell based expression systems. Mammalian expression systems can utilize attached cell lines, for example, in T-flasks, roller bottles, or cell factories, or suspension cultures, for example, in 1L and 5L spinners, 5L, 14L, 40L, 100L and 200L stir tank bioreactors, or 20/50L and 100/200L WAVE bioreactors, among others known in the art.

Also included is cell-free expression of proteins. These and related embodiments typically utilize purified RNA polymerase, ribosomes, tRNA and ribonucleotides; these reagents may be produced by extraction from cells or from a cell-based expression system.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids, viral *e.g.* phage, or phagemid, as appropriate. For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Second Edition, Ausubel et al. eds., John Wiley & Sons, 1992, or subsequent updates thereto.

The term "host cell" is used to refer to a cell into which has been introduced, or which is capable of having introduced into it, a nucleic acid sequence encoding one or more of the herein described antibodies, and which further expresses or is capable of expressing a selected gene of interest, such as a gene encoding any herein described antibody. The term includes the progeny of the parent cell, whether or not the progeny are identical in morphology or in genetic make-up to the original parent, so long as the selected gene is present. The introduction of the nucleic acid sequence may employ any available technique. For eukaryotic cells, suitable techniques may include calcium phosphate transfection, DEAE-Dextran, electroporation, liposome-mediated transfection and transduction using retrovirus or other virus, *e.g.* vaccinia or, for insect cells, baculovirus. For bacterial cells, suitable techniques may include calcium chloride transformation, electroporation and transfection using bacteriophage. The introduction may be followed by causing or allowing expression from the nucleic acid, *e.g.* by culturing host cells under conditions for expression of the gene. In one embodiment, the nucleic acid is integrated into the genome (*e.g*. chromosome) of the host cell. Integration may be promoted by inclusion of sequences which promote recombination with the genome, in accordance-with standard techniques.

The term "transduction" is used to refer to the transfer of genes from one bacterium to another, usually by a phage. "Transduction" also refers to the acquisition and transfer of eukaryotic cellular sequences by retroviruses. The term "transfection" is used to refer to the uptake of foreign or exogenous DNA by a cell, and a cell has been "transfected" when the exogenous DNA has been introduced inside the cell membrane. A number of transfection techniques are well known in the art and are disclosed herein. See, *e.g*., Graham et al., 1973, Virology 52:456; Sambrook et al., 2001, MOLECULAR CLONING, A LABORATORY MANUAL, Cold Spring Harbor Laboratories; Davis et al., 1986, BASIC METHODS IN MOLECULAR BIOLOGY, Elsevier; and Chu et al., 1981, Gene 13:197. Such techniques can be used to introduce one or more exogenous DNA moieties into suitable host cells.

The term "transformation" as used herein refers to a change in a cell's genetic characteristics, and a cell has been transformed when it has been modified to contain a new DNA. For example, a cell is transformed where it is genetically modified from its native state. Following transfection or transduction, the transforming DNA may recombine with that of the cell by physically integrating into a chromosome of the cell, or may be maintained transiently as an episomal element without being replicated, or may replicate independently as a plasmid. A cell is considered to have been stably transformed when the DNA is replicated with the division of the cell. The term "naturally occurring" or "native" when used in connection with biological materials such as nucleic acid molecules, polypeptides, host cells, and the like, refers to materials which are found in nature and are not manipulated by a human. Similarly, "non-naturally occurring" or "non-native" as used herein refers to a material that is not found in nature or that has been structurally modified or synthesized by a human.

In a further embodiment of invention, anti-plazomicin antibodies are derived from rabbit monoclonal antibodies, and in particular are generated using RabMAb^{®} technology. These antibodies are advantageous as they require minimal sequence modifications, thereby facilitating retention of functional properties after humanization using mutational lineage guided (MLG) humanization technology (see e.g., U.S. Patent No. 7,462,697). Thus, illustrative methods for making the anti-plazomicin antibodies of the present disclosure include the RabMab^{®} rabbit monoclonal antibody technology described, for example, in U.S. Patents 5,675,063 and 7,429,487. In this regard, in certain embodiments, the anti-plazomicin antibodies of the disclosure are produced in rabbits. In particular embodiments, a rabbit-derived immortal B-lymphocyte capable of fusion with a rabbit splenocyte is used to produce a hybrid cell that produces an antibody. The immortal B-lymphocyte does not detectably express endogenous immunoglobulin heavy chain and may contain, in certain embodiments, an altered immunoglobulin heavy chain-encoding gene.

In some embodiments, the anti-plazomicin antibodies, and antigen-binding fragments thereof are produced using hybridoma methods, such as those described by Kohler and Milstein, Nature, 256:495 (1975). In a hybridoma method, a mouse, hamster, or other appropriate host animal, is immunized with an immunizing agent to elicit lymphocytes that produce or are capable of producing antibodies that will specifically bind to the immunizing agent. Alternatively, the lymphocytes can be immunized *in vitro.*

The immunizing agent will typically include the antigen, a fragment thereof or a fusion construct thereof. Generally for *in vitro* immunization, either peripheral blood lymphocytes are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells can be cultured in a suitable culture medium that contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

In some embodiments, the immortalized cell lines used fuse efficiently, support stable high level expression of antibody by the selected antibody-producing cells, and are sensitive to a medium such as HAT medium. Examples of immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies. (*See* Kozbor, J. Immunol., 133:3001 (1984); Brodeur et al., Monoclonal Antibody Production Techniques and Applications, Marcel Dekker, Inc., New York, (1987) pp. 51-63)).

The culture medium in which the hybridoma cells are cultured can then be assayed for the presence of monoclonal antibodies directed against the antigen. The binding specificity of monoclonal antibodies produced by the hybridoma cells can be determined by immunoprecipitation or by an *in vitro* binding assay, such as an enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980). In certain embodiments, identified antibodies have a high degree of specificity and a high binding affinity for the target antigen, in particular for plazomicin.

After the desired hybridoma cells are identified, the clones can be subcloned by limiting dilution procedures and grown by standard methods. (*See* Goding, Monoclonal Antibodies: Principles and Practice, Academic Press, (1986) pp. 59-103). Suitable culture media for this purpose include, for example, Dulbecco's Modified Eagle's Medium and RPMI-1640 medium. Alternatively, the hybridoma cells can be grown *in vivo* as ascites in a mammal.

These just-described techniques are, in and of themselves, known as such in the art. The skilled person will, however, be able to use such techniques to obtain antibodies or antigen-binding fragments thereof according to several embodiments of the invention described herein, using routine methodology in the art.

### Methods of Use

The present disclosure teaches methods related to the use of the antibodies, and antigen-binding fragments thereof, described herein in immunoassays for the detection of plazomicin in biological samples. Certain teachings include methods of treating bacterial infections, including infections with gram-negative bacteria, multi-drug resistant bacteria, and urinary tract infections.

Teachings of methods disclosed herein are directed to determining a level of plazomicin in a biological sample comprising: (i) contacting the sample with any of the isolated antibodies, or antigen-binding fragments thereof, described herein; and (ii) detecting the presence or absence of, or amount of, the antibody bound to plazomicin by an immunoassay, thereby determining the presence or absence or, or level of, plazomicin in the sample. Contacting the sample with the isolated antibodies, or antigen-binding fragments thereof, can occur *in vitro.*

As used herein, a "biological sample" refers to a sample taken from a subject, e.g., a mammal such as a human. These can include, but are not limited to cells, tissue samples, and body fluids (including, but not limited to, mucus, blood, plasma, serum, urine, saliva, sputum, and semen). In certain teachings, the sample is a liquid matrix sample including, but not limited to, blood, plasma, serum, urine, or sputum. Samples can be provided by the subject under treatment or testing. Alternatively samples can be obtained by the physician according to routine practice in the art. In certain teachings, the sample is taken from a subject to whom plazomicin has been administered.

As used herein, "immunoassay" refers to a biochemical test that measures the presence of or concentration of an analyte, *e.g*., the compound to be detected in a solution through the use of an antibody, or antigen-binding fragment thereof, *e.g.* a plazomicin-specific antibody described herein. In some teachings, the analyte is an antigen. In further teachings, the analyte is plazomicin. Certain immunoassays employ the use of antigen-specific antibodies, *e.g.* anti-plazomicin antibodies labeled with a detectable label or "primary antibodies", to directly detect the presence of or concentration of an analyte or antigen. Certain immunoassays utilize antigen-specific antibodies that are not labeled, and require the application of one or more additional antibodies, or "secondary antibodies", to detect the presence of the primary antibody and thus indirectly detect the presence or concentration of the analyte or antigen. When performing an immunoassay, controls typically include a reaction well or tube that contains an antibody or antigen-binding fragment thereof alone (*i.e*., in the absence of antigen), wherein an amount of reactivity (*e.g.*, non-specific binding to the well) by the antibody or antigen-binding fragment thereof in the absence of the antigen is considered to be background. This allows for the accurate assessment of antigen specific-binding. Non-limiting examples of immunoassays include direct enzyme-linked immunosorbent assays (ELISAs), competitive ELISAs, cloned enzyme donor immunoassay (CEIDA), real-time immunoquantitative polymerase chain reaction (iqPCR), lateral flow tests, magnetic immunoassays, radioimmunoassays, surround optical fiber immunoassays (SOFIA), and enzyme multiplied immunoassay technique (EMIT), and turbidity assays.

A "detectable label" as use herein refers to a molecule or material that can produce a detectable (visually, electronically, or otherwise) signal that indicates the presence and/or concentration of the label in a sample. In some teachings, the antigen-specific antibody, or antigen-binding fragment thereof, is linked to a detectable label. Non-limiting examples of detectable labels include fluorescent labels, polymer particles, metal particles, haptens, enzyme labels, luminescent labels, electrochemiluminescent labels, bioluminescent labels, radioisotopes, oligonucleotides, and nanoparticles.

Examples of fluorescent labels include 5-(and 6)-carboxyfluorescein, 5- or 6-carboxyfluorescein, 6-(fluorescein)-5-(and 6)-carboxamido hexanoic acid, fluorescein isothiocyanate (FITC), rhodamine, tetramethylrhodamine, and dyes such as Cy2, Cy3, and Cy5, optionally substituted coumarin including AMCA, PerCP, phycobiliproteins including R-phycoerythrin (RPE) and allophycoerythrin (APC), Texas Red, Princeton Red, green fluorescent protein (GFP) and analogs thereof, and conjugates of R-phycoerythrin or allophycoerythrin, inorganic fluorescent labels such as particles based on semiconductor material like coated CdSe nanocrystallites.

Examples of polymer particle labels include micro particles or latex particles of polystyrene, PMMA or silica, which can be embedded with fluorescent dyes, or polymer micelles or capsules which contain dyes, enzymes or substrates.

Examples of metal particle labels include gold particles and coated gold particles, which can be converted by silver stains. Examples of haptens include dinitrophenyl (DNP), fluorescein isothiocyanate (FITC), biotin, and digoxigenin. Examples of enzymatic labels include horseradish peroxidase (HRP), alkaline phosphatase (ALP or AP), β-galactosidase (GAL), glucose-6-phosphate dehydrogenase, β-N-acetylglucosamimidase, β-glucuronidase, invertase, Xanthine Oxidase, firefly luciferase and glucose oxidase (GO). Examples of commonly used substrates for horseradish peroxidase include 3,3'-diaminobenzidine (DAB), diaminobenzidine with nickel enhancement, 3-amino-9-ethylcarbazole (AEC), Benzidine dihydrochloride (BDHC), Hanker-Yates reagent (HYR), Indophane blue (IB), tetramethylbenzidine (TMB), 4-chloro-1-naphtol (CN), .alpha.-naphtol pyronin (.alpha.-NP), o-dianisidine (OD), 5-bromo-4-chloro-3-indolylphosp- hate (BCIP), Nitro blue tetrazolium (NBT), 2-(p-iodophenyl)-3-p-nitropheny- 1-5-phenyl tetrazolium chloride (INT), tetranitro blue tetrazolium (TNBT), 5-bromo-4-chloro-3-indoxyl-beta-D-galactoside/ferro-ferricyanide (BCIG/FF).

Examples of commonly used substrates for Alkaline Phosphatase include Naphthol-AS-B 1-phosphate/fast red TR (NABP/FR), Naphthol-AS-MX-phosphate/fast red TR (NAMP/FR), Naphthol-AS-B 1-phosphate/- fast red TR (NABP/FR), Naphthol-AS-MX-phosphate/fast red TR (NAMP/FR), Naphthol-AS-B 1-phosphate/new fuschin (NABP/NF), bromochloroindolyl phosphate/nitroblue tetrazolium (BCIP/NBT), 5-Bromo-4-chloro-3-indolyl-b- d-galactopyranoside (BCIG).

Examples of luminescent labels include luminol, isoluminol, acridinium esters, 1,2-dioxetanes and pyridopyridazines. Examples of electrochemiluminescent labels include ruthenium derivatives. Examples of radioactive labels include radioactive isotopes of iodide, cobalt, selenium, tritium, carbon, sulfur and phosphorous.

Nanoparticles particles range from 1-1000 nm in size and include diverse chemical structures such as gold and silver particles and quantum dots. When irradiated with angled incident white light, silver or gold nanoparticles ranging from 40-120 nm will scatter monochromatic light with high intensity. The wavelength of the scattered light is dependent on the size of the particle. Four to five different particles in close proximity will each scatter monochromatic light, which when superimposed will give a specific, unique color. The particles are being manufactured by companies such as Genicon Sciences (Carlsbad, CA). Derivatized silver or gold particles can be attached to a broad array of molecules including, proteins, antibodies, small molecules, receptor ligands, and nucleic acids.

Further examples of nanoparticles include quantum dots. Quantum dots are fluorescing crystals 1-5 nm in diameter that are excitable by light over a large range of wavelengths. Upon excitation by light having an appropriate wavelength, these crystals emit light, such as monochromatic light, with a wavelength dependent on their chemical composition and size. Quantum dots such as CdSe, ZnSe, InP, or InAs possess unique optical properties; these and similar quantum dots are available from a number of commercial sources (*e.g*., NN-Labs, Fayetteville, AR; Ocean Nanotech, Fayetteville, AR; Nanoco Technologies, Manchester, UK; Sigma-Aldrich, St. Louis, MO).

Many dozens of classes of particles can be created according to the number of size classes of the quantum dot crystals. The size classes of the crystals are created either 1) by tight control of crystal formation parameters to create each desired size class of particle, or 2) by creation of batches of crystals under loosely controlled crystal formation parameters, followed by sorting according to desired size and/or emission wavelengths. Two examples of references in which quantum dots are embedded within intrinsic silicon epitaxial layers of semiconductor light emitting/detecting devices are United States Patent Nos. 5,293,050 and 5,354,707 to Chapple Sokol, et al.

Detectable labels may be linked to the antibodies described herein or to any other molecule that specifically binds to a biological marker of interest, *e.g*., an antibody, a nucleic acid probe, or a polymer. Furthermore, one of ordinary skill in the art would appreciate that detectable labels can also be conjugated to second, and/or third, and/or fourth, and/or fifth binding agents or antibodies, etc. Moreover, the skilled artisan would appreciate that each additional binding agent or antibody used to characterize a biological marker of interest may serve as a signal amplification step. In certain teachings, the biological marker may be detected visually using, *e.g.,* light microscopy, fluorescent microscopy, electron microscopy where the detectable substance is for example a dye, a colloidal gold particle, a luminescent reagent. Visually detectable substances bound to a biological marker may also be detected using a spectrophotometer. Where the detectable substance is a radioactive isotope, detection can be visually by autoradiography, or non-visually using a scintillation counter. See, *e.g.,* Larsson, 1988, Immunocytochemistry: Theory and Practice, (CRC Press, Boca Raton, Fla.); Methods in Molecular Biology, vol. 80 1998, John D. Pound (ed.) (Humana Press, Totowa, N.J.). In certain teachings, an anti-plazomicin antibody, or antigen binding fragment thereof, is bound to a detectable label that emits an altered or different signal (e.g., increased or reduced) when bound to an analyte, *e.g.* plazomicin.

In some teachings, an immunoassay includes plazomicin bound to a solid support, *e.g.* an ELISA plate. In such an teaching, any of the anti-plazomicin antibodies described herein can be contacted in solution with a sample comprising plazomicin. In this instance, the amount of anti-plazomicin antibody left unbound after contact with a sample comprising plazomicin is inversely proportional to the concentration of plazomicin in the sample. Upon application of this sample/anti-plazomicin antibody solution to the plazomicin-bound solid support, the unbound anti-plazomicin antibodies are free to bind to the plazomicin affixed to the support. In further teachings, the anti-plazomicin antibodies are labeled with a detectable label described herein. Measurements of the detectable label inform the amount of anti-plazomicin antibody bound to the plazomicin affixed to the solid support. In the above described "competitive" immunoassay, the level of detected anti-plazomicin is indirectly correlated with the amount of plazomicin present in the sample. In additional teachings, the anti-plazomicin antibodies are not labeled with a detectable label. In such teachings, a secondary antibody labeled with a detectable label can be used to measure the amount of anti-plazomicin antibodies bound to the solid support.

In additional teachings, any of the anti-plazomicin antibodies are bound to a solid support, *e.g.,* an ELISA plate. Application of samples comprising plazomicin to the solid support results in plazomicin binding to the affixed antibodies. The amount of plazomicin bound to the anti-plazomicin antibodies is then measured by application of an additional anti-plazomicin antibody described herein. The additional anti-plazomicin antibody can be labeled with a detectable label described herein. Measurement of the detectable label informs the amount of plazomicin bound to the anti-plazomicin antibodies. In the above described "direct" immunoassay, the level of detectable label measured is positively correlated with the amount of plazomicin bound to the additional anti-plazomicin antibody. In additional teachings, the anti-plazomicin antibodies are not labeled with a detectable label. In such teachings, a secondary antibody labeled with a detectable label can be used to measure the amount of anti-plazomicin antibodies bound to the solid support.

Certain teachings comprise determining a level of plazomicin in a sample, wherein the sample was taken from a subject to whom plazomicin was administered. As used herein, a "subject" includes any mammalian subject from which a sample can be acquired. In certain teachings, the mammal is a human. The methods of the present disclosure can also be employed for the treatment of non-human primates (e.g. monkeys, baboons, and chimpanzees) mice, rats, bovines, horses, cats, dogs, pigs, rabbits, goats, deer, sheep, ferrets, gerbils, guinea pigs, hamsters, bats, birds (e.g., chickens, turkeys, and ducks), fish and reptiles.

In some teachings, a method of determining a level or amount of plazomicin or a pharmaceutically acceptable salt thereof in a sample (such as a biological sample), comprises (i) contacting the sample with an isolated antibody or antigen binding fragment that specifically binds to plazomicin or the pharmaceutically acceptable salt thereof, and (ii) detecting the presence or absence of, or an amount of the antibody bound to the plazomicin or the pharmaceutically acceptable salt thereof, thereby determining the level of plazomicin or the pharmaceutically acceptable salt thereof in the sample. The method may include, for example, an immunological assay such as an ELISA, competitive ELISA, a turbidity assay, or a cloned enzyme donor immunoassay (CEDIA). The biological sample can be a liquid sample, such as a serum, a plasma, blood, urine, saliva, or sputum. In some teachings, the biological sample was obtained from a subject (e.g., a human patient) to whom plazomicin or a pharmaceutically acceptable salt thereof had been administered. The sample can be obtained from the subject after a period of time after the plazomicin or pharmaceutically acceptable salt thereof had been administered.

The determined level of plazomicin or pharmaceutically acceptable salt thereof in a biological sample obtained from a subject to whom the plazomicin or pharmaceutically acceptable salt thereof has been administered can be used to determine if a subsequent dose should be administered, or how large of a subsequent dose should be administered, to the subject. For example, in some teachings, the subject is selected for administering a subsequent dose of plazomicin or a pharmaceutically acceptable salt thereof, wherein if the plazomicin or a pharmaceutically acceptable salt thereof in the biological sample is below or equal to a predetermined cut-off value, the subject is selected to receive the subsequent dose; or if the plazomicin or a pharmaceutically acceptable salt thereof in the biological sample is above the predetermined cut-off value, the subject is selected to not receive the subsequent dose. In some teachings, the method comprises selecting a subsequent dose of plazomicin or a pharmaceutically acceptable salt thereof for administration to the subject, wherein if the plazomicin or a pharmaceutically acceptable salt thereof in the biological sample is below or equal to a predetermined cut-off value, the subject is selected to receive a subsequent dose equal to or greater than the prior dose; or if the plazomicin or a pharmaceutically acceptable salt thereof in the biological sample is above the predetermined cut-off value, the subject is selected to receive a subsequent dose that is lower than the prior dose. The cut-off value can be or can be correlated to a physiological level of plazomicin or pharmaceutically acceptable salt thereof in the serum of the subject. The clinical range for the concentration of plazomicin in a patient's sera is expected to be between 0.5 µg/mL and 40 µg/ml. Thus, in some teachings, the cut-off value is about 40 µg/mL or less (such as about 35 µg/mL or less, about 30 µg/mL or less, about 25 µg/mL or less, about 20 µg/mL or less, about 15 µg/mL or less, about 10 µg/mL or less, about 5 µg/mL or less, about 1 µg/mL or less, or about 0.5 µg/mL or less. In some teachings, the method comprises administering the subsequent dose to the subject.

In some teachings, the initial or subsequent dose of plazomicin or the pharmaceutically acceptable salt thereof is about 1 mg/kg or more (such as about 2 mg/kg or more, about 4 mg/kg or more, about 6 mg/kg or more, about 8 mg/kg or more, about 10 mg/kg or more, about 12 mg/kg or more, about 15 mg/kg or more, about 20 mg/kg or more, or about 25 mg/kg or more). In some teachings, the initial or subsequent dose of plazomicin or the pharmaceutically acceptable salt thereof is about 50 mg/kg or less (such as about 25 mg/kg or less, about 20 mg/kg or less, about 15 mg/kg or less, about 12 mg/kg or less, about 10 mg/kg or less, about 8 mg/kg or less, about 6 mg/kg or less, about 4 mg/kg or less, or about 2 mg/kg or less). In some teachings, the subsequent dose is about 1.25 times or more (such as about 1.5 times or more, about 1.75 times or more, about 2 times or more, about 2.5 times or more, about 3 times or more, about 3.5 times or more, about 4 times or more, about 4.5 times or more, or about 5 times or more) than the initial dose. In some teachings, the subsequent dose is between about 1 times to about 5 times (such as between about 1 times to about 4.5 times, about 1 times to about 4 times, about 1 times to about 3.5 times, about 1 times to about 3 times, about 1 times to about 2.5 times, about 1 times to about 2 times, about 1 times to about 1.75 times, about 1 times to about 1.5 times, or about 1 times to about 1.25 times) the initial dose. In some teachings, the subsequent dose is less than 1 times the initial dose (such as about 90% or less, about 80% or less, about 70% or less, about 60% or less, about 50% or less, about 40% or less, about 30% or less, about 20% or less, or about 10% or less times the initial dose).

Some teachings are directed to methods of treating bacterial infection in a subject in need thereof comprising (i) providing to the subject an effective amount of plazomicin, (ii) waiting for a first period of time, and (iii) determining the level of plazomicin in a biological sample collected from the subject through the use of an immunoassay. Further teachings comprise (iv) providing additional plazomicin to the subject if the level of plazomicin is below a defined cut-off value, or not providing additional plazomicin, or providing a reduced amount of plazomicin to the subject, if the level of plazomicin is above a defined cut-off value. The method may be repeated once or more, *e.g.*, over the course of treatment of a subject with plazomicin. Methods of the present invention may be used to monitor plazomicin levels in a subject over the course of treatment with plazomicin, allowing the amount of plazomicin provided to the subject to be adjusted to maintain a desired level of plazomicin in the subject. The clinical range for the concentration of plazomicin in a patient's sera is expected to be between 0.5 µg/mL and 40 µg/ml.

In some teachings a method of treating a bacterial infection in a subject comprises (i) administering a dose of plazomicin or a pharmaceutically acceptable salt thereof to the subject; and (ii) determining the level of plazomicin or a pharmaceutically acceptable salt thereof in a biological sample obtained from the subject, for example as discussed above. In some teachings, the level of plazomicin or the pharmaceutically acceptable salt thereof is determined after the dose is administered. In some teachings, the level of plazomicin or the pharmaceutically acceptable salt thereof is determined before the dose is administered. For example, the level of plazomicin or the pharmaceutically acceptable salt thereof can include (i) contacting the sample with an isolated antibody or antigen binding fragment that specifically binds to plazomicin or the pharmaceutically acceptable salt thereof, and (ii) detecting the presence or absence of, or an amount of the antibody bound to the plazomicin or the pharmaceutically acceptable salt thereof, thereby determining the level of plazomicin or the pharmaceutically acceptable salt thereof in the sample. The method may include, for example, an immunological assay such as an ELISA, competitive ELISA, a turbidity assay, or a cloned enzyme donor immunoassay (CEDIA). The biological sample can be a liquid sample, such as a serum, a plasma, blood, urine, saliva, or sputum. In some teachings, the method includes waiting a period of time between administering the dose and determining the level of plazomicin or the pharmaceutically acceptable salt therein the biological sample obtained from the subject.

The method of treating the subject can optionally include administering a subsequent dose of plazomicin or the pharmaceutically acceptable salt thereof to the subject if the determined level of plazomicin or the pharmaceutically acceptable salt thereof is below or equal to a predetermined cut-off value. In some teachings, the method includes administering a subsequent dose of plazomicin or the pharmaceutically acceptable salt thereof to the subject, wherein the subsequent dose is (a) greater than the prior dose if a determined level of plazomicin or the pharmaceutically acceptable salt thereof in a biological sample obtained from the subject is below or equal to a predetermined cut-off value; or (b) lower than the prior does if the determined level of plazomicin or the pharmaceutically acceptable salt thereof in the biological sample is above the predetermined cut-off value. In some teachings, the initial or subsequent dose of plazomicin or the pharmaceutically acceptable salt thereof is about 1 mg/kg or more (such as about 2 mg/kg or more, about 4 mg/kg or more, about 6 mg/kg or more, about 8 mg/kg or more, about 10 mg/kg or more, about 12 mg/kg or more, about 15 mg/kg or more, about 20 mg/kg or more, or about 25 mg/kg or more). In some teachings, the initial or subsequent dose of plazomicin or the pharmaceutically acceptable salt thereof is about 50 mg/kg or less (such as about 25 mg/kg or less, about 20 mg/kg or less, about 15 mg/kg or less, about 12 mg/kg or less, about 10 mg/kg or less, about 8 mg/kg or less, about 6 mg/kg or less, about 4 mg/kg or less, or about 2 mg/kg or less). In some teachings, the subsequent dose is about 1.25 times or more (such as about 1.5 times or more, about 1.75 times or more, about 2 times or more, about 2.5 times or more, about 3 times or more, about 3.5 times or more, about 4 times or more, about 4.5 times or more, or about 5 times or more) than the initial dose. In some teachings, the subsequent dose is between about 1 times to about 5 times (such as between about 1 times to about 4.5 times, about 1 times to about 4 times, about 1 times to about 3.5 times, about 1 times to about 3 times, about 1 times to about 2.5 times, about 1 times to about 2 times, about 1 times to about 1.75 times, about 1 times to about 1.5 times, or about 1 times to about 1.25 times) the initial dose. In some teachings, the subsequent dose is less than 1 times the initial dose (such as about 90% or less, about 80% or less, about 70% or less, about 60% or less, about 50% or less, about 40% or less, about 30% or less, about 20% or less, or about 10% or less times the initial dose).

As used herein, an "effective amount" refers to an amount of plazomicin required to achieve a particular biological result. Such results may include, but are not limited to, reduction of bacteria load. Bacterial load may be measured by techniques known in the art (e.g., colony forming units (CFU)/unit of sample). A reduction in bacterial load may be defined as at least about 30%, 40%, 50%, 60%, 70%, 80%, 90%, 95%, or 99% reduction in bacterial load after plazomicin treatment, compared to the bacterial load present prior to treatment initiation. The effective amount will vary based on the metabolic stability and length of action of the compound, (*e.g*., plazomicin), and on the age, body weight, general health, sex, and diet of the subject, as well as the severity and nature of the infection.

A "period of time" refers to the amount of time allowed to elapse after an initial or previous treatment with plazomicin (*e.g*., "a first period of time"). The first period of time may be at least 24 hours, at least 48 hours, at least 72 hours, at least 4 days, at least 5 days, at least 6 days, at least 7 days, at least 14 days, or at least 21 days.

"Providing additional plazomicin" may comprise providing additional plazomicin equal to an initial dose of plazomicin. As used herein, "dose" refers to the amount of administered drug, *e.g.*, the amount of administered plazomicin. In further teachings, an additional dose of plazomicin that is equal to an initial dose of plazomicin is administered at least once after initial plazomicin administration. In further teachings, an additional dose of plazomicin equal to an initial dose of plazomicin is administered at least 2, 3, 4, 5, 6, 7, 8, 9, 10, 15 times or more after initial plazomicin administration. In some teachings, "providing additional plazomicin" may comprise providing an additional dose of plazomicin greater than an initial dose of plazomicin. In further teachings, an additional dose of plazomicin that is greater than an initial dose of plazomicin is administered at least once after initial plazomicin administration. In further teachings, an additional dose of plazomicin greater than an initial dose of plazomicin is administered at least 2, 3, 4, 5, 6, 7, 8, 9, 10, 15, or more time after initial plazomicin administration. In any of the above described teachings, the presence or absence of, or an amount of plazomicin can be detected before or after each, some, or all administrations of additional plazomicin.

As used herein, "not providing additional plazomicin," may comprise cessation of plazomicin administration. In some teachings, "not providing additional plazomicin" may comprise providing a dose of plazomicin less than an initial or previous dose of plazomicin. In further teachings, a dose of plazomicin less than an initial dose of plazomicin is administered at least once. In further teachings, a dose of plazomicin less than an initial dose of plazomicin is administered at least 2, 3, 4, 5, 6, 7, 8, 9, 10, 15 or more times after initial plazomicin administration. In any of the above described teachings, the presence or absence of, or an amount of plazomicin can be detected before or after each, some, or all administrations of plazomicin.

As used herein, a "cut-off value" refers to a value used to determine providing of additional plazomicin or not providing additional plazomicin. The cut-off value may be binary in nature, e.g. "one" and "zero", wherein "one" indicates the presence of plazomicin in a biological sample and wherein "zero" indicates the absence (or an amount below the limit of detection) of plazomicin in a biological sample. The cut-off value may be a concentration, e.g., µg/L. In some teachings, additional plazomicin is administered to the subject for a second period of time if the determined level of plazomicin is below or equal to a cut-off value. In some teachings, additional plazomicin is not administered to the subject for a second period of time if the determined level of plazomicin is above a cut-off value. A suitable range for plazomicin concentration in human plasma is 0.5-40 µg/ml. For example, in some teachings a cut-off value is 0.5 µg/mL of plazomicin in human plasma, below which additional plazomicin is administered. In some teachings, a cut-off value is 40 µg/mL of plazomicin in human plasma, above which additional plazomicin is not administered. The suitable range for plazomicin concentration in human plasma may be narrowed based on factors including the age, body weight, general health, sex, and diet of the subject, as well as the severity and nature of the infection.

Certain methods described herein are directed to anti-plazomicin antibodies, and antigen-binding fragments thereof, for use in the treatment of bacterial infections. In specific teachings, the bacteria are Gram-negative bacteria. Gram-negative bacteria are known in the art and refer to bacteria that do not retain the crystal violet stain used in the Gram staining method of bacterial differentiation. Gram-negative bacteria are further characterized by an outer membrane containing lipopolysaccharide (LPS). Non-limiting examples of Gram-negative bacteria include *Pseudomonas aeruginosa*, *Stenotrophomonas maltophila*, *Burkholderia cepacia, Alcaligenes xylosoxidans, Haemophilus, Franciscellaceae (e.g., Franciscella tularensis*) *Neisseria species*, *Enterobacteriaceae,* such as *Serratia*, *Proteus*, *Klebsiella, Enterobacter, Citrobacter, Salmonella, Providencia, Yersinia (e.g., Yersinia pestis*), *Morganella*, *Cedecea*, *Edwardsiella species,* and *Escherichia coli (E. coli).* Further teachings are directed to anti-plazomicin antibodies, and antigen-binding fragments thereof, for use in the treatment of "multi-drug resistant" or "MDR" bacteria, particularly Gram-negative MDR bacteria. MDR bacteria refer to bacteria that have acquired resistance to at least one agent (*e.g*., an antibiotic) in three or more antimicrobial categories. Multi-drug resistance occurs in bacteria by the accumulation, on resistance plasmids or transposons, of genes, with each encoding for resistance to a specific agent, and/or by the action of multidrug efflux pumps, each of which can pump out more than one drug type. Non-limiting examples of clinically relevant gram-negative MDR bacteria include carbapenem-resistant *Enterobacteriaceae* (CRE), carbapenemase-producing *Klebsiella pneumoniae*, *MDR E. coli*, *Pseudomonas aeruginosa*, *Acinetobacter* species including *Acinetobacter baumannii*, *Y, pestis*, *F, Tularensis*, and *P. aeruginosa.*

Further teachings are contemplated for the use of anti-plazomicin antibodies, and antigen-binding fragments thereof, in the treatment of "extreme drug resistant" or "XDR" bacteria, particularly Gram-negative XDR bacteria. XDR bacteria refer to bacteria that have acquired resistance to at least one agent in all but one or two antimicrobial categories (XDR bacterial isolates remain susceptible to only one or two categories of antibacterial agents). Further contemplated teachings are directed to anti-plazomicin antibodies, and antigen-binding fragments thereof, for use in the treatment of "pan drug resistant" or "PDR" bacteria, particularly Gram-negative PDR bacteria. PDR bacteria refer to bacteria that have acquired resistance to all agents in all antimicrobial categories.

Some teachings are directed to the use of anti-plazomicin antibodies, and antigen-binding fragments thereof, for use in the treatment of a urinary tract infection (UTI). In some teachings, said UTI is an infection of the lower urinary tract (*e.g*., a bladder infection or cystitis). In some teachings, said UTI is an infection of the upper urinary tract (*e.g*., a kidney infection, or pyelonephritis). In certain teachings, said UTI is an infection with gram-negative bacteria. In certain teachings, said UTI is an infection with MDR gram-negative bacteria. Some teachings are directed to the use of anti-plazomicin antibodies, and antigen-binding fragments thereof, for use in the treatment of bacterial septicemia, serious infections of the respiratory tract, bones, joints, skin, soft tissue, and central nervous system (*e*.*g*. meningitis), intra-abdominal infections (peritonitis), infections arising around or on the site of a burn, postoperative infections (including post-vascular surgery).

### EXAMPLES

The following examples are included for illustrative purposes only and are not intended to limit the scope of the invention.

### EXAMPLE 1

### GENERATION OF PLAZOMICIN-SPECIFIC ANTIBODIES

Wild type Balb/c mice were immunized via the hock route with 10 µg/mouse plazomicin conjugated to the hapten, keyhole limpet hemocyanin (KLH, plazomicin-KLH) in PBS. After initial immunization on day 0, mice were boosted three times on days 14, 21, and 28. On day 32, mice were sacrificed and the popliteal, axillary, and inguinal lymph nodes were harvested.

### Antibody Generation

After harvest, the lymph node cells were fused with SP2/O-Ag14 hybridoma cell lines (purchased from ATCC). Hybridomas were selected in hypoxanthine-aminopterin-thymidine (HAT) medium cultured for 10 days. After 10 days, hybridoma supernatants were collected and tested for antibody binding to BSA-plazomicin by ELISA. Positive hybridoma cultures were then subcloned and antibodies were purified from hybridoma-containing supernatants using G-sepharose.

Sequences for the antibody variable regions (light and heavy chains) obtained from the hybridomas were determined, as shown in Table 4.

**Table 4: Sequences of Hybridoma Antibody Variable Regions**

| **Hybridoma** | **Chain** | **SEQ ID NO** |
|---|---|---|
| MC329 | Light | 26, 30 |
| | Heavy | 22 |
| MC330 | Light | 38 |
| | Heavy | 24 |
| MC331 | Light | 46 |
| | Heavy | 42 |
| MC332 | Light | 54 |
| | Heavy | 50 |
| MC334 | Light | 62 |
| | Heavy | 58 |
| MC335 | Light | 70 |
| | Heavy | 66 |
| MC336 | Light | 78 |
| | Heavy | 74 |
| MC337 | Light | 86 |
| | Heavy | 82 |
| MC339 | Light | 94 |
| | Heavy | 90 |
| MC340 | Light | 102 |
| | Heavy | 98 |

### Functional Screening of Hybridomas

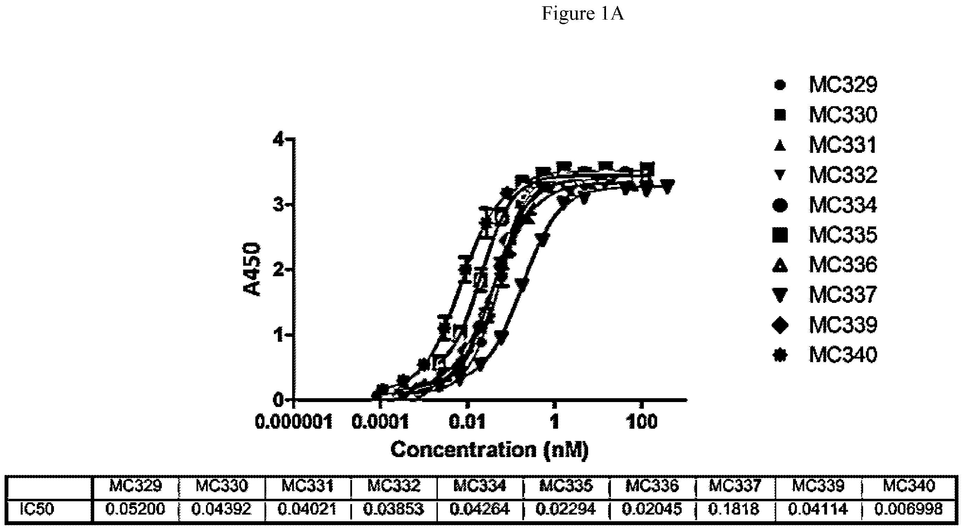

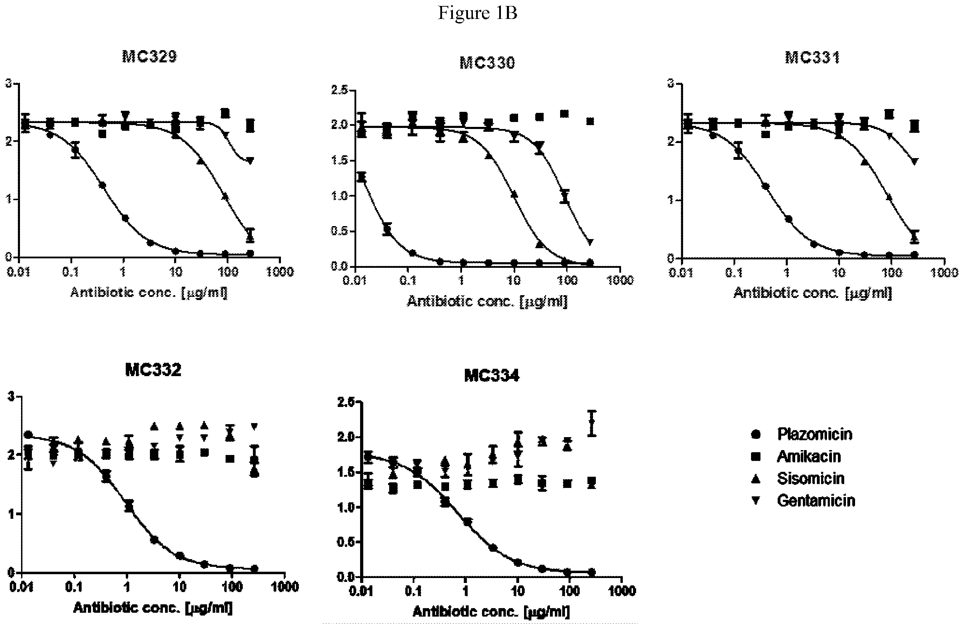

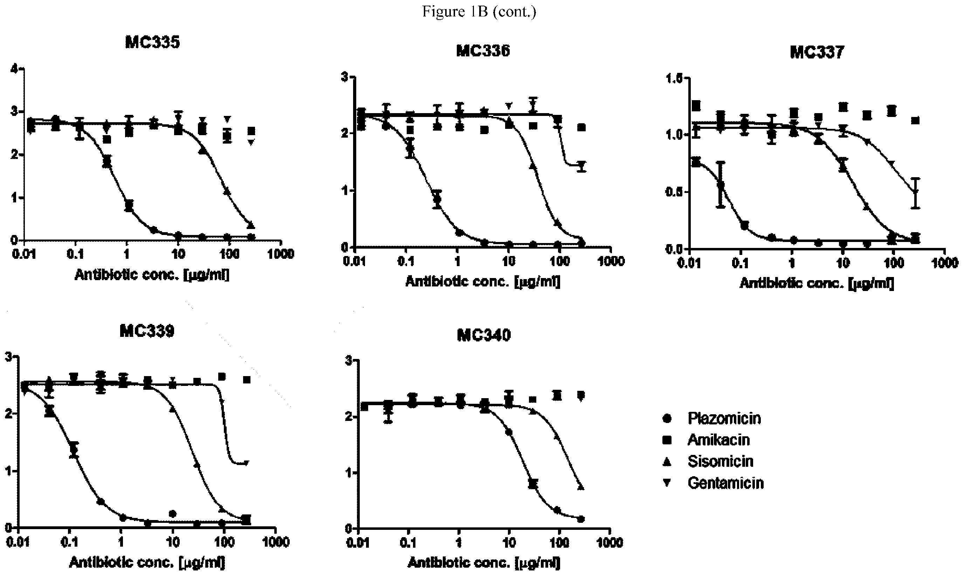

The purified antibodies were then analyzed for binding to BSA-plazomicin by ELISA (the tested concentrations of antibodies ranged from 0.0001 nM to 100 nM). (FIG. 1A) and competitive ELISA (FIG. 1B) on BSA-bromo-plazomicin coated ELISA plates. For competitive ELISAs, free aminoglycosides (plazomicin, gentamicin, amikacin, and sisomicin) at concentrations; 270 µg/ml, 90 µg/ml 30 µg/ml, 10 µg/ml, 3.3 µg/ml, 1.1 µg/ml, 0.4 µg/ml, 0.12 µg/ml, 0.04 µg/ml and 0.013 µg/ml were incubated with one of 10 purified monoclonal antibodies derived from the cell cultures described above; MC329, MC330, MC331, MC332, MC334, MC335, MC335, MC337, MC339, MC340 at 0.05 nM concentration (FIG. 1B). The antibody/aminoglycoside solutions were then added to BSA-bromo-plazomicin-coated ELISA plates. Plates were washed to remove unbound antibody, and IC50 values (µg/mL) were determined for each aminoglycoside and are shown below in Table 5.

**Table 5: Antibiotic IC50 Values for Aminoglycosides**

| **Antibiotic IC50 Values for Amino glycosides** | | | | |
|---|---|---|---|---|
| Antibody | Plazomicin | Amikacin | Sisomicin | Gentamicin |
| MC329 | 0.43 | nc | 83.0 | 107.0 |
| MC330 | 0.02 | nc | 9.9 | 92.0 |
| MC331 | 0.43 | nc | 83.0 | 214.4 |
| MC332 | 0.94 | nc | nc | nc |
| MC334 | 0.72 | nc | nc | nc |
| MC335 | 0.59 | nc | 69.0 | nc |
| MC336 | 0.25 | nc | 37.6 | ∼ 106.1 |
| MC337 | 0.06 | nc | 16.1 | 107.6 |
| MC339 | 0.12 | nc | 24.4 | ∼ 101.7 |
| MC340 | 19.0 | nc | 153 | nc |

| | | | | |
|---|---|---|---|---|
| nc = Not Calculated | | | | |

## Claims

1. An isolated antibody, or an antigen-binding fragment thereof, comprising:
(a) a light chain variable region comprising VL-CDR1, a VL-CDR2 and VL-CDR3 set forth in SEQ ID NO: 196, SEQ ID NO: 199, and SEQ ID NO: 29, respectively; and a heavy chain variable region comprising VH-CDR1, VH-CDR2 and VH-CDR3 set forth in SEQ ID NO: 106, SEQ ID NO: 109, and SEQ ID NO: 112, respectively;
(b) a light chain variable region comprising VL-CDR1, a VL-CDR2 and VL-CDR3 set forth in SEQ ID NO: 203, SEQ ID NO: 206, and SEQ ID NO: 208, respectively; and a heavy chain variable region comprising VH-CDR1, VH-CDR2 and VH-CDR3 set forth in SEQ ID NO: 106, SEQ ID NO: 109, and SEQ ID NO: 112, respectively;
(c) a light chain variable region comprising VL-CDR1, a VL-CDR2 and VL-CDR3 set forth in SEQ ID NO: 211, SEQ ID NO: 214, and SEQ ID NO: 41, respectively; and a heavy chain variable region comprising VH-CDR1, VH-CDR2 and VH-CDR3 set forth in SEQ ID NO: 115, SEQ ID NO: 118, and SEQ ID NO: 121, respectively;
(d) a light chain variable region comprising VL-CDR1, a VL-CDR2 and VL-CDR3 set forth in SEQ ID NO: 218, SEQ ID NO: 221, and SEQ ID NO: 223, respectively; and a heavy chain variable region comprising VH-CDR1, VH-CDR2 and VH-CDR3 set forth in SEQ ID NO: 124, SEQ ID NO: 127, and SEQ ID NO: 130, respectively;
(e) a light chain variable region comprising VL-CDR1, a VL-CDR2 and VL-CDR3 set forth in SEQ ID NO: 226, SEQ ID NO: 229, and SEQ ID NO: 57, respectively; and a heavy chain variable region comprising VH-CDR1, VH-CDR2 and VH-CDR3 set forth in SEQ ID NO: 133, SEQ ID NO: 136, and SEQ ID NO: 139, respectively;
(f) a light chain variable region comprising VL-CDR1, a VL-CDR2 and VL-CDR3 set forth in SEQ ID NO: 240, SEQ ID NO: 243, and SEQ ID NO: 73, respectively; and a heavy chain variable region comprising VH-CDR1, VH-CDR2 and VH-CDR3 set forth in SEQ ID NO: 151, SEQ ID NO: 154, and SEQ ID NO: 157, respectively;
(g) a light chain variable region comprising VL-CDR1, a VL-CDR2 and VL-CDR3 set forth in SEQ ID NO: 247, SEQ ID NO: 250, and SEQ ID NO: 81, respectively; and a heavy chain variable region comprising VH-CDR1, VH-CDR2 and VH-CDR3 set forth in SEQ ID NO: 160, SEQ ID NO: 163, and SEQ ID NO: 166, respectively;
(h) a light chain variable region comprising VL-CDR1, a VL-CDR2 and VL-CDR3 set forth in SEQ ID NO: 254, SEQ ID NO: 257, and SEQ ID NO: 89, respectively; and a heavy chain variable region comprising VH-CDR1, VH-CDR2 and VH-CDR3 set forth in SEQ ID NO: 169, SEQ ID NO: 172, and SEQ ID NO: 175, respectively;
(i) a light chain variable region comprising VL-CDR1, a VL-CDR2 and VL-CDR3 set forth in SEQ ID NO: 261, SEQ ID NO: 264, and SEQ ID NO: 97, respectively; and a heavy chain variable region comprising VH-CDR1, VH-CDR2 and VH-CDR3 set forth in SEQ ID NO: 178, SEQ ID NO: 181, and SEQ ID NO: 184, respectively; or
(j) a light chain variable region comprising VL-CDR1, a VL-CDR2 and VL-CDR3 set forth in SEQ ID NO: 268, SEQ ID NO: 271, and SEQ ID NO: 105, respectively; and a heavy chain variable region comprising VH-CDR1, VH-CDR2 and VH-CDR3 set forth in SEQ ID NO: 187, SEQ ID NO: 190, and SEQ ID NO: 193, respectively;
wherein the antibody or antigen-binding fragment thereof specifically binds plazomicin or a pharmaceutically acceptable salt thereof, optionally wherein the antibody or antigen-binding fragment thereof specifically binds plazomicin.

2. The isolated antibody or antigen-binding fragment thereof according to claim 1, comprising:
(a) a variable light chain region that is at least 85% identical to SEQ ID NO: 26, and a variable heavy chain region that is at least 85% identical to SEQ ID NO: 22;
(b) a variable light chain region that is at least 85% identical to SEQ ID NO: 30, and a variable heavy chain region that is at least 85% identical to SEQ ID NO: 22;
(c) a variable light chain region that is at least 85% identical to SEQ ID NO: 38, and a variable heavy chain region that is at least 85% identical to SEQ ID NO: 34;
(d) a variable light chain region that is at least 85% identical to SEQ ID NO: 46, and a variable heavy chain region that is at least 85% identical to SEQ ID NO: 42;
(e) a variable light chain region that is at least 85% identical to SEQ ID NO: 54, and a variable heavy chain region that is at least 85% identical to SEQ ID NO: 50;
(f) a variable light chain region that is at least 85% identical to SEQ ID NO: 62, and a variable heavy chain region that is at least 85% identical to SEQ ID NO: 58;
(g) a variable light chain region that is at least 85% identical to SEQ ID NO: 70, and a variable heavy chain region that is at least 85% identical to SEQ ID NO: 66;
(h) a variable light chain region that is at least 85% identical to SEQ ID NO: 78, and a variable heavy chain region that is at least 85% identical to SEQ ID NO: 74;
(i) a variable light chain region that is at least 85% identical to SEQ ID NO: 86, and a variable heavy chain region that is at least 85% identical to SEQ ID NO: 82;
(j) a variable light chain region that is at least 85% identical to SEQ ID NO: 94, and a variable heavy chain region that is at least 85% identical to SEQ ID NO: 90; or
(k) a variable light chain region that is at least 85% identical to SEQ ID NO: 102, and a variable heavy chain region that is at least 85% identical to SEQ ID NO: 98.

3. The isolated antibody or antigen-binding fragment thereof according to any claim 1 or 2, comprising:
(a) a variable light chain region according to SEQ ID NO: 26, and a variable heavy chain region according to SEQ ID NO: 22;
(b) a variable light chain region according to SEQ ID NO: 30, and a variable heavy chain region according to SEQ ID NO: 22;
(c) a variable light chain region according to SEQ ID NO: 38, and a variable heavy chain region according 1 to SEQ ID NO: 34;
(d) a variable light chain region according to SEQ ID NO: 46, and a variable heavy chain region according to SEQ ID NO: 42;
(e) a variable light chain region according to SEQ ID NO: 54, and a variable heavy chain region according to SEQ ID NO: 50;
(f) a variable light chain region according to SEQ ID NO: 62, and a variable heavy chain region according to SEQ ID NO: 58;
(g) a variable light chain region according to SEQ ID NO: 70, and a variable heavy chain region according to SEQ ID NO: 66;
(h) a variable light chain region according to SEQ ID NO: 78, and a variable heavy chain region according to SEQ ID NO: 74;
(i) a variable light chain region according to SEQ ID NO: 86, and a variable heavy chain region according to SEQ ID NO: 82;
(j) a variable light chain region according to SEQ ID NO: 94, and a variable heavy chain region according to SEQ ID NO: 90; or
(k) a variable light chain region according to SEQ ID NO: 102, and a variable heavy chain region according to SEQ ID NO: 98.

4. The antibody or antigen-binding fragment thereof according to any of claims 1-3 that is: (a) a whole antibody; or (b) an antigen-binding fragment selected from the group consisting of a univalent antibody generated from an IgG4 antibody with the hinge region removed , a single chain variable fragment (scFv), a Fab fragment and a F(ab')₂ fragment.

5. The isolated antibody or antigen binding fragment thereof according to any one of claims 1-4, wherein:
(a) the antibody or antigen binding fragment thereof specifically binds to plazomicin or a pharmaceutically acceptable salt thereof with an IC50 of about 100 µg/mL or less or an IC50 of 19 µg/mL or less, optionally wherein the antibody or antigen binding fragment thereof specifically binds to plazomicin or a pharmaceutically acceptable salt thereof with an IC50 of about 0.02 µg/mL or more, optionally wherein the IC50 is determined by a competitive ELISA assay; and/or
(b) the antibody or antigen binding fragment thereof binds to plazomicin or the pharmaceutically acceptable salt thereof with an equilibrium dissociation constant of about 10⁻⁷ M or less.

6. The isolated antibody or antigen binding fragment thereof according to any one of claims 1-5, wherein the antibody or antigen binding fragment thereof binds to plazomicin or the pharmaceutically acceptable salt thereof with a binding affinity greater than the binding affinity of the antibody or antigen binding fragment thereof to a non-plazomicin aminoglycoside, optionally wherein the non-plazomicin aminoglycoside is amikacin, sisomicin, or gentamicin.

7. The antibody, or antigen-binding fragment thereof, of any one of claims 1-6, wherein the antibody or antigen-binding fragment further comprises a detectable marker.

8. A nucleic acid encoding:
the antibody or antigen-binding fragment of any one of claims 1-7 .

9. A vector comprising the nucleic acid of claim 8, optionally wherein the vector is an expression vector.

10. A host cell comprising the nucleic acid of claim 8 or the vector of claim 9.

11. A method of producing an antibody comprising culturing the host cell of claim 10 under a condition that produces the antibody, optionally wherein the method comprises recovering the antibody produced by the host cell.

12. A composition comprising the antibody or antigen-binding fragment of any of claims 1-7; optionally wherein the composition further comprises a pharmaceutically acceptable carrier.

13. A solid support, comprising immobilized thereto an antibody or antigen-binding fragment of any of claims 1-7; optionally wherein the solid support is a bead, a column, an array, an assay plate, a microwell, a stick, a filter, or a strip.

14. An in vitro method of determining a level of plazomicin or a pharmaceutically acceptable salt thereof in a biological sample, comprising:
(i) contacting the sample with the isolated antibody, or the antigen-binding fragment thereof, of any one of claims 1-7; and
(ii) detecting the presence or absence of, or an amount of, the antibody bound to the plazomicin or the pharmaceutically acceptable salt thereof, thereby determining the level of plazomicin or the pharmaceutically acceptable salt thereof in the biological sample; optionally wherein:
(a) the biological sample is a serum, a plasma, blood, urine, saliva, or sputum; and/or
(b) the detecting is performed using an immunoassay; further optionally wherein the immunoassay is selected from the group consisting of a cloned enzyme donor immunoassay (CEDIA), a turbidity assay, and a competitive ELISA

15. The method of claim 14, wherein the biological sample was obtained from a subject to whom plazomicin or a pharmaceutically acceptable salt thereof had been administered; optionally wherein the subject is human.

16. A composition comprising an antibody or antigen binding fragment thereof according to any one of claims 1-7 for in vitro use in detecting the presence, level, or amount of plazomicin or a pharmaceutically acceptable salt thereof in a sample; optionally wherein the sample is a biological sample.

## Patentansprüche

1. Verbindung mit Formel I: 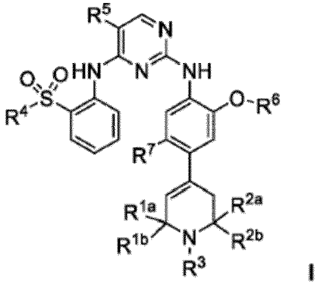 oder ein pharmazeutisch verträgliches Salz oder Solvat davon, wobei:
R^{1a} und R^{1b} unabhängig ausgewählt sind aus der Gruppe, die besteht aus Wasserstoff, C₁₋₆-Alkyl und C₃₋₈-Cycloalkyl;
R^{2a} und R^{2b} unabhängig ausgewählt sind aus der Gruppe, die besteht aus Wasserstoff, C₁₋₆-Alkyl und C₃₋₈-Cycloalkyl;
R³ ausgewählt ist aus der Gruppe, die besteht aus C₃₋₆-Cycloalkyl und C₄₋₈-Heterocyclo,
R⁴ ausgewählt ist aus der Gruppe, die besteht aus C₁₋₄-Alkyl und C₃₋₆-Cycloalkyl;
R⁵ Halogen ist;
R⁶ ausgewählt ist aus der Gruppe, die besteht aus C₁₋₄-Alkyl und C₃₋₆-Cycloalkyl; und
R⁷ ausgewählt ist aus der Gruppe, die besteht aus Wasserstoff, C₁₋₄-Alkyl und C₃₋₆-Cycloalkyl.

2. Verbindung nach Anspruch 1, mit Formel II: 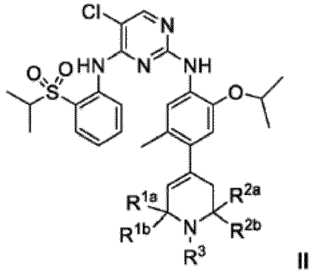 oder ein pharmazeutisch verträgliches Salz oder Solvat davon, wobei:
R^{1a} und R^{1b} unabhängig ausgewählt sind aus der Gruppe, die besteht aus Wasserstoff, C₁₋₄-Alkyl und C₃₋₆-Cycloalkyl;
R^{2a} und R^{2b} unabhängig ausgewählt sind aus der Gruppe, die besteht aus Wasserstoff, C₁₋₄-Alkyl und C₃₋₆-Cycloalkyl; und
R³ ausgewählt ist aus der Gruppe, die besteht aus C₃₋₆-Cycloalkyl und C₄₋₈-Heterocyclo.

3. Verbindung nach Anspruch 1 oder 2, oder ein pharmazeutisch verträgliches Salz oder Solvat davon, wobei R^{1b} und R^{2b} jeweils Wasserstoff sind.

4. Verbindung nach Anspruch 3, oder ein pharmazeutisch verträgliches Salz oder Solvat davon, wobei R^{1a} und R^{2a} jeweils Wasserstoff sind.

5. Verbindung nach Anspruch 2, oder ein pharmazeutisch verträgliches Salz oder Solvat davon, mit Formel III: 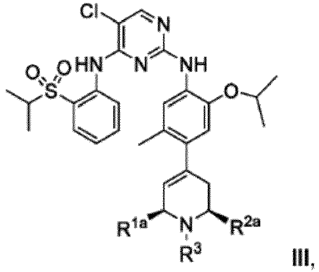 wobei:
R^{1a} und R^{2a} jeweils unabhängig ausgewählt sind aus der Gruppe, die besteht aus C₁₋₄-Alkyl und C₃₋₆-Cycloalkyl; und
die Verbindung einen Enantiomerenüberschuss von 90% oder mehr aufweist.

6. Verbindung nach Anspruch 3, oder ein pharmazeutisch verträgliches Salz oder Solvat davon, mit Formel IV: 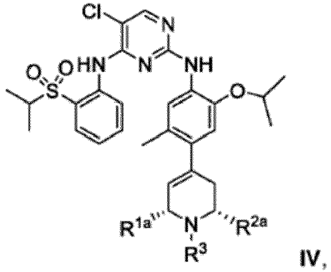 wobei:
R^{1a} und R^{2a} jeweils unabhängig ausgewählt sind aus der Gruppe, die besteht aus C₁₋₄-Alkyl und C₃₋₆-Cycloalkyl; und
die Verbindung einen Enantiomerenüberschuss von 90% oder mehr aufweist.

7. Verbindung nach Anspruch 1, oder ein pharmazeutisch verträgliches Salz oder Solvat davon, mit Formel V: 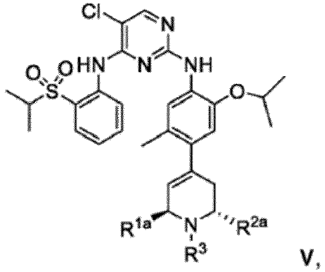 wobei:
R^{1a} und R^{2a} jeweils unabhängig ausgewählt sind aus der Gruppe, die besteht aus C₁₋₄-Alkyl und C₃₋₆-Cycloalkyl; und
die Verbindung einen Enantiomerenüberschuss von 90% oder mehr aufweist.

8. Verbindung nach Anspruch 1, oder ein pharmazeutisch verträgliches Salz oder Solvat davon, mit Formel VI: 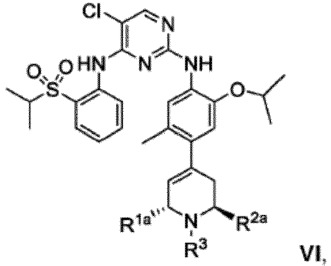 wobei:
R^{1a} und R^{2a} jeweils unabhängig ausgewählt sind aus der Gruppe, die besteht aus C₁₋₄-Alkyl und C₃₋₆-Cycloalkyl; und
die Verbindung einen Enantiomerenüberschuss von 90% oder mehr aufweist.

9. Verbindung nach Anspruch 8, oder ein pharmazeutisch verträgliches Salz oder Solvat davon, wobei R^{1a} und R^{2a} jeweils Methyl sind.

10. Verbindung nach Anspruch 2, oder ein pharmazeutisch verträgliches Salz oder Solvat davon, wobei R^{1a} und R^{2a} jeweils Cyclopropyl sind.

11. Verbindung nach Anspruch 2, oder ein pharmazeutisch verträgliches Salz oder Solvat davon, wobei R³ aus der Gruppe ausgewählt ist, die besteht aus:

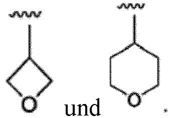

12. Verbindung nach Anspruch 1, oder ein pharmazeutisch verträgliches Salz oder Hydrat davon, ausgewählt aus der Gruppe, die besteht aus:
5-Chlor-*N*²-(2-isopropoxy-5-methyl-4-(1-(tetrahydro-2H-pyran-4-yl)-1,2,3,6-tetrahydropyridin-4-yl)phenyl)-*N*⁴-(2-(isopropylsulfonyl)phenyl)pyrimidin-2,4-diamin;
5-Chlor-*N²*-(2-isopropoxy-5-methyl-4-(1-(oxetan-3-yl)-1,2,3,6-tetrahydropyridin-4-yl)phenyl)-*N⁴-*(2-(isopropylsulfonyl)phenyl)pyrimidin-2,4-diamin,
5-Chlor-*N²*-(4-((cis)-2,6-dimethyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((cis)-2,6-dimethyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((trans)-2,6-diethyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2S,6S)-2,6-diethyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((trans)-2,6-dimethyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((trans)-2,6-dimethyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5 -Chlor-*N²*-(4-((cis)-2,6-dicyclopropyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((cis)-2,6-dicyclopropyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((trans)-2,6-dicyclobutyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((trans)-2,6-dicyclobutyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((trans)-2,6-dicyclopropyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((trans)-2,6-dicyclopropyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2R,6S)-2,6-dimethyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2R,6S)-2,6-dimethyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2S,6S)-2,6-dimethyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2S,6S)-2,6-dimethyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2R,6R)-2,6-dimethyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2R,6R)-2,6-dimethyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2S,6S)-2,6-dicyclopropyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2S,6S)-2,6-dicyclopropyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2R,6S)-2,6-dicyclopropyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2R,6S)-2,6-dicyclopropyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin;
5-Chlor-*N²*-(4-((2S,6R)-2,6-dicyclopropyl-1-(tetrahydro-2H-pyran-4-yl)-1,2,3,6-tetrahydropyridin-4-yl)-2-isopropoxy-5-methylphenyl)-*N⁴*-(2-(isopropylsulfonyl)phenyl)pyrimidin-2,4-diamin; und
5-Chlor-*N²*-(4-((2S,6R)-2,6-dicyclopropyl-1-(oxetan-3-yl)-1,2,3,6-tetrahydropyridin-4-yl)-2-isopropoxy-5-methylphenyl)-*N⁴*-(2-(isopropylsulfonyl)phenyl)pyrimidin-2,4-diamin.

13. Verbindung nach Anspruch 12, oder ein pharmazeutisch verträgliches Salz oder Hydrat davon, die 5-Chlor-*N²*-(2-isopropoxy-5-methyl-4-(1-(tetrahydro-2H-pyran-4-yl)-1,2,3,6-tetrahydropyridin-4-yl)phenyl)-*N⁴*-(2-(isopropylsulfonyl)phenyl)pyrimidin-2,4-diamin ist.

14. Pharmazeutische Zusammensetzung, umfassend die Verbindung nach einem der Ansprüche 1-13, oder ein pharmazeutisch verträgliches Salz oder Hydrat davon, und einen pharmazeutisch verträglichen Träger.

15. Verbindung nach einem der Ansprüche 1-13, oder ein pharmazeutisch verträgliches Salz, Hydrat oder Solvat davon, oder pharmazeutische Zusammensetzung nach Anspruch 14, zur Verwendung in der Behandlung von Krebs.

16. Verbindung zur Verwendung nach Anspruch 15, wobei der Krebs ausgewählt ist aus der Gruppe, die besteht aus anaplastisch-großzelligem Lymphom, nicht kleinzelligem Lungenkrebs, diffus großzelligem B-Zell-Lymphom, entzündlichen myofibroblastischen Tumoren, Neuroblastom, anaplastischem Schilddrüsenkrebs, Rhabdomyosarkom, Brustkrebs, Kolorektalkrebs, Ösophagus-Plattenepithelkrebs und Nierenzellkarzinom.

## Revendications

1. Composé présentant la Formule I : 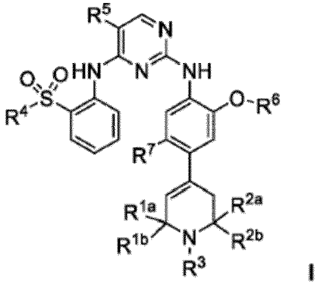 ou un sel ou solvate pharmaceutiquement acceptable de celui-ci, dans lequel :
R^{1a} et R^{1b} sont indépendamment choisis dans le groupe constitué d'un hydrogène, d'un alkyle en C₁₋₆ et d'un cycloalkyle en C₃₋₈;
R^{2a} et R^{2b} sont indépendamment choisis dans le groupe constitué d'un hydrogène, d'un alkyle en C₁₋₆ et d'un cycloalkyle en C₃₋₈ ;
R³ est choisi dans le groupe constitué d'un cycloalkyle en C₃₋₆ et d'un hétérocylco en C₄₋₈ ;
R⁴ est choisi dans le groupe constitué d'un alkyle en C₁₋₄ et d'un cycloalkyle en C₃₋₆ ;
R⁵ est un halo ;
R⁶ est choisi dans le groupe constitué d'un alkyle en C₁₋₄ et d'un cycloalkyle en C₃₋₆ ; et
R⁷ est choisi dans le groupe constitué d'un hydrogène, d'un alkyle en C₁₋₄ et d'un cycloalkyle en C₃₋₆.

2. Composé selon la revendication 1 présentant la Formule II : 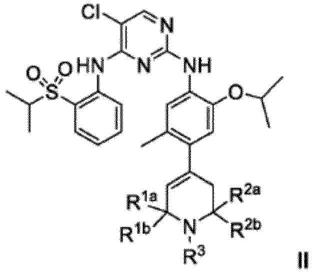 ou un sel ou solvate pharmaceutiquement acceptable de celui-ci, dans lequel :
R^{1a} et R^{1b} sont indépendamment choisis dans le groupe constitué d'un hydrogène, d'un alkyle en C₁₋₄ et d'un cycloalkyle en C₃₋₆ ;
R^{2a} et R^{2b} sont indépendamment choisis dans le groupe constitué d'un hydrogène, d'un alkyle en C₁₋₄ et d'un cycloalkyle en C₃₋₆ ; et
R³ est choisi dans le groupe constitué d'un cycloalkyle en C₃₋₆ et d'un hétérocyclo en C₄₋₈.

3. Composé selon les revendications 1 ou 2, ou un sel ou solvate pharmaceutiquement acceptable de celui-ci, dans lequel R^{1b} et R^{2b} sont chacun un hydrogène.

4. Composé selon la revendication 3, ou un sel ou solvate pharmaceutiquement acceptable de celui-ci, dans lequel R^{1a} et R^{2a} sont chacun un hydrogène.

5. Composé selon la revendication 2, ou un sel ou solvate pharmaceutiquement acceptable de celui-ci, présentant la Formule III : 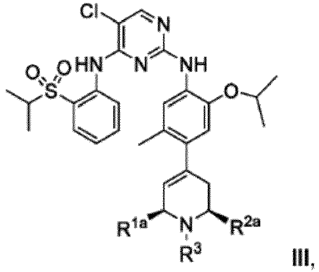 dans lequel :
R^{1a} et R^{2a} sont chacun indépendamment choisis dans le groupe constitué d'un alkyle en C₁₋₄ et d'un cycloalkyle en C₃₋₆ ; et
le composé a un excès énantiomère de 90% ou plus.

6. Composé selon la revendication 3, ou un sel ou solvate pharmaceutiquement acceptable de celui-ci, présentant la Formule IV : 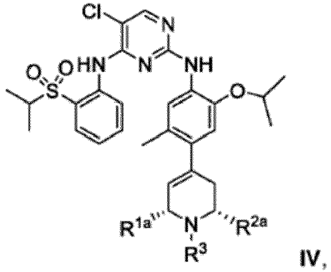 dans lequel :
R^{1a} et R^{2a} sont chacun indépendamment choisis dans le groupe constitué d'un alkyle en C₁₋₄ et d'un cycloalkyle en C₃₋₆ ; et
le composé a un excès énantiomère de 90% ou plus.

7. Composé selon la revendication 1, ou un sel ou solvate pharmaceutiquement acceptable de celui-ci, présentant la Formule V : 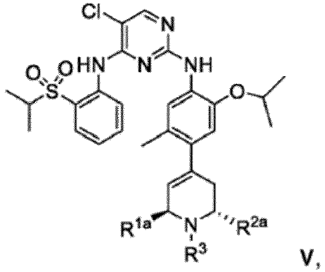 dans lequel :
R^{1a} et R^{2a} sont chacun indépendamment choisis dans le groupe constitué d'un alkyle en C₁₋₄ et d'un cycloalkyle en C₃₋₆ ; et
le composé a un excès énantiomère de 90% ou plus.

8. Composé selon la revendication 1, ou un sel ou solvate pharmaceutiquement acceptable de celui-ci, présentant la Formule VI : 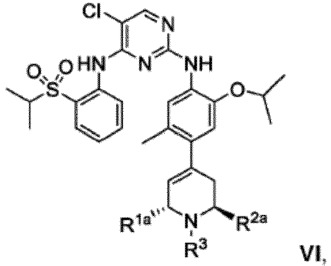 dans lequel :
R^{1a} et R^{2a} sont chacun indépendamment choisis dans le groupe constitué d'un alkyle en C₁₋₄ et d'un cycloalkyle en C₃₋₆ ; et
le composé a un excès énantiomère de 90% ou plus.

9. Composé selon la revendication 8, ou un sel ou solvate pharmaceutiquement acceptable de celui-ci, dans lequel R^{1a} et R^{2a} sont chacun un méthyle.

10. Composé selon la revendication 2, ou un sel ou solvate pharmaceutiquement acceptable de celui-ci, dans lequel R^{1a} et R^{2a} sont chacun un cyclopropyle.

11. Composé selon la revendication 2, ou un sel ou solvate pharmaceutiquement acceptable de celui-ci, dans lequel R³ est choisi dans le groupe constitué de :

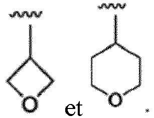

12. Composé selon la revendication 1, ou un sel ou hydrate pharmaceutiquement acceptable de celui-ci, choisi dans le groupe constitué de :
5-chloro-*N²*-(2-isopropoxy-5-méthyl-4-(1-(tétrahydro-2H-pyran-4-yl)-1,2,3,6-tétrahydropyridin-4-yl)phényl)-*N⁴*-(2-(isopropylsulfonyl)phényl)pyrimidine-2,4-diamine ;
5-chloro-*N²*-(2-isopropoxy-5-méthyl-4-(1-(oxétan-3-yl)-1,2,3,6-tétrahydropyridin-4-yl)phényl)-*N⁴*-(2-(isopropylsulfonyl)phényl)pyrimidine-2,4-diamine ;
5-chloro-*N²*-(4-((cis)-2,6-diméthyl-1-(tétrahydro-2H-pyran-4-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-*N²*-(4-((cis)-2,6-diméthyl-1-(oxétan-3-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-*N²*-(4-((trans)-2,6-diéthyl-1-(tétrahydro-2H-pyran-4-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-N²-(4-((2S,6S)-2,6-diéthyl-1-(oxétan-3-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-N²-(4-((trans)-2,6-diméthyl-1-(tétrahydro-2H-pyran-4-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-N²-(4-((trans)-2,6-diméthyl-1-(oxétan-3-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-*N²*-(4-((cis)-2,6-dicyclopropyl-1-(tétrahydro-2H-pyran-4-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-N²-(4-((cis)-2,6-dicyclopropyl-1-(oxétan-3-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-N²-(4-((trans)-2,6-dicyclobutyl-1-(tétrahydro-2H-pyran-4-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-N²-(4-((trans)-2,6-dicyclobutyl-1-(oxétan-3-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-N²-(4-((trans)-2,6-dicyclopropyl-1-(tétrahydro-2H-pyran-4-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-N²-(4-((trans)-2,6-dicyclopropyl-1-(oxétan-3-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-N²-(4-((2R,6S)-2,6-diméthyl-1-(tétrahydro-2H-pyran-4-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-N²-(4-((2R,6S)-2,6-diméthyl-1-(oxétan-3-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-*N²*-(4-((2S,6S)-2,6-diméthyl-1-(tétrahydro-2H-pyran-4-yl)-1,2,3,6-tétrahydropyridin-4-yl)-2-isopropoxy-5-méthylphényl)-*N⁴*-(2-(isopropylsulfonyl)phényl)pyrimidine-2,4-diamine ;
5-chloro-N²-(4-((2S,6S)-2,6-diméthyl-1-(oxétan-3-yl)-1,2,3,6-tétrahydropyridin-4-yl)-2-isopropoxy-5-méthylphényl)-N⁴-(2-(isopropylsulfonyl)phényl)pyrimidine-2,4-diamine ;
5-chloro-N²-(4-((2R,6R)-2,6-diméthyl-1-(tétrahydro-2H-pyran-4-yl)-1,2,3,6-tétrahydropyridin-4-yl)-2-isopropoxy-5-méthylphényl)-N⁴-(2-(isopropylsulfonyl)phényl)pyrimidine-2,4-diamine ;
5-chloro-N²-(4-((2R,6R)-2,6-diméthyl-1-(oxétan-3-yl)-1,2,3,6-tétrahydropyridin-4-yl)-2-isopropoxy-5-méthylphényl)-N⁴-(2-(isopropylsulfonyl)phényl)pyrimidine-2,4-diamine ;
5-chloro-N²-(4-((2S,6S)-2,6-dicyclopropyl-1-(tétrahydro-2H-pyran-4-yl)-1,2,3,6-tétrahydropyridin-4-yl)-2-isopropoxy-5-méthylphényl)-N⁴-(2-(isopropylsulfonyl)phényl)pyrimidine-2,4-diamine ;
5-chloro-N²-(4-((2S,6S)-2,6-dicyclopropyl-1-(oxétan-3-yl)-1,2,3,6-tétrahydropyridin-4-yl)-2-isopropoxy-5-méthylphényl)-N⁴-(2-(isopropylsulfonyl)phényl)pyrimidine-2,4-diamine ;
5-chloro-N²-(4-((2R,6S)-2,6-dicyclopropyl-1-(tétrahydro-2H-pyran-4-yl)-1,2,3,6-tétrahydropyridin-4-yl)-2-isopropoxy-5-méthylphényl)-N⁴-(2-(isopropylsulfonyl)phényl)pyrimidine-2,4-diamine ;
5-chloro-N²-(4-((2R,6S)-2,6-dicyclopropyl-1-(oxétan-3-yl)-1,2,3,6-tétrahydropyridin-4-yl)-2-isopropoxy-5-méthylphényl)-N⁴-(2-(isopropylsulfonyl)phényl)pyrimidine-2,4-diamine ;
5-chloro-N²-(4-((2S,6R)-2,6-dicyclopropyl-1-(tétrahydro-2H-pyran-4-yl)-1,2,3,6-tétrahydropyridin-4-yl)-2-isopropoxy-5-méthylphényl)-N⁴-(2-(isopropylsulfonyl)phényl)pyrimidine-2,4-diamine ; et
5-chloro-N²-(4-((2S,6R)-2,6-dicyclopropyl-1-(oxétan-3-yl)-1,2,3,6-tétrahydropyridin-4-yl)-2-isopropoxy-5-méthylphényl)-N⁴-(2-(isopropylsulfonyl)phényl)pyrimidine-2,4-diamine.

13. Composé selon la revendication 12, ou un sel ou hydrate pharmaceutiquement acceptable de celui-ci, qui est la 5-chloro-N²-(2-isopropoxy-5-méthyl-4-(1-(tétrahydro-2H-pyran-4-yl)-1,2,3,6-tétrahydropyridin-4-yl)phényl)-N⁴-(2-(isopropylsulfonyl)phényl)pyrimidine-2,4-diamine.

14. Composition pharmaceutique comprenant le composé selon l'une quelconque des revendications 1-13, ou un sel ou hydrate pharmaceutiquement acceptable de celui-ci, et un véhicule pharmaceutiquement acceptable.

15. Composé selon l'une quelconque des revendications 1-13, ou un sel, un hydrate ou un solvate pharmaceutiquement acceptable de celui-ci, ou composition pharmaceutique selon la revendication 14, pour utilisation dans le traitement d'un cancer.

16. Composé pour l'utilisation selon la revendication 15, où le cancer est choisi dans le groupe constitué de : lymphome anaplasique à grandes cellules, cancer pulmonaire non à petites cellules, lymphome diffus à grandes cellules B, tumeurs myofibroblastiques inflammatoires, neuroblastome, cancer anaplasique de la thyroïde, rhabdomyosarcome, cancer du sein, cancer colorectal, cancer œsophagien à cellules squameuses et carcinome des cellules rénales.


### SEQUENCE LISTING

<110> ACHAOGEN INC Bender, Kristina Oresic Henry, Christopher E Theolis, Richard Jr Wu, Shuang
<120> PLAZOMICIN ANTIBODIES AND METHODS OF USE
<130> 757832000440
<140> Not Yet Assigned
   <141> Concurrently Herewith
<150> US 62/370,580
   <151> 2016-08-03
<160> 285
<170> PatentIn version 3.5
<210> 1
   <211> 351
   <212> DNA
   <213> Mus musculus
<400> 1
   tctgatgtgc agcttcagga gtcgggacct ggcctggtga aaccttctca gtctctgtcc 60
   ctcacctgca ctgtcactgg ctactcaatc accactaatt atgcctggaa ctggatccgg 120
   cagtttccag gacacaaact ggagtggatg ggctacataa cctccagtgg taggactagc 180
   tacaacccac ctctcaaaag tcgaatctcg atcactcgag acacatccag gagccagttc 240
   ttcctgcgat tgaattctgt gactactgag gacacagcca catatttctg tacacacact 300
   acggcctact tggactactg gggccaaggc accactctca cagtctcctc a 351
<210> 2
   <211> 321
   <212> DNA
   <213> Mus musculus
<400> 2
   gacatcaaga tgacccagtc tccatcctcc atgtatgcat cgctgggaga gagagtcact 60
   atcacttgca aggcgagtca ggacattaaa agctatttaa gctggtacca gcagatacca 120
   tggaaatctc ctaagaccct gatctattat gcaacaagct tggcagatgg ggtcccatca 180
   agattcagtg gcagtggatc tgggcaagat tattctctaa ccatcagcag cctggagtct 240
   gacgatacag caacttatta ctgtctacag catggtgaga gccctctcac gttcggtgct 300
   gggaccaagc tggagctgaa a 321
<210> 3
   <211> 321
   <212> DNA
   <213> Mus musculus
<400> 3
   agtattgtga tgacccagac tcccaaattc ctgcttgtat cagcaggaga cagggttacc 60
   ataacctgca aggccagtca gagtgtgagt aatgatgtag cttggtacca acagaagcca 120
   gggcagtctc ctaaactgct gatatcctat gcatccaatc gctacactgg agtccctgat 180
   cgcttcactg gcagtggata tgggacggat ttcgctttca ccatcaacac tgtgcaggct 240
   gaagacctgg cattttattt ctgtcagcag gattatagct ctccgtacac gttcggaggg 300
   gggaccaagc tggaaataaa a 321
<210> 4
   <211> 351
   <212> DNA
   <213> Mus musculus
<400> 4
   tctgatgtgc agcttcagga gtcgggacct ggcctggtga caccttctca gtctctgtcc      60
   ctcacctgca ctgtcactgg ctactcaatc accagtgctt atgcctggaa ctggatccgg     120
   cagtttccag gaaacaaact ggagtggctg ggctacataa actccagtgg tagcactagc     180
   taccacccat ctctcaaagg tcgaatctct atcactcgag acacatccaa gaaccagttc     240
   ttcctgcaat tgaattctgt gactactgag gacacagcca catattactg tacacacact     300
   acgtcctact ttgactactg gggccaaggc accactctca cagtctcctc a              351
<210> 5
   <211> 321
   <212> DNA
   <213> Mus musculus
<400> 5
   agtattgtga tgacccagac gcccaaattc ctgattgtgt cagcaggaga cagggttacc      60
   ataacctgca aggccagtca gagtgtgagt aatgatgtag cttggtacca acagaagcca     120
   gggcagtctc ctaaactgct gatttcctat gcatccaatc gctacactgg agtccctgat     180
   cgcttcactg gcagtggata tgggacggat ttcactttcg ccatcactac tgtgcaggct     240
   gaggacctgg cagtttattt ctgtcagcag gattatagct ctccgtacac gttcggaggg     300
   gggaccaagg tggagataaa a                                               321
<210> 6
   <211> 348
   <212> DNA
   <213> Mus musculus
<400> 6
   caggtccagc ttcagcagtc tggggctgaa ctggcaaaac ctggggcctc agtgaagatg      60
   tcctgcaagg cttctggcaa cacatttact agttactgga tgcactggat aaaacagagg     120
   cctggacagg gtctggaatg gattggatac attgatccca gcactggtta tactgagtac     180
   aatcagaaat tcaaggacag ggccacattg actgcagaca agtcctccaa cacagcctat     240
   atgcaactga acagcctgac atctgaggac tctgcagtct attactgtac aaaatcaggg     300
   aagtgggacg actactgggg ccaaggcacc actctcacag tctcttca                  348
<210> 7
   <211> 321
   <212> DNA
   <213> Mus musculus
<400> 7
   agtattgtga tgacccagac tcccaaattc ctgcttgtat cagcaggaga cagggttacc 60
   ataacctgca aggccagtca gagtgtgact aatgatgtag cttggtacca acagaagcca 120
   gggcagtctc ctaaactgct gatatgttat gcatccaatc gctacactgg agtccctgat 180
   cgcttcactg gcagtggata tgggacggat ttcactttca ccatcagcac tgtgcaggct 240
   gaagacctgg cagtttattt ctgccagcag gatcatacct ctccgttcac gttcggaggg 300
   gggaccaagt tggaaataaa a 321
<210> 8
   <211> 357
   <212> DNA
   <213> Mus musculus
<400> 8
   caggttcagc tgcagcagtc tggagctgaa ctgatgaagc ctggggcctc agtgaagata 60
   tcctgcaagg ctactggcta caaattcagt aggtactgga tagagtgggt aaaggagagc 120
   cctggacatg gccttgagtg gattggagag atttttcctg gaagtgatag taccgactac 180
   aatgagaagt tcaagggcaa ggccacactc actgcagatt catcctccaa cacagcctac 240
   atgcaactca gcagcctgac atctgaggac tcagccgtct attattgtgc gagagacggt 300
   ggtggcctgg acgagtttgc ttactggggc caagggactc cggtaattgt ctctgca 357
<210> 9
   <211> 336
   <212> DNA
   <213> Mus musculus
<400> 9
   gatgttgtgg tgactcaaaa tccactctcc ctgtctgtca gttttggaga tcaagtttct 60
   atatcttgca ggtctagtca gagtcttaca aacagttatg ggtacaccta tttgtcttgg 120
   tacctgcaca agcctggcca gtctccacag ctcctcatct atgggatttc caacagattt 180
   tctggggtgc cagacaggtt cagtggcagt ggttcgggga cagatttcac actcaagatc 240
   agcacaataa agcctgagga cttgggaatg tattactgct tacaaggtac acatcagccg 300
   tacacgttcg gaggggggac caagctggaa ataaga 336
<210> 10
   <211> 357
   <212> DNA
   <213> Mus musculus
<400> 10
   caggttcagc tgcagcagtc tggagctgaa ctgatgaagc ctggggcctc agtgaagata 60
   tcctgcaagg ctactggcta caaattcagt aggtactgga tagagtgggt aaaggagagc 120
   cctggacatg gccttgagtg gattggagag atttttcctg gaagtgatag taccgactac 180
   aatgagaagt tcaagggcaa ggccacactc actgcagatt catcctccaa cacagcctac 240
   atgcaactca gcagcctgac atctgaggac tcagccgtct attattgtgc gagagacggt 300
   ggtggcctgg acgagtttgc ttactggggc caagggactc cggtaattgt ctctgca 357
<210> 11
   <211> 336
   <212> DNA
   <213> Mus musculus
<400> 11
   gatgttgtgg tgactcaaat tccactctcc ctgtctgtca gctttgggga tcaagtttct 60
   atatcttgca ggtctagtca gagtcttaca aacagttatg ggtacaccta tttgtcttgg 120
   tacctgcaca agcctggcca gtctccacag ctcctcatct atgggatttc caacagattt 180
   tctggggtgc cagacaggtt cagtggcagt ggttcgggga cagatttcac actcaagatc 240
   agcacaataa agcctgagga cttgggaatg tattactgct tacaaggtac acatcagccg 300
   tacacgttcg gaggggggac caagctggaa ataaga 336
<210> 12
   <211> 349
   <212> DNA
   <213> Mus musculus
<400> 12
   gcaggtcagc tgaaggagtc aggacctggc ctggtgaaac cttctcagtc tctgtccctc 60
   acctgcactg tcactggctc ctcaatcacc agtgattatg cctggaactg gatccggcag 120
   tttccaggaa acaaactgga gtggatgggc tacataagcc aaagtggtag cactagctac 180
   aacccatctc tcaaaagtcg aatctctatc actcgagaca catccaagaa ccagttcttc 240
   ctgcagttga attctgtgac tactgaggac acagccacat attactgtac gggtaactca 300
   tatactatgg actactgggg tcaaggaacc tcagtcaccg tctcctcag 349
<210> 13
   <211> 322
   <212> DNA
   <213> Mus musculus
<400> 13
   gacattgtga tgacccagtc tcccaagttc ctgcttgttt cagcaggaga cagggttacc 60
   ataacctgca aggccagtca gagtgtgagt aatgatgtgg cttggtacca acagaagcca 120
   ggacagtctc ctaaactgct gatatcctat gcatccaatc gctacactgg agtccctgat 180
   cgcttcactg gcagtggata tgggacggat ttcactttca ccatcaccac tgtgcaggct 240
   gaagacctgg cagtttattt ctgtcagcag gattatagct ctccgtggac gttcggtgga 300
   ggcaccaagc tggaaatcaa ac 322
<210> 14
   <211> 349
   <212> DNA
   <213> Mus musculus
<400> 14
   cagatgcagc ttcaggagtc aggacctggc ctggtgaaac cttctcagtc tctgtccctc 60
   acctgcactg tcactggcga ctcaatcacc agtgattatg cctggaactg gatccggcag 120
   tttccaggaa acaaactgga gtggatgggc tacataagct acagtggtag cactagttac 180
   aacccatctc tcaaaagtcg aatctctatc agtcgagaca catccaagaa ccagttcttc 240
   ctgcagttga attctgtgac tattgaggac acagccacat attactgtac gggtaactca 300
   tatactatgg actactgggg tcaaggaacc tcagtcaccg tctcctcag 349
<210> 15
   <211> 322
   <212> DNA
   <213> Mus musculus
<400> 15
   agtattgtga tgacccagac tcccaagttc ctgcttgtat cagcaggaga cagggttacc 60
   ataacctgca aggccagtca gagtgtgagt aatgatgtag cttggtacca acagaagccg 120
   gggcagtctc ctaacctgct gatatcctat gcatccaatc gctacactgg agtccctgat 180
   cgcttcactg gcagtggata tgggacggat ttcactttca ccatcagcac tgtgcaggct 240
   gaagacctgg cagtttattt ctgtcagcag gattatatct ctccgtggac gttcggtgga 300
   ggcaccaagc tggaaatcaa ac 322
<210> 16
   <211> 371
   <212> DNA
   <213> Mus musculus
<220>
   <221> misc_feature
   <222> (100)..(100)
   <223> n is a, c, g, or t
<400> 16
   caggtccaac tgcagcagcc tggacctgag ctggtaaagc ctggggcttc agtgaagatg      60
   tcctgcaagg cttctggata cacattcact agctatgttn cagagtgtgc gtaatgatat     120
   tcactgggtg aagcagaagc ctgggcaggg ccttgagtgg attggatata ttaatccttt     180
   caatgatggt actaagtaca atgagaagtt caaaggcaag gccacactga cttcagacaa     240
   atcctccagc actgcctaca tggagctcag cagcctgacc tctgaggact ctgcggtcta     300
   ttactgtgaa ggcgtgggca gctcgggctc cgactactgg ggccaaggca ctactctcac     360
   agtctcctca g                                                          371
<210> 17
   <211> 322
   <212> DNA
   <213> Mus musculus
<400> 17
   gacattgtga tgacacagac tcccaaattc ctgcttgtat cagcaggaga cagggttacc      60
   ataacctgca aggccagtca gagtgtgcgt aatgatgtag cttggtacca acagaagcca     120
   gggcagtctc ctaaattgct gatatcctat gcatccaatc ggtacactgg agtccctgat     180
   cgcttcactg gcagtggata tgggacggat ttcactttca ccatcagcac tgtgcaggct     240
   gaagacctgg caatttattt ctgtcagcag gattatagct ctccgtacac gttcggaggg     300
   gggaccaagc tggaaataaa ac                                              322
<210> 18
   <211> 349
   <212> DNA
   <213> Mus musculus
<400> 18
   caggtccagt tggtgcagtc tggacctgag ctggtgaagc ctggggcttc agtgaaggta      60
   tcctgcaagg cttctggttt ctcattcact gactacaaca tgtactgggt gaagcagagc     120
   catggaaaga gccttgagtg gattggatat attgatcctt acaatggtgg tactagctac     180
   aaccagaagt tcaagggcaa ggccacattg acttttgaca agtcctccag cacagccttc     240
   atgcatctca acagcctgac atctgaggac tctgcagtct attactgtgc aagtgctggt     300
   aactacgatg actactgggg ccaaggcacc actctcacag tctcctcag                 349
<210> 19
   <211> 322
   <212> DNA
   <213> Mus musculus
<400> 19
   gacattgtga tgacacagac tcccaaattc ctgcttgtat cagcaggaga cagggttacc 60
   ataacctgca aggccagtca gagtgtgagt aatgatgtag cttggtacca acagaagcca 120
   gggcagtctc ctacattgct gatatactat gcatccaatc gctacactgg agtccctgat 180
   cgcttcactg gcagtggata tgggacggat ttcactttca ccatcagcac tgtgcaggct 240
   gaagacctgg cagtttattt ctgtcagcag gattatacct ctccgtacac gttcggaggg 300
   gggaccaagc tggaaataaa ac 322
<210> 20
   <211> 355
   <212> DNA
   <213> Mus musculus
<400> 20
   caggttactc tgaaagagtc tggaggaggc ttggtgcaac caggaggatc catgaaaatc 60
   tcctgtgttg tctctggatt gactttcaga aactactgga tgtcttgggt ccgccagtct 120
   ccagagaagg ggcttgagtg ggttgctgaa attagattga aatctgataa ttatgcaaca 180
   cattatgcgg agtctgtgaa agggaagttc accatctcaa gagatgattc taaaagtcgt 240
   ctctacctgc aaatgaacag cttaagagct gaagacactg gaatttatta ctgtacggtc 300
   tatggtaaag actttgacta ctggggccaa ggcaccactc tcacagtctc ctcag 355
<210> 21
   <211> 322
   <212> DNA
   <213> Mus musculus
<400> 21
   gatgttgtga tgacccaaac tcccaaattc ctgcttgtat cagcaggaga cagggttacc 60
   ataacctgca aggccagtca gagtgtgagt aatgatgtag cttggtacca acagaagcca 120
   gggcagtctc ctaaactgct gatatcctat gcatccaatc gctacactgg agtccctgat 180
   cgcttcactg gcagtggata tgggacggat ttcactttca ccatcagcac tgtgcaggct 240
   gaagacctgg cagtttattt ctgtcagcag gattatagct ctccgtggac gttcggtgga 300
   ggcaccaagc tggaaatcaa ac 322
<210> 22
   <211> 117
   <212> PRT
   <213> Mus musculus
<400> 22
   Ser Asp Val Gln Leu Gln Glu Ser Gly Pro Gly Leu Val Lys Pro Ser
   1               5                   10                  15
   Gln Ser Leu Ser Leu Thr Cys Thr Val Thr Gly Tyr Ser Ile Thr Thr
               20                  25                  30
   Asn Tyr Ala Trp Asn Trp Ile Arg Gln Phe Pro Gly His Lys Leu Glu
           35                  40                  45
   Trp Met Gly Tyr Ile Thr Ser Ser Gly Arg Thr Ser Tyr Asn Pro Pro
       50                  55                  60
   Leu Lys Ser Arg Ile Ser Ile Thr Arg Asp Thr Ser Arg Ser Gln Phe
   65                  70                  75                  80
   Phe Leu Arg Leu Asn Ser Val Thr Thr Glu Asp Thr Ala Thr Tyr Phe
                   85                  90                  95
   Cys Thr His Thr Thr Ala Tyr Leu Asp Tyr Trp Gly Gln Gly Thr Thr
               100                 105                 110
   Leu Thr Val Ser Ser
           115
<210> 23
   <211> 8
   <212> PRT
   <213> Mus musculus
<400> 23
   Gly Tyr Ser Ile Thr Thr Asn Tyr
   1               5
<210> 24
   <211> 7
   <212> PRT
   <213> Mus musculus
<400> 24
   Ile Thr Ser Ser Gly Arg Thr
   1               5
<210> 25
   <211> 9
   <212> PRT
   <213> Mus musculus
<400> 25
   Thr His Thr Thr Ala Tyr Leu Asp Tyr
   1 5
<210> 26
   <211> 107
   <212> PRT
   <213> Mus musculus
<400> 26
   Ser Ile Val Met Thr Gln Thr Pro Lys Phe Leu Leu Val Ser Ala Gly
   1 5 10 15
   Asp Arg Val Thr Ile Thr Cys Lys Ala Ser Gln Ser Val Ser Asn Asp
   20 25 30
   Val Ala Trp Tyr Gln Gln Lys Pro Gly Gln Ser Pro Lys Leu Leu Ile
   35 40 45
   Ser Tyr Ala Ser Asn Arg Tyr Thr Gly Val Pro Asp Arg Phe Thr Gly
   50 55 60
   Ser Gly Tyr Gly Thr Asp Phe Ala Phe Thr Ile Asn Thr Val Gln Ala
   65 70 75 80
   Glu Asp Leu Ala Phe Tyr Phe Cys Gln Gln Asp Tyr Ser Ser Pro Tyr
   85 90 95
   Thr Phe Gly Gly Gly Thr Lys Leu Glu Ile Lys
   100 105
<210> 27
   <211> 6
   <212> PRT
   <213> Mus musculus
<400> 27
   Gln Ser Val Ser Asn Asp
   1 5
<210> 28
   <211> 3
   <212> PRT
   <213> Mus musculus
<400> 28
   Tyr Ala Ser
   1
<210> 29
   <211> 9
   <212> PRT
   <213> Mus musculus
<400> 29
   Gln Gln Asp Tyr Ser Ser Pro Tyr Thr
   1               5
<210> 30
   <211> 107
   <212> PRT
   <213> Mus musculus
<400> 30
   Asp Ile Lys Met Thr Gln Ser Pro Ser Ser Met Tyr Ala Ser Leu Gly
   1               5                   10                  15
   Glu Arg Val Thr Ile Thr Cys Lys Ala Ser Gln Asp Ile Lys Ser Tyr
               20                  25                  30
   Leu Ser Trp Tyr Gln Gln Ile Pro Trp Lys Ser Pro Lys Thr Leu Ile
           35                  40                  45
   Tyr Tyr Ala Thr Ser Leu Ala Asp Gly Val Pro Ser Arg Phe Ser Gly
       50                  55                  60
   Ser Gly Ser Gly Gln Asp Tyr Ser Leu Thr Ile Ser Ser Leu Glu Ser
   65                  70                  75                  80
   Asp Asp Thr Ala Thr Tyr Tyr Cys Leu Gln His Gly Glu Ser Pro Leu
                   85                  90                  95
   Thr Phe Gly Ala Gly Thr Lys Leu Glu Leu Lys
               100                 105
<210> 31
   <211> 6
   <212> PRT
   <213> Mus musculus
<400> 31
   Gln Asp Ile Lys Ser Tyr
   1               5
<210> 32
   <211> 3
   <212> PRT
   <213> Mus musculus
<400> 32
   Tyr Ala Thr
   1
<210> 33
   <211> 19
   <212> PRT
   <213> Mus musculus
<400> 33
   Leu Gln His Gly Glu Ser Pro Leu Thr Phe Gly Ala Gly Thr Lys Leu
   1 5 10 15
   Glu Leu Lys
<210> 34
   <211> 117
   <212> PRT
   <213> Mus musculus
<400> 34
   Ser Asp Val Gln Leu Gln Glu Ser Gly Pro Gly Leu Val Thr Pro Ser
   1 5 10 15
   Gln Ser Leu Ser Leu Thr Cys Thr Val Thr Gly Tyr Ser Ile Thr Ser
   20 25 30
   Ala Tyr Ala Trp Asn Trp Ile Arg Gln Phe Pro Gly Asn Lys Leu Glu
   35 40 45
   Trp Leu Gly Tyr Ile Asn Ser Ser Gly Ser Thr Ser Tyr His Pro Ser
   50 55 60
   Leu Lys Gly Arg Ile Ser Ile Thr Arg Asp Thr Ser Lys Asn Gln Phe
   65 70 75 80
   Phe Leu Gln Leu Asn Ser Val Thr Thr Glu Asp Thr Ala Thr Tyr Tyr
   85 90 95
   Cys Thr His Thr Thr Ser Tyr Phe Asp Tyr Trp Gly Gln Gly Thr Thr
   100 105 110
   Leu Thr Val Ser Ser
   115
<210> 35
   <211> 9
   <212> PRT
   <213> Mus musculus
<400> 35
   Gly Tyr Ser Ile Thr Ser Ala Tyr Ala
   1               5
<210> 36
   <211> 7
   <212> PRT
   <213> Mus musculus
<400> 36
   Ile Asn Ser Ser Gly Ser Thr
   1               5
<210> 37
   <211> 9
   <212> PRT
   <213> Mus musculus
<400> 37
   Thr His Thr Thr Ser Tyr Phe Asp Tyr
   1               5
<210> 38
   <211> 107
   <212> PRT
   <213> Mus musculus
<400> 38
   Ser Ile Val Met Thr Gln Thr Pro Lys Phe Leu Ile Val Ser Ala Gly
   1               5                   10                  15
   Asp Arg Val Thr Ile Thr Cys Lys Ala Ser Gln Ser Val Ser Asn Asp
               20                  25                  30
   Val Ala Trp Tyr Gln Gln Lys Pro Gly Gln Ser Pro Lys Leu Leu Ile
           35                  40                  45
   Ser Tyr Ala Ser Asn Arg Tyr Thr Gly Val Pro Asp Arg Phe Thr Gly
       50                  55                  60
   Ser Gly Tyr Gly Thr Asp Phe Thr Phe Ala Ile Thr Thr Val Gln Ala
   65                  70                  75                  80
   Glu Asp Leu Ala Val Tyr Phe Cys Gln Gln Asp Tyr Ser Ser Pro Tyr
                   85                  90                  95
   Thr Phe Gly Gly Gly Thr Lys Val Glu Ile Lys
               100                 105
<210> 39
   <211> 6
   <212> PRT
   <213> Mus musculus
<400> 39
   Gln Ser Val Ser Asn Asp
   1               5
<210> 40
   <211> 3
   <212> PRT
   <213> Mus musculus
<400> 40
   Tyr Ala Ser
   1
<210> 41
   <211> 9
   <212> PRT
   <213> Mus musculus
<400> 41
   Gln Gln Asp Tyr Ser Ser Pro Tyr Thr
   1               5
<210> 42
   <211> 116
   <212> PRT
   <213> Mus musculus
<400> 42
   Gln Val Gln Leu Gln Gln Ser Gly Ala Glu Leu Ala Lys Pro Gly Ala
   1               5                   10                  15
   Ser Val Lys Met Ser Cys Lys Ala Ser Gly Asn Thr Phe Thr Ser Tyr
               20                  25                  30
   Trp Met His Trp Ile Lys Gln Arg Pro Gly Gln Gly Leu Glu Trp Ile
           35                  40                  45
   Gly Tyr Ile Asp Pro Ser Thr Gly Tyr Thr Glu Tyr Asn Gln Lys Phe
       50                  55                  60
   Lys Asp Arg Ala Thr Leu Thr Ala Asp Lys Ser Ser Asn Thr Ala Tyr
   65                  70                  75                  80
   Met Gln Leu Asn Ser Leu Thr Ser Glu Asp Ser Ala Val Tyr Tyr Cys
                   85                  90                  95
   Thr Lys Ser Gly Lys Trp Asp Asp Tyr Trp Gly Gln Gly Thr Thr Leu
               100                 105                 110
   Thr Val Ser Ser
           115
<210> 43
   <211> 8
   <212> PRT
   <213> Mus musculus
<400> 43
   Gly Asn Thr Phe Thr Ser Tyr Trp
   1               5
<210> 44
   <211> 8
   <212> PRT
   <213> Mus musculus
<400> 44
   Ile Asp Pro Ser Thr Gly Tyr Thr
   1               5
<210> 45
   <211> 9
   <212> PRT
   <213> Mus musculus
<400> 45
   Thr Lys Ser Gly Lys Trp Asp Asp Tyr
   1 5
<210> 46
   <211> 107
   <212> PRT
   <213> Mus musculus
<400> 46
   Ser Ile Val Met Thr Gln Thr Pro Lys Phe Leu Leu Val Ser Ala Gly
   1 5 10 15
   Asp Arg Val Thr Ile Thr Cys Lys Ala Ser Gln Ser Val Thr Asn Asp
   20 25 30
   Val Ala Trp Tyr Gln Gln Lys Pro Gly Gln Ser Pro Lys Leu Leu Ile
   35 40 45
   Cys Tyr Ala Ser Asn Arg Tyr Thr Gly Val Pro Asp Arg Phe Thr Gly
   50 55 60
   Ser Gly Tyr Gly Thr Asp Phe Thr Phe Thr Ile Ser Thr Val Gln Ala
   65 70 75 80
   Glu Asp Leu Ala Val Tyr Phe Cys Gln Gln Asp His Thr Ser Pro Phe
   85 90 95
   Thr Phe Gly Gly Gly Thr Lys Leu Glu Ile Lys
   100 105
<210> 47
   <211> 6
   <212> PRT
   <213> Mus musculus
<400> 47
   Gln Ser Val Thr Asn Asp
   1 5
<210> 48
   <211> 3
   <212> PRT
   <213> Mus musculus
<400> 48
   Tyr Ala Ser
   1
<210> 49
   <211> 10
   <212> PRT
   <213> Mus musculus
<400> 49
   Gln Gln Asp His Thr Ser Pro Phe Thr Phe
   1               5                   10
<210> 50
   <211> 119
   <212> PRT
   <213> Mus musculus
<400> 50
   Gln Val Gln Leu Gln Gln Ser Gly Ala Glu Leu Met Lys Pro Gly Ala
   1               5                   10                  15
   Ser Val Lys Ile Ser Cys Lys Ala Thr Gly Tyr Lys Phe Ser Arg Tyr
               20                  25                  30
   Trp Ile Glu Trp Val Lys Glu Ser Pro Gly His Gly Leu Glu Trp Ile
           35                  40                  45
   Gly Glu Ile Phe Pro Gly Ser Asp Ser Thr Asp Tyr Asn Glu Lys Phe
       50                  55                  60
   Lys Gly Lys Ala Thr Leu Thr Ala Asp Ser Ser Ser Asn Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Asp Gly Gly Gly Leu Asp Glu Phe Ala Tyr Trp Gly Gln Gly
               100                 105                 110
   Thr Pro Val Ile Val Ser Ala
           115
<210> 51
   <211> 8
   <212> PRT
   <213> Mus musculus
<400> 51
   Gly Tyr Lys Phe Ser Arg Tyr Trp
   1               5
<210> 52
   <211> 8
   <212> PRT
   <213> Mus musculus
<400> 52
   Ile Phe Pro Gly Ser Asp Ser Thr
   1               5
<210> 53
   <211> 12
   <212> PRT
   <213> Mus musculus
<400> 53
   Ala Arg Asp Gly Gly Gly Leu Asp Glu Phe Ala Tyr
   1               5                   10
<210> 54
   <211> 112
   <212> PRT
   <213> Mus musculus
<400> 54
   Asp Val Val Val Thr Gln Asn Pro Leu Ser Leu Ser Val Ser Phe Gly
   1               5                   10                  15
   Asp Gln Val Ser Ile Ser Cys Arg Ser Ser Gln Ser Leu Thr Asn Ser
               20                  25                  30
   Tyr Gly Tyr Thr Tyr Leu Ser Trp Tyr Leu His Lys Pro Gly Gln Ser
           35                  40                  45
   Pro Gln Leu Leu Ile Tyr Gly Ile Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Thr Ile Lys Pro Glu Asp Leu Gly Met Tyr Tyr Cys Leu Gln Gly
                   85                  90                  95
   Thr His Gln Pro Tyr Thr Phe Gly Gly Gly Thr Lys Leu Glu Ile Arg
               100                 105                 110
<210> 55
   <211> 9
   <212> PRT
   <213> Mus musculus
<400> 55
   Gln Ser Leu Thr Asn Ser Tyr Gly Tyr
   1 5
<210> 56
   <211> 3
   <212> PRT
   <213> Mus musculus
<400> 56
   Gly Ile Ser
   1
<210> 57
   <211> 9
   <212> PRT
   <213> Mus musculus
<400> 57
   Leu Gln Gly Thr His Gln Pro Tyr Thr
   1 5
<210> 58
   <211> 119
   <212> PRT
   <213> Mus musculus
<400> 58
   Gln Val Gln Leu Gln Gln Ser Gly Ala Glu Leu Met Lys Pro Gly Ala
   1 5 10 15
   Ser Val Lys Ile Ser Cys Lys Ala Thr Gly Tyr Lys Phe Ser Arg Tyr
               20                  25                  30
   Trp Ile Glu Trp Val Lys Glu Ser Pro Gly His Gly Leu Glu Trp Ile
           35                  40                  45
   Gly Glu Ile Phe Pro Gly Ser Asp Ser Thr Asp Tyr Asn Glu Lys Phe
       50                  55                  60
   Lys Gly Lys Ala Thr Leu Thr Ala Asp Ser Ser Ser Asn Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Asp Gly Gly Gly Leu Asp Glu Phe Ala Tyr Trp Gly Gln Gly
               100                 105                 110
   Thr Pro Val Ile Val Ser Ala
           115
<210> 59
   <211> 8
   <212> PRT
   <213> Mus musculus
<400> 59
   Gly Tyr Lys Phe Ser Arg Tyr Trp
   1               5
<210> 60
   <211> 8
   <212> PRT
   <213> Mus musculus
<400> 60
   Ile Phe Pro Gly Ser Asp Ser Thr
   1               5
<210> 61
   <211> 12
   <212> PRT
   <213> Mus musculus
<400> 61
   Ala Arg Asp Gly Gly Gly Leu Asp Glu Phe Ala Tyr
   1               5                   10
<210> 62
   <211> 112
   <212> PRT
   <213> Mus musculus
<400> 62
   Asp Val Val Val Thr Gln Ile Pro Leu Ser Leu Ser Val Ser Phe Gly
   1               5                   10                  15
   Asp Gln Val Ser Ile Ser Cys Arg Ser Ser Gln Ser Leu Thr Asn Ser
               20                  25                  30
   Tyr Gly Tyr Thr Tyr Leu Ser Trp Tyr Leu His Lys Pro Gly Gln Ser
           35                  40                  45
   Pro Gln Leu Leu Ile Tyr Gly Ile Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Thr Ile Lys Pro Glu Asp Leu Gly Met Tyr Tyr Cys Leu Gln Gly
                   85                  90                  95
   Thr His Gln Pro Tyr Thr Phe Gly Gly Gly Thr Lys Leu Glu Ile Arg
               100                 105                 110
<210> 63
   <211> 11
   <212> PRT
   <213> Mus musculus
<400> 63
   Gln Ser Leu Thr Asn Ser Tyr Gly Tyr Thr Tyr
   1               5                   10
<210> 64
   <211> 3
   <212> PRT
   <213> Mus musculus
<400> 64
   Gly Ile Ser
   1
<210> 65
   <211> 9
   <212> PRT
   <213> Mus musculus
<400> 65
   Leu Gln Gly Thr His Gln Pro Tyr Thr
   1 5
<210> 66
   <211> 116
   <212> PRT
   <213> Mus musculus
<400> 66
   Ala Gly Gln Leu Lys Glu Ser Gly Pro Gly Leu Val Lys Pro Ser Gln
   1 5 10 15
   Ser Leu Ser Leu Thr Cys Thr Val Thr Gly Ser Ser Ile Thr Ser Asp
   20 25 30
   Tyr Ala Trp Asn Trp Ile Arg Gln Phe Pro Gly Asn Lys Leu Glu Trp
   35 40 45
   Met Gly Tyr Ile Ser Gln Ser Gly Ser Thr Ser Tyr Asn Pro Ser Leu
   50 55 60
   Lys Ser Arg Ile Ser Ile Thr Arg Asp Thr Ser Lys Asn Gln Phe Phe
   65 70 75 80
   Leu Gln Leu Asn Ser Val Thr Thr Glu Asp Thr Ala Thr Tyr Tyr Cys
   85 90 95
   Thr Gly Asn Ser Tyr Thr Met Asp Tyr Trp Gly Gln Gly Thr Ser Val
   100 105 110
   Thr Val Ser Ser
   115
<210> 67
   <211> 9
   <212> PRT
   <213> Mus musculus
<400> 67
   Gly Ser Ser Ile Thr Ser Asp Tyr Ala
   1 5
<210> 68
   <211> 7
   <212> PRT
   <213> Mus musculus
<400> 68
   Ile Ser Gln Ser Gly Ser Thr
   1               5
<210> 69
   <211> 9
   <212> PRT
   <213> Mus musculus
<400> 69
   Thr Gly Asn Ser Tyr Thr Met Asp Tyr
   1               5
<210> 70
   <211> 107
   <212> PRT
   <213> Mus musculus
<400> 70
   Asp Ile Val Met Thr Gln Ser Pro Lys Phe Leu Leu Val Ser Ala Gly
   1               5                   10                  15
   Asp Arg Val Thr Ile Thr Cys Lys Ala Ser Gln Ser Val Ser Asn Asp
               20                  25                  30
   Val Ala Trp Tyr Gln Gln Lys Pro Gly Gln Ser Pro Lys Leu Leu Ile
           35                  40                  45
   Ser Tyr Ala Ser Asn Arg Tyr Thr Gly Val Pro Asp Arg Phe Thr Gly
       50                  55                  60
   Ser Gly Tyr Gly Thr Asp Phe Thr Phe Thr Ile Thr Thr Val Gln Ala
   65                  70                  75                  80
   Glu Asp Leu Ala Val Tyr Phe Cys Gln Gln Asp Tyr Ser Ser Pro Trp
                   85                  90                  95
   Thr Phe Gly Gly Gly Thr Lys Leu Glu Ile Lys
               100                 105
<210> 71
   <211> 6
   <212> PRT
   <213> Mus musculus
<400> 71
   Gln Ser Val Ser Asn Asp
   1               5
<210> 72
   <211> 3
   <212> PRT
   <213> Mus musculus
<400> 72
   Tyr Ala Ser
   1
<210> 73
   <211> 9
   <212> PRT
   <213> Mus musculus
<400> 73
   Gln Gln Asp Tyr Ser Ser Pro Trp Thr
   1               5
<210> 74
   <211> 116
   <212> PRT
   <213> Mus musculus
<400> 74
   Gln Met Gln Leu Gln Glu Ser Gly Pro Gly Leu Val Lys Pro Ser Gln
   1               5                   10                  15
   Ser Leu Ser Leu Thr Cys Thr Val Thr Gly Asp Ser Ile Thr Ser Asp
               20                  25                  30
   Tyr Ala Trp Asn Trp Ile Arg Gln Phe Pro Gly Asn Lys Leu Glu Trp
           35                  40                  45
   Met Gly Tyr Ile Ser Tyr Ser Gly Ser Thr Ser Tyr Asn Pro Ser Leu
       50                  55                  60
   Lys Ser Arg Ile Ser Ile Ser Arg Asp Thr Ser Lys Asn Gln Phe Phe
   65                  70                  75                  80
   Leu Gln Leu Asn Ser Val Thr Ile Glu Asp Thr Ala Thr Tyr Tyr Cys
                   85                  90                  95
   Thr Gly Asn Ser Tyr Thr Met Asp Tyr Trp Gly Gln Gly Thr Ser Val
               100                 105                 110
   Thr Val Ser Ser
           115
<210> 75
   <211> 9
   <212> PRT
   <213> Mus musculus
<400> 75
   Gly Asp Ser Ile Thr Ser Asp Tyr Ala
   1               5
<210> 76
   <211> 7
   <212> PRT
   <213> Mus musculus
<400> 76
   Ile Ser Tyr Ser Gly Ser Thr
   1               5
<210> 77
   <211> 9
   <212> PRT
   <213> Mus musculus
<400> 77
   Thr Gly Asn Ser Tyr Thr Met Asp Tyr
   1               5
<210> 78
   <211> 107
   <212> PRT
   <213> Mus musculus
<400> 78
   Ser Ile Val Met Thr Gln Thr Pro Lys Phe Leu Leu Val Ser Ala Gly
   1               5                   10                  15
   Asp Arg Val Thr Ile Thr Cys Lys Ala Ser Gln Ser Val Ser Asn Asp
               20                  25                  30
   Val Ala Trp Tyr Gln Gln Lys Pro Gly Gln Ser Pro Asn Leu Leu Ile
           35                  40                  45
   Ser Tyr Ala Ser Asn Arg Tyr Thr Gly Val Pro Asp Arg Phe Thr Gly
       50                  55                  60
   Ser Gly Tyr Gly Thr Asp Phe Thr Phe Thr Ile Ser Thr Val Gln Ala
   65                  70                  75                  80
   Glu Asp Leu Ala Val Tyr Phe Cys Gln Gln Asp Tyr Ile Ser Pro Trp
                   85                  90                  95
   Thr Phe Gly Gly Gly Thr Lys Leu Glu Ile Lys
               100                 105
<210> 79
   <211> 6
   <212> PRT
   <213> Mus musculus
<400> 79
   Gln Ser Val Ser Asn Asp
   1               5
<210> 80
   <211> 3
   <212> PRT
   <213> Mus musculus
<400> 80
   Tyr Ala Ser
   1
<210> 81
   <211> 9
   <212> PRT
   <213> Mus musculus
<400> 81
   Gln Gln Asp Tyr Ile Ser Pro Trp Thr
   1               5
<210> 82
   <211> 117
   <212> PRT
   <213> Mus musculus
<400> 82
   Gln Val Gln Leu Gln Gln Pro Gly Pro Glu Leu Val Lys Pro Gly Ala
   1               5                   10                  15
   Ser Val Lys Met Ser Cys Lys Ala Ser Gly Tyr Thr Phe Thr Ser Tyr
               20                  25                  30
   Val Ile His Trp Val Lys Gln Lys Pro Gly Gln Gly Leu Glu Trp Ile
           35                  40                  45
   Gly Tyr Ile Asn Pro Phe Asn Asp Gly Thr Lys Tyr Asn Glu Lys Phe
       50                  55                  60
   Lys Gly Lys Ala Thr Leu Thr Ser Asp Lys Ser Ser Ser Thr Ala Tyr
   65                  70                  75                  80
   Met Glu Leu Ser Ser Leu Thr Ser Glu Asp Ser Ala Val Tyr Tyr Cys
                   85                  90                  95
   Glu Gly Val Gly Ser Ser Gly Ser Asp Tyr Trp Gly Gln Gly Thr Thr
                   100                 105                 110
   Leu Thr Val Ser Ser
           115
<210> 83
   <211> 8
   <212> PRT
   <213> Mus musculus
<400> 83
   Gly Tyr Thr Phe Thr Ser Tyr Val
   1               5
<210> 84
   <211> 8
   <212> PRT
   <213> Mus musculus
<400> 84
   Ile Asn Pro Phe Asn Asp Gly Thr
   1               5
<210> 85
   <211> 10
   <212> PRT
   <213> Mus musculus
<400> 85
   Glu Gly Val Gly Ser Ser Gly Ser Asp Tyr
   1               5                   10
<210> 86
   <211> 107
   <212> PRT
   <213> Mus musculus
<400> 86
   Asp Ile Val Met Thr Gln Thr Pro Lys Phe Leu Leu Val Ser Ala Gly
   1               5                   10                  15
   Asp Arg Val Thr Ile Thr Cys Lys Ala Ser Gln Ser Val Arg Asn Asp
               20                  25                  30
   Val Ala Trp Tyr Gln Gln Lys Pro Gly Gln Ser Pro Lys Leu Leu Ile
           35                  40                  45
   Ser Tyr Ala Ser Asn Arg Tyr Thr Gly Val Pro Asp Arg Phe Thr Gly
       50                  55                  60
   Ser Gly Tyr Gly Thr Asp Phe Thr Phe Thr Ile Ser Thr Val Gln Ala
   65                  70                  75                  80
   Glu Asp Leu Ala Ile Tyr Phe Cys Gln Gln Asp Tyr Ser Ser Pro Tyr
                   85                  90                  95
   Thr Phe Gly Gly Gly Thr Lys Leu Glu Ile Lys
               100                 105
<210> 87
   <211> 6
   <212> PRT
   <213> Mus musculus
<400> 87
   Gln Ser Val Arg Asn Asp
   1               5
<210> 88
   <211> 3
   <212> PRT
   <213> Mus musculus
<400> 88
   Tyr Ala Ser
   1
<210> 89
   <211> 9
   <212> PRT
   <213> Mus musculus
<400> 89
   Gln Gln Asp Tyr Ser Ser Pro Tyr Thr
   1 5
<210> 90
   <211> 116
   <212> PRT
   <213> Mus musculus
<400> 90
   Gln Val Gln Leu Val Gln Ser Gly Pro Glu Leu Val Lys Pro Gly Ala
   1 5 10 15
   Ser Val Lys Val Ser Cys Lys Ala Ser Gly Phe Ser Phe Thr Asp Tyr
   20 25 30
   Asn Met Tyr Trp Val Lys Gln Ser His Gly Lys Ser Leu Glu Trp Ile
   35 40 45
   Gly Tyr Ile Asp Pro Tyr Asn Gly Gly Thr Ser Tyr Asn Gln Lys Phe
   50 55 60
   Lys Gly Lys Ala Thr Leu Thr Phe Asp Lys Ser Ser Ser Thr Ala Phe
   65 70 75 80
   Met His Leu Asn Ser Leu Thr Ser Glu Asp Ser Ala Val Tyr Tyr Cys
   85 90 95
   Ala Ser Ala Gly Asn Tyr Asp Asp Tyr Trp Gly Gln Gly Thr Thr Leu
   100 105 110
   Thr Val Ser Ser
   115
<210> 91
   <211> 8
   <212> PRT
   <213> Mus musculus
<400> 91
   Gly Phe Ser Phe Thr Asp Tyr Asn
   1 5
<210> 92
   <211> 8
   <212> PRT
   <213> Mus musculus
<400> 92
   Ile Asp Pro Tyr Asn Gly Gly Thr
   1               5
<210> 93
   <211> 9
   <212> PRT
   <213> Mus musculus
<400> 93
   Ala Ser Ala Gly Asn Tyr Asp Asp Tyr
   1               5
<210> 94
   <211> 107
   <212> PRT
   <213> Mus musculus
<400> 94
   Asp Ile Val Met Thr Gln Thr Pro Lys Phe Leu Leu Val Ser Ala Gly
   1               5                   10                  15
   Asp Arg Val Thr Ile Thr Cys Lys Ala Ser Gln Ser Val Ser Asn Asp
               20                  25                  30
   Val Ala Trp Tyr Gln Gln Lys Pro Gly Gln Ser Pro Thr Leu Leu Ile
           35                  40                  45
   Tyr Tyr Ala Ser Asn Arg Tyr Thr Gly Val Pro Asp Arg Phe Thr Gly
       50                  55                  60
   Ser Gly Tyr Gly Thr Asp Phe Thr Phe Thr Ile Ser Thr Val Gln Ala
   65                  70                  75                  80
   Glu Asp Leu Ala Val Tyr Phe Cys Gln Gln Asp Tyr Thr Ser Pro Tyr
                   85                  90                  95
   Thr Phe Gly Gly Gly Thr Lys Leu Glu Ile Lys
               100                 105
<210> 95
   <211> 6
   <212> PRT
   <213> Mus musculus
<400> 95
   Gln Ser Val Ser Asn Asp
   1 5
<210> 96
   <211> 3
   <212> PRT
   <213> Mus musculus
<400> 96
   Tyr Ala Ser
   1
<210> 97
   <211> 9
   <212> PRT
   <213> Mus musculus
<400> 97
   Gln Gln Asp Tyr Thr Ser Pro Tyr Thr
   1 5
<210> 98
   <211> 118
   <212> PRT
   <213> Mus musculus
<400> 98
   Gln Val Thr Leu Lys Glu Ser Gly Gly Gly Leu Val Gln Pro Gly Gly
   1               5                   10                  15
   Ser Met Lys Ile Ser Cys Val Val Ser Gly Leu Thr Phe Arg Asn Tyr
               20                  25                  30
   Trp Met Ser Trp Val Arg Gln Ser Pro Glu Lys Gly Leu Glu Trp Val
           35                  40                  45
   Ala Glu Ile Arg Leu Lys Ser Asp Asn Tyr Ala Thr His Tyr Ala Glu
       50                  55                  60
   Ser Val Lys Gly Lys Phe Thr Ile Ser Arg Asp Asp Ser Lys Ser Arg
   65                  70                  75                  80
   Leu Tyr Leu Gln Met Asn Ser Leu Arg Ala Glu Asp Thr Gly Ile Tyr
                   85                  90                  95
   Tyr Cys Thr Val Tyr Gly Lys Asp Phe Asp Tyr Trp Gly Gln Gly Thr
               100                 105                 110
   Thr Leu Thr Val Ser Ser
           115
<210> 99
   <211> 8
   <212> PRT
   <213> Mus musculus
<400> 99
   Gly Leu Thr Phe Arg Asn Tyr Trp
   1               5
<210> 100
   <211> 10
   <212> PRT
   <213> Mus musculus
<400> 100
   Ile Arg Leu Lys Ser Asp Asn Tyr Ala Thr
   1               5                   10
<210> 101
   <211> 9
   <212> PRT
   <213> Mus musculus
<400> 101
   Thr Val Tyr Gly Lys Asp Phe Asp Tyr
   1               5
<210> 102
   <211> 107
   <212> PRT
   <213> Mus musculus
<400> 102
   Asp Val Val Met Thr Gln Thr Pro Lys Phe Leu Leu Val Ser Ala Gly
   1               5                   10                  15
   Asp Arg Val Thr Ile Thr Cys Lys Ala Ser Gln Ser Val Ser Asn Asp
               20                  25                  30
   Val Ala Trp Tyr Gln Gln Lys Pro Gly Gln Ser Pro Lys Leu Leu Ile
           35                  40                  45
   Ser Tyr Ala Ser Asn Arg Tyr Thr Gly Val Pro Asp Arg Phe Thr Gly
       50                  55                  60
   Ser Gly Tyr Gly Thr Asp Phe Thr Phe Thr Ile Ser Thr Val Gln Ala
   65                  70                  75                  80
   Glu Asp Leu Ala Val Tyr Phe Cys Gln Gln Asp Tyr Ser Ser Pro Trp
                   85                  90                  95
   Thr Phe Gly Gly Gly Thr Lys Leu Glu Ile Lys
               100                 105
<210> 103
   <211> 6
   <212> PRT
   <213> Mus musculus
<400> 103
   Gln Ser Val Ser Asn Asp
   1               5
<210> 104
   <211> 3
   <212> PRT
   <213> Mus musculus
<400> 104
   Tyr Ala Ser
   1
<210> 105
   <211> 9
   <212> PRT
   <213> Mus musculus
<400> 105
   Gln Gln Asp Tyr Ser Ser Pro Trp Thr
   1 5
<210> 106
   <211> 6
   <212> PRT
   <213> Mus musculus
<400> 106
   Thr Asn Tyr Ala Trp Asn
   1 5
<210> 107
   <211> 7
   <212> PRT
   <213> Mus musculus
<400> 107
   Gly Tyr Ser Ile Thr Thr Asn
   1 5
<210> 108
   <211> 7
   <212> PRT
   <213> Mus musculus
<400> 108
   Thr Thr Asn Tyr Ala Trp Asn
   1 5
<210> 109
   <211> 16
   <212> PRT
   <213> Mus musculus
<400> 109
   Tyr Ile Thr Ser Ser Gly Arg Thr Ser Tyr Asn Pro Pro Leu Lys Ser
   1 5 10 15
<210> 110
   <211> 3
   <212> PRT
   <213> Mus musculus
<400> 110
   Ser Ser Gly
   1
<210> 111
   <211> 12
   <212> PRT
   <213> Mus musculus
<400> 111
   Trp Met Gly Tyr Ile Thr Ser Ser Gly Arg Thr Ser
   1 5 10
<210> 112
   <211> 7
   <212> PRT
   <213> Mus musculus
<400> 112
   Thr Thr Ala Tyr Leu Asp Tyr
   1 5
<210> 113
   <211> 5
   <212> PRT
   <213> Mus musculus
<400> 113
   Thr Ala Tyr Leu Asp
   1 5
<210> 114
   <211> 8
   <212> PRT
   <213> Mus musculus
<400> 114
   Thr His Thr Thr Ala Tyr Leu Asp
   1 5
<210> 115
   <211> 6
   <212> PRT
   <213> Mus musculus
<400> 115
   Ser Ala Tyr Ala Trp Asn
   1 5
<210> 116
   <211> 7
   <212> PRT
   <213> Mus musculus
<400> 116
   Gly Tyr Ser Ile Thr Ser Ala
   1 5
<210> 117
   <211> 7
   <212> PRT
   <213> Mus musculus
<400> 117
   Thr Ser Ala Tyr Ala Trp Asn
   1 5
<210> 118
   <211> 16
   <212> PRT
   <213> Mus musculus
<400> 118
   Tyr Ile Asn Ser Ser Gly Ser Thr Ser Tyr His Pro Ser Leu Lys Gly
   1 5 10 15
<210> 119
   <211> 3
   <212> PRT
   <213> Mus musculus
<400> 119
   Ser Ser Gly
   1
<210> 120
   <211> 12
   <212> PRT
   <213> Mus musculus
<400> 120
   Trp Leu Gly Tyr Ile Asn Ser Ser Gly Ser Thr Ser
   1 5 10
<210> 121
   <211> 7
   <212> PRT
   <213> Mus musculus
<400> 121
   Thr Thr Ser Tyr Phe Asp Tyr
   1 5
<210> 122
   <211> 5
   <212> PRT
   <213> Mus musculus
<400> 122
   Thr Ser Tyr Phe Asp
   1 5
<210> 123
   <211> 8
   <212> PRT
   <213> Mus musculus
<400> 123
   Thr His Thr Thr Ser Tyr Phe Asp
   1 5
<210> 124
   <211> 5
   <212> PRT
   <213> Mus musculus
<400> 124
   Ser Tyr Trp Met His
   1 5
<210> 125
   <211> 7
   <212> PRT
   <213> Mus musculus
<400> 125
   Gly Asn Thr Phe Thr Ser Tyr
   1 5
<210> 126
   <211> 6
   <212> PRT
   <213> Mus musculus
<400> 126
   Thr Ser Tyr Trp Met His
   1 5
<210> 127
   <211> 17
   <212> PRT
   <213> Mus musculus
<400> 127
   Tyr Ile Asp Pro Ser Thr Gly Tyr Thr Glu Tyr Asn Gln Lys Phe Lys
   1 5 10 15
   Asp
<210> 128
   <211> 3
   <212> PRT
   <213> Mus musculus
<400> 128
   Ser Thr Gly
   1
<210> 129
   <211> 13
   <212> PRT
   <213> Mus musculus
<400> 129
   Trp Ile Gly Tyr Ile Asp Pro Ser Thr Gly Tyr Thr Glu
   1 5 10
<210> 130
   <211> 7
   <212> PRT
   <213> Mus musculus
<400> 130
   Ser Gly Lys Trp Asp Asp Tyr
   1 5
<210> 131
   <211> 5
   <212> PRT
   <213> Mus musculus
<400> 131
   Gly Lys Trp Asp Asp
   1 5
<210> 132
   <211> 8
   <212> PRT
   <213> Mus musculus
<400> 132
   Thr Lys Ser Gly Lys Trp Asp Asp
   1 5
<210> 133
   <211> 5
   <212> PRT
   <213> Mus musculus
<400> 133
   Arg Tyr Trp Ile Glu
   1 5
<210> 134
   <211> 7
   <212> PRT
   <213> Mus musculus
<400> 134
   Gly Tyr Lys Phe Ser Arg Tyr
   1 5
<210> 135
   <211> 5
   <212> PRT
   <213> Mus musculus
<400> 135
   Gly Tyr Lys Phe Ser
   1 5
<210> 136
   <211> 17
   <212> PRT
   <213> Mus musculus
<400> 136
   Glu Ile Phe Pro Gly Ser Asp Ser Thr Asp Tyr Asn Glu Lys Phe Lys
   1 5 10 15
   Gly
<210> 137
   <211> 3
   <212> PRT
   <213> Mus musculus
<400> 137
   Gly Ser Asp
   1
<210> 138
   <211> 13
   <212> PRT
   <213> Mus musculus
<400> 138
   Trp Ile Gly Glu Ile Phe Pro Gly Ser Asp Ser Thr Asp
   1 5 10
<210> 139
   <211> 10
   <212> PRT
   <213> Mus musculus
<400> 139
   Asp Gly Gly Gly Leu Asp Glu Phe Ala Tyr
   1 5 10
<210> 140
   <211> 8
   <212> PRT
   <213> Mus musculus
<400> 140
   Gly Gly Gly Leu Asp Glu Phe Ala
   1 5
<210> 141
   <211> 11
   <212> PRT
   <213> Mus musculus
<400> 141
   Ala Arg Asp Gly Gly Gly Leu Asp Glu Phe Ala
   1 5 10
<210> 142
   <211> 5
   <212> PRT
   <213> Mus musculus
<400> 142
   Arg Tyr Trp Ile Glu
   1 5
<210> 143
   <211> 7
   <212> PRT
   <213> Mus musculus
<400> 143
   Gly Tyr Lys Phe Ser Arg Tyr
   1 5
<210> 144
   <211> 5
   <212> PRT
   <213> Mus musculus
<400> 144
   Gly Tyr Lys Phe Ser
   1 5
<210> 145
   <211> 17
   <212> PRT
   <213> Mus musculus
<400> 145
   Glu Ile Phe Pro Gly Ser Asp Ser Thr Asp Tyr Asn Glu Lys Phe Lys
   1 5 10 15
   Gly
<210> 146
   <211> 3
   <212> PRT
   <213> Mus musculus
<400> 146
   Gly Ser Asp
   1
<210> 147
   <211> 13
   <212> PRT
   <213> Mus musculus
<400> 147
   Trp Ile Gly Glu Ile Phe Pro Gly Ser Asp Ser Thr Asp
   1 5 10
<210> 148
   <211> 10
   <212> PRT
   <213> Mus musculus
<400> 148
   Asp Gly Gly Gly Leu Asp Glu Phe Ala Tyr
   1 5 10
<210> 149
   <211> 8
   <212> PRT
   <213> Mus musculus
<400> 149
   Gly Gly Gly Leu Asp Glu Phe Ala
   1 5
<210> 150
   <211> 11
   <212> PRT
   <213> Mus musculus
<400> 150
   Ala Arg Asp Gly Gly Gly Leu Asp Glu Phe Ala
   1 5 10
<210> 151
   <211> 6
   <212> PRT
   <213> Mus musculus
<400> 151
   Ser Asp Tyr Ala Trp Asn
   1 5
<210> 152
   <211> 7
   <212> PRT
   <213> Mus musculus
<400> 152
   Gly Ser Ser Ile Thr Ser Asp
   1 5
<210> 153
   <211> 7
   <212> PRT
   <213> Mus musculus
<400> 153
   Thr Ser Asp Tyr Ala Trp Asn
   1 5
<210> 154
   <211> 16
   <212> PRT
   <213> Mus musculus
<400> 154
   Tyr Ile Ser Gln Ser Gly Ser Thr Ser Tyr Asn Pro Ser Leu Lys Ser
   1 5 10 15
<210> 155
   <211> 3
   <212> PRT
   <213> Mus musculus
<400> 155
   Gln Ser Gly
   1
<210> 156
   <211> 12
   <212> PRT
   <213> Mus musculus
<400> 156
   Trp Met Gly Tyr Ile Ser Gln Ser Gly Ser Thr Ser
   1 5 10
<210> 157
   <211> 7
   <212> PRT
   <213> Mus musculus
<400> 157
   Asn Ser Tyr Thr Met Asp Tyr
   1 5
<210> 158
   <211> 5
   <212> PRT
   <213> Mus musculus
<400> 158
   Ser Tyr Thr Met Asp
   1 5
<210> 159
   <211> 8
   <212> PRT
   <213> Mus musculus
<400> 159
   Thr Gly Asn Ser Tyr Thr Met Asp
   1 5
<210> 160
   <211> 6
   <212> PRT
   <213> Mus musculus
<400> 160
   Ser Asp Tyr Ala Trp Asn
   1 5
<210> 161
   <211> 7
   <212> PRT
   <213> Mus musculus
<400> 161
   Gly Asp Ser Ile Thr Ser Asp
   1 5
<210> 162
   <211> 7
   <212> PRT
   <213> Mus musculus
<400> 162
   Thr Ser Asp Tyr Ala Trp Asn
   1 5
<210> 163
   <211> 16
   <212> PRT
   <213> Mus musculus
<400> 163
   Tyr Ile Ser Tyr Ser Gly Ser Thr Ser Tyr Asn Pro Ser Leu Lys Ser
   1 5 10 15
<210> 164
   <211> 3
   <212> PRT
   <213> Mus musculus
<400> 164
   Tyr Ser Gly
   1
<210> 165
   <211> 12
   <212> PRT
   <213> Mus musculus
<400> 165
   Trp Met Gly Tyr Ile Ser Tyr Ser Gly Ser Thr Ser
   1 5 10
<210> 166
   <211> 7
   <212> PRT
   <213> Mus musculus
<400> 166
   Asn Ser Tyr Thr Met Asp Tyr
   1 5
<210> 167
   <211> 5
   <212> PRT
   <213> Mus musculus
<400> 167
   Ser Tyr Thr Met Asp
   1 5
<210> 168
   <211> 8
   <212> PRT
   <213> Mus musculus
<400> 168
   Thr Gly Asn Ser Tyr Thr Met Asp
   1 5
<210> 169
   <211> 5
   <212> PRT
   <213> Mus musculus
<400> 169
   Ser Tyr Val Ile His
   1 5
<210> 170
   <211> 7
   <212> PRT
   <213> Mus musculus
<400> 170
   Gly Tyr Thr Phe Thr Ser Tyr
   1 5
<210> 171
   <211> 6
   <212> PRT
   <213> Mus musculus
<400> 171
   Thr Ser Tyr Val Ile His
   1 5
<210> 172
   <211> 17
   <212> PRT
   <213> Mus musculus
<400> 172
   Tyr Ile Asn Pro Phe Asn Asp Gly Thr Lys Tyr Asn Glu Lys Phe Lys
   1 5 10 15
   Gly
<210> 173
   <211> 3
   <212> PRT
   <213> Mus musculus
<400> 173
   Phe Asn Asp
   1
<210> 174
   <211> 13
   <212> PRT
   <213> Mus musculus
<400> 174
   Trp Ile Gly Tyr Ile Asn Pro Phe Asn Asp Gly Thr Lys
   1               5                   10
<210> 175
   <211> 8
   <212> PRT
   <213> Mus musculus
<400> 175
   Val Gly Ser Ser Gly Ser Asp Tyr
   1               5
<210> 176
   <211> 6
   <212> PRT
   <213> Mus musculus
<400> 176
   Gly Ser Ser Gly Ser Asp
   1               5
<210> 177
   <211> 9
   <212> PRT
   <213> Mus musculus
<400> 177
   Glu Gly Val Gly Ser Ser Gly Ser Asp
   1               5
<210> 178
   <211> 5
   <212> PRT
   <213> Mus musculus
<400> 178
   Asp Tyr Asn Met Tyr
   1               5
<210> 179
   <211> 7
   <212> PRT
   <213> Mus musculus
<400> 179
   Gly Phe Ser Phe Thr Asp Tyr
   1               5
<210> 180
   <211> 6
   <212> PRT
   <213> Mus musculus
<400> 180
   Thr Asp Tyr Asn Met Tyr
   1 5
<210> 181
   <211> 17
   <212> PRT
   <213> Mus musculus
<400> 181
   Tyr Ile Asp Pro Tyr Asn Gly Gly Thr Ser Tyr Asn Gln Lys Phe Lys
   1 5 10 15
   Gly
<210> 182
   <211> 3
   <212> PRT
   <213> Mus musculus
<400> 182
   Tyr Asn Gly
   1
<210> 183
   <211> 13
   <212> PRT
   <213> Mus musculus
<400> 183
   Trp Ile Gly Tyr Ile Asp Pro Tyr Asn Gly Gly Thr Ser
   1 5 10
<210> 184
   <211> 7
   <212> PRT
   <213> Mus musculus
<400> 184
   Ala Gly Asn Tyr Asp Asp Tyr
   1 5
<210> 185
   <211> 5
   <212> PRT
   <213> Mus musculus
<400> 185
   Gly Asn Tyr Asp Asp
   1 5
<210> 186
   <211> 8
   <212> PRT
   <213> Mus musculus
<400> 186
   Ala Ser Ala Gly Asn Tyr Asp Asp
   1 5
<210> 187
   <211> 5
   <212> PRT
   <213> Mus musculus
<400> 187
   Asn Tyr Trp Met Ser
   1 5
<210> 188
   <211> 7
   <212> PRT
   <213> Mus musculus
<400> 188
   Gly Leu Thr Phe Arg Asn Tyr
   1 5
<210> 189
   <211> 5
   <212> PRT
   <213> Mus musculus
<400> 189
   Gly Leu Thr Phe Arg
   1 5
<210> 190
   <211> 19
   <212> PRT
   <213> Mus musculus
<400> 190
   Glu Ile Arg Leu Lys Ser Asp Asn Tyr Ala Thr His Tyr Ala Glu Ser
   1 5 10 15
   Val Lys Gly
<210> 191
   <211> 3
   <212> PRT
   <213> Mus musculus
<400> 191
   Asp Asn Tyr
   1
<210> 192
   <211> 15
   <212> PRT
   <213> Mus musculus
<400> 192
   Trp Val Ala Glu Ile Arg Leu Lys Ser Asp Asn Tyr Ala Thr His
   1 5 10 15
<210> 193
   <211> 7
   <212> PRT
   <213> Mus musculus
<400> 193
   Tyr Gly Lys Asp Phe Asp Tyr
   1 5
<210> 194
   <211> 5
   <212> PRT
   <213> Mus musculus
<400> 194
   Gly Lys Asp Phe Asp
   1 5
<210> 195
   <211> 8
   <212> PRT
   <213> Mus musculus
<400> 195
   Thr Val Tyr Gly Lys Asp Phe Asp
   1 5
<210> 196
   <211> 11
   <212> PRT
   <213> Mus musculus
<400> 196
   Lys Ala Ser Gln Ser Val Ser Asn Asp Val Ala
   1 5 10
<210> 197
   <211> 7
   <212> PRT
   <213> Mus musculus
<400> 197
   Ser Gln Ser Val Ser Asn Asp
   1 5
<210> 198
   <211> 7
   <212> PRT
   <213> Mus musculus
<400> 198
   Ser Asn Asp Val Ala Trp Tyr
   1 5
<210> 199
   <211> 7
   <212> PRT
   <213> Mus musculus
<400> 199
   Tyr Ala Ser Asn Arg Tyr Thr
   1 5
<210> 200
   <211> 10
   <212> PRT
   <213> Mus musculus
<400> 200
   Leu Leu Ile Ser Tyr Ala Ser Asn Arg Tyr
   1 5 10
<210> 201
   <211> 6
   <212> PRT
   <213> Mus musculus
<400> 201
   Asp Tyr Ser Ser Pro Tyr
   1 5
<210> 202
   <211> 8
   <212> PRT
   <213> Mus musculus
<400> 202
   Gln Gln Asp Tyr Ser Ser Pro Tyr
   1 5
<210> 203
   <211> 11
   <212> PRT
   <213> Mus musculus
<400> 203
   Lys Ala Ser Gln Asp Ile Lys Ser Tyr Leu Ser
   1 5 10
<210> 204
   <211> 7
   <212> PRT
   <213> Mus musculus
<400> 204
   Ser Gln Asp Ile Lys Ser Tyr
   1 5
<210> 205
   <211> 7
   <212> PRT
   <213> Mus musculus
<400> 205
   Lys Ser Tyr Leu Ser Trp Tyr
   1 5
<210> 206
   <211> 7
   <212> PRT
   <213> Mus musculus
<400> 206
   Tyr Ala Thr Ser Leu Ala Asp
   1 5
<210> 207
   <211> 10
   <212> PRT
   <213> Mus musculus
<400> 207
   Thr Leu Ile Tyr Tyr Ala Thr Ser Leu Ala
   1 5 10
<210> 208
   <211> 9
   <212> PRT
   <213> Mus musculus
<400> 208
   Leu Gln His Gly Glu Ser Pro Leu Thr
   1 5
<210> 209
   <211> 6
   <212> PRT
   <213> Mus musculus
<400> 209
   His Gly Glu Ser Pro Leu
   1 5
<210> 210
   <211> 8
   <212> PRT
   <213> Mus musculus
<400> 210
   Leu Gln His Gly Glu Ser Pro Leu
   1 5
<210> 211
   <211> 11
   <212> PRT
   <213> Mus musculus
<400> 211
   Lys Ala Ser Gln Ser Val Ser Asn Asp Val Ala
   1 5 10
<210> 212
   <211> 7
   <212> PRT
   <213> Mus musculus
<400> 212
   Ser Gln Ser Val Ser Asn Asp
   1 5
<210> 213
   <211> 7
   <212> PRT
   <213> Mus musculus
<400> 213
   Ser Asn Asp Val Ala Trp Tyr
   1 5
<210> 214
   <211> 7
   <212> PRT
   <213> Mus musculus
<400> 214
   Tyr Ala Ser Asn Arg Tyr Thr
   1 5
<210> 215
   <211> 10
   <212> PRT
   <213> Mus musculus
<400> 215
   Leu Leu Ile Ser Tyr Ala Ser Asn Arg Tyr
   1 5 10
<210> 216
   <211> 5
   <212> PRT
   <213> Mus musculus
<400> 216
   Tyr Ser Ser Pro Tyr
   1 5
<210> 217
   <211> 8
   <212> PRT
   <213> Mus musculus
<400> 217
   Gln Gln Asp Tyr Ser Ser Pro Tyr
   1 5
<210> 218
   <211> 11
   <212> PRT
   <213> Mus musculus
<400> 218
   Lys Ala Ser Gln Ser Val Thr Asn Asp Val Ala
   1 5 10
<210> 219
   <211> 6
   <212> PRT
   <213> Mus musculus
<400> 219
   Gln Ser Val Thr Asn Asp
   1 5
<210> 220
   <211> 7
   <212> PRT
   <213> Mus musculus
<400> 220
   Thr Asn Asp Val Ala Trp Tyr
   1 5
<210> 221
   <211> 7
   <212> PRT
   <213> Mus musculus
<400> 221
   Tyr Ala Ser Asn Arg Tyr Thr
   1 5
<210> 222
   <211> 10
   <212> PRT
   <213> Mus musculus
<400> 222
   Leu Leu Ile Cys Tyr Ala Ser Asn Arg Tyr
   1 5 10
<210> 223
   <211> 9
   <212> PRT
   <213> Mus musculus
<400> 223
   Gln Gln Asp His Thr Ser Pro Phe Thr
   1 5
<210> 224
   <211> 6
   <212> PRT
   <213> Mus musculus
<400> 224
   Asp His Thr Ser Pro Phe
   1 5
<210> 225
   <211> 8
   <212> PRT
   <213> Mus musculus
<400> 225
   Gln Gln Asp His Thr Ser Pro Phe
   1 5
<210> 226
   <211> 16
   <212> PRT
   <213> Mus musculus
<400> 226
   Arg Ser Ser Gln Ser Leu Thr Asn Ser Tyr Gly Tyr Thr Tyr Leu Ser
   1               5                   10                  15
<210> 227
   <211> 12
   <212> PRT
   <213> Mus musculus
<400> 227
   Ser Gln Ser Leu Thr Asn Ser Tyr Gly Tyr Thr Tyr
   1               5                   10
<210> 228
   <211> 7
   <212> PRT
   <213> Mus musculus
<400> 228
   Tyr Thr Tyr Leu Ser Trp Tyr
   1               5
<210> 229
   <211> 7
   <212> PRT
   <213> Mus musculus
<400> 229
   Gly Ile Ser Asn Arg Phe Ser
   1               5
<210> 230
   <211> 10
   <212> PRT
   <213> Mus musculus
<400> 230
   Leu Leu Ile Tyr Gly Ile Ser Asn Arg Phe
   1               5                   10
<210> 231
   <211> 6
   <212> PRT
   <213> Mus musculus
<400> 231
   Gly Thr His Gln Pro Tyr
   1               5
<210> 232
   <211> 8
   <212> PRT
   <213> Mus musculus
<400> 232
   Leu Gln Gly Thr His Gln Pro Tyr
   1 5
<210> 233
   <211> 16
   <212> PRT
   <213> Mus musculus
<400> 233
   Arg Ser Ser Gln Ser Leu Thr Asn Ser Tyr Gly Tyr Thr Tyr Leu Ser
   1 5 10 15
<210> 234
   <211> 12
   <212> PRT
   <213> Mus musculus
<400> 234
   Ser Gln Ser Leu Thr Asn Ser Tyr Gly Tyr Thr Tyr
   1 5 10
<210> 235
   <211> 7
   <212> PRT
   <213> Mus musculus
<400> 235
   Tyr Thr Tyr Leu Ser Trp Tyr
   1 5
<210> 236
   <211> 7
   <212> PRT
   <213> Mus musculus
<400> 236
   Gly Ile Ser Asn Arg Phe Ser
   1 5
<210> 237
   <211> 10
   <212> PRT
   <213> Mus musculus
<400> 237
   Leu Leu Ile Tyr Gly Ile Ser Asn Arg Phe
   1 5 10
<210> 238
   <211> 6
   <212> PRT
   <213> Mus musculus
<400> 238
   Gly Thr His Gln Pro Tyr
   1 5
<210> 239
   <211> 8
   <212> PRT
   <213> Mus musculus
<400> 239
   Leu Gln Gly Thr His Gln Pro Tyr
   1 5
<210> 240
   <211> 11
   <212> PRT
   <213> Mus musculus
<400> 240
   Lys Ala Ser Gln Ser Val Ser Asn Asp Val Ala
   1 5 10
<210> 241
   <211> 7
   <212> PRT
   <213> Mus musculus
<400> 241
   Ser Gln Ser Val Ser Asn Asp
   1 5
<210> 242
   <211> 7
   <212> PRT
   <213> Mus musculus
<400> 242
   Ser Asn Asp Val Ala Trp Tyr
   1 5
<210> 243
   <211> 7
   <212> PRT
   <213> Mus musculus
<400> 243
   Tyr Ala Ser Asn Arg Tyr Thr
   1 5
<210> 244
   <211> 10
   <212> PRT
   <213> Mus musculus
<400> 244
   Leu Leu Ile Ser Tyr Ala Ser Asn Arg Tyr
   1 5 10
<210> 245
   <211> 6
   <212> PRT
   <213> Mus musculus
<400> 245
   Asp Tyr Ser Ser Pro Trp
   1 5
<210> 246
   <211> 8
   <212> PRT
   <213> Mus musculus
<400> 246
   Gln Gln Asp Tyr Ser Ser Pro Trp
   1 5
<210> 247
   <211> 11
   <212> PRT
   <213> Mus musculus
<400> 247
   Lys Ala Ser Gln Ser Val Ser Asn Asp Val Ala
   1 5 10
<210> 248
   <211> 7
   <212> PRT
   <213> Mus musculus
<400> 248
   Ser Gln Ser Val Ser Asn Asp
   1 5
<210> 249
   <211> 7
   <212> PRT
   <213> Mus musculus
<400> 249
   Ser Asn Asp Val Ala Trp Tyr
   1 5
<210> 250
   <211> 7
   <212> PRT
   <213> Mus musculus
<400> 250
   Tyr Ala Ser Asn Arg Tyr Thr
   1 5
<210> 251
   <211> 6
   <212> PRT
   <213> Mus musculus
<400> 251
   Tyr Ala Ser Asn Arg Tyr
   1 5
<210> 252
   <211> 6
   <212> PRT
   <213> Mus musculus
<400> 252
   Asp Tyr Ile Ser Pro Trp
   1 5
<210> 253
   <211> 8
   <212> PRT
   <213> Mus musculus
<400> 253
   Gln Gln Asp Tyr Ile Ser Pro Trp
   1 5
<210> 254
   <211> 11
   <212> PRT
   <213> Mus musculus
<400> 254
   Lys Ala Ser Gln Ser Val Arg Asn Asp Val Ala
   1 5 10
<210> 255
   <211> 7
   <212> PRT
   <213> Mus musculus
<400> 255
   Ser Gln Ser Val Arg Asn Asp
   1 5
<210> 256
   <211> 7
   <212> PRT
   <213> Mus musculus
<400> 256
   Arg Asn Asp Val Ala Trp Tyr
   1 5
<210> 257
   <211> 7
   <212> PRT
   <213> Mus musculus
<400> 257
   Tyr Ala Ser Asn Arg Tyr Thr
   1 5
<210> 258
   <211> 10
   <212> PRT
   <213> Mus musculus
<400> 258
   Leu Leu Ile Ser Tyr Ala Ser Asn Arg Tyr
   1 5 10
<210> 259
   <211> 6
   <212> PRT
   <213> Mus musculus
<400> 259
   Asp Tyr Ser Ser Pro Tyr
   1 5
<210> 260
   <211> 8
   <212> PRT
   <213> Mus musculus
<400> 260
   Gln Gln Asp Tyr Ser Ser Pro Tyr
   1 5
<210> 261
   <211> 11
   <212> PRT
   <213> Mus musculus
<400> 261
   Lys Ala Ser Gln Ser Val Ser Asn Asp Val Ala
   1 5 10
<210> 262
   <211> 7
   <212> PRT
   <213> Mus musculus
<400> 262
   Ser Gln Ser Val Ser Asn Asp
   1 5
<210> 263
   <211> 7
   <212> PRT
   <213> Mus musculus
<400> 263
   Ser Asn Asp Val Ala Trp Tyr
   1 5
<210> 264
   <211> 7
   <212> PRT
   <213> Mus musculus
<400> 264
   Tyr Ala Ser Asn Arg Tyr Thr
   1 5
<210> 265
   <211> 10
   <212> PRT
   <213> Mus musculus
<400> 265
   Leu Leu Ile Tyr Tyr Ala Ser Asn Arg Tyr
   1 5 10
<210> 266
   <211> 6
   <212> PRT
   <213> Mus musculus
<400> 266
   Asp Tyr Thr Ser Pro Tyr
   1 5
<210> 267
   <211> 8
   <212> PRT
   <213> Mus musculus
<400> 267
   Gln Gln Asp Tyr Thr Ser Pro Tyr
   1 5
<210> 268
   <211> 11
   <212> PRT
   <213> Mus musculus
<400> 268
   Lys Ala Ser Gln Ser Val Ser Asn Asp Val Ala
   1 5 10
<210> 269
   <211> 7
   <212> PRT
   <213> Mus musculus
<400> 269
   Ser Gln Ser Val Ser Asn Asp
   1 5
<210> 270
   <211> 7
   <212> PRT
   <213> Mus musculus
<400> 270
   Ser Asn Asp Val Ala Trp Tyr
   1 5
<210> 271
   <211> 7
   <212> PRT
   <213> Mus musculus
<400> 271
   Tyr Ala Ser Asn Arg Tyr Thr
   1 5
<210> 272
   <211> 10
   <212> PRT
   <213> Mus musculus
<400> 272
   Leu Leu Ile Ser Tyr Ala Ser Asn Arg Tyr
    1               5                  10
<210> 273
   <211> 6
   <212> PRT
   <213> Mus musculus
<400> 273
   Asp Tyr Ser Ser Pro Trp
    1               5
<210> 274
   <211> 8
   <212> PRT
   <213> Mus musculus
<400> 274
   Gln Gln Asp Tyr Ser Ser Pro Trp
    1               5
<210> 275
   <211> 11
   <212> PRT
   <213> Artificial Sequence
<220>
   <223> Mus musculus consensus
<220>
   <221> VARIANT
   <222> 7
   <223> Xaa = Ser, Thr, or Arg
<400> 275
   Lys Ala Ser Gln Ser Val Xaa Asn Asp Val Ala
    1               5                  10
<210> 276
   <211> 7
   <212> PRT
   <213> Artificial Sequence
<220>
   <223> Mus musculus consensus
<220>
   <221> VARIANT
   <222> 5
   <223> Xaa = Ser, Thr, or Arg
<400> 276
   Ser Gln Ser Val Xaa Asn Asp
   1 5
<210> 277
   <211> 7
   <212> PRT
   <213> Artificial Sequence
<220>
   <223> Mus musculus consensus
<220>
   <221> VARIANT
   <222> 1
   <223> Xaa = Ser, Thr, or Arg
<400> 277
   Xaa Asn Asp Val Ala Trp Tyr
   1 5
<210> 278
   <211> 13
   <212> PRT
   <213> Artificial Sequence
<220>
   <223> Mus musculus consensus
<220>
   <221> VARIANT
   <222> 7
   <223> Xaa = Ser, Thr, or Arg
<400> 278
   Lys Ala Ser Gln Ser Val Xaa Asn Asp Val Ala Trp Tyr
   1 5 10
<210> 279
   <211> 7
   <212> PRT
   <213> Artificial Sequence
<220>
   <223> Mus musculus consensus
<400> 279
   Tyr Ala Ser Asn Arg Tyr Thr
   1 5
<210> 280
   <211> 3
   <212> PRT
   <213> Artificial Sequence
<220>
   <223> Mus musculus consensus
<400> 280
   Tyr Ala Ser
   1
<210> 281
   <211> 10
   <212> PRT
   <213> Artificial Sequence
<220>
   <223> Mus musculus consensus
<220>
   <221> VARIANT
   <222> 4
   <223> Xaa = Tyr, Ser or Cys
<400> 281
   Leu Leu Ile Xaa Tyr Ala Ser Asn Arg Tyr
   1 5 10
<210> 282
   <211> 11
   <212> PRT
   <213> Artificial Sequence
<220>
   <223> Mus musculus consensus
<220>
   <221> VARIANT
   <222> 4
   <223> Xaa = Tyr, Ser or Cys
<400> 282
   Leu Leu Ile Xaa Tyr Ala Ser Asn Arg Tyr Thr
   1 5 10
<210> 283
   <211> 9
   <212> PRT
   <213> Artificial Sequence
<220>
   <223> Mus musculus consensus
<220>
   <221> VARIANT
   <222> 4
   <223> Xaa = Tyr or His
<220>
   <221> VARIANT
   <222> 5
   <223> Xaa = Ser, Thr, or Ile
<220>
   <221> VARIANT
   <222> 8
   <223> Xaa = Tyr, Phe, or Trp
<400> 283
   Gln Gln Asp Xaa Xaa Ser Pro Xaa Thr
    1               5
<210> 284
   <211> 8
   <212> PRT
   <213> Artificial Sequence
<220>
   <223> Mus musculus consensus
<220>
   <221> VARIANT
   <222> 4
   <223> Xaa = Tyr or His
<220>
   <221> VARIANT
   <222> 5
   <223> Xaa = Ser, Thr, or Ile
<220>
   <221> VARIANT
   <222> 8
   <223> Xaa = Tyr, Phe, or Trp
<400> 284
   Gln Gln Asp Xaa Xaa Ser Pro Xaa
    1               5
<210> 285
   <211> 6
   <212> PRT
   <213> Artificial Sequence
<220>
   <223> Mus musculus consensus
<220>
   <221> VARIANT
   <222> 2
   <223> Xaa = Tyr or His
<220>
   <221> VARIANT
   <222> 3
   <223> Xaa = Ser, Thr, or Ile
<220>
   <221> VARIANT
   <222> 6
   <223> Xaa = Tyr, Phe, or Trp
<400> 285
   Asp Xaa Xaa Ser Pro Xaa
    1               5